(19) Europäisches Patentamt
European Patent Office
Office européen des brevets

(11) EP 0 757 982 B1

(12) FASCICULE DE BREVET EUROPEEN

(45) Date de publication et mention de la délivrance du brevet: **20.09.2000 Bulletin 2000/38**

(21) Numéro de dépôt: **96401745.3**

(22) Date de dépôt: **07.08.1996**

(51) Int Cl.[7]: **C07C 233/65**, C07C 235/20, C07C 255/57, C07C 237/30, C07C 259/10, C07C 255/54, C07D 257/04, A61K 31/16, A61K 31/165, A61K 31/41, A61K 31/275

(54) **Composés biphényles, leur procédé de préparation et les intermédiaires de ce procédé, leur application en tant qu'inhibiteur de la 5-alpha-réductase et les compositions pharmaceutiques les contenant**

Biphenylverbindungen, Verfahren zu ihrer Herstellung und Intermediate für dieses Verfahren, ihre Verwendung als 5-alpha-Reduktase-Inhibitoren und diese enthaltende pharmazeutische Zusammensetzungen

Biphenylic compounds, process for their preparations and intermediates for this process, their use as 5-alpha-reductase inhibitors and pharmaceutical compositions containing them

(84) Etats contractants désignés:
**CH DE FR GB IT LI NL**

(30) Priorité: **08.08.1995 FR 9509618**

(43) Date de publication de la demande:
**12.02.1997 Bulletin 1997/07**

(73) Titulaire: **HOECHST MARION ROUSSEL
92800 Puteaux (FR)**

(72) Inventeurs:
- **Gourvest, Jean-François
77410 Claye Souilly (FR)**
- **Lesuisse, Dominique
75018 Paris (FR)**
- **Teutsch, Jean-Georges
93500 Pantin (FR)**

(74) Mandataire: **Vieillefosse, Jean-Claude et al
Hoechst Marion Roussel
Département des Brevets
102, Route de Noisy
93235 Romainville Cédex (FR)**

(56) Documents cités:

| | |
|---|---|
| **EP-A- 0 296 802** | **EP-A- 0 533 266** |
| **WO-A-93/03012** | **WO-A-93/21178** |
| **WO-A-93/24442** | **WO-A-95/26328** |
| **GB-A- 2 276 160** | **GB-A- 2 276 161** |

- **JOURNAL OF ORGANIC CHEMISTRY, vol. 57, no. 15, 1992, EASTON US, pages 4066-68, XP000567808 S. SENGUPTA ET AL.: "Ni(0)-catalyzed cross coupling of aryl O-carbamates and aryl triflates with grignard reagents."**
- **CHEMICAL ABSTRACTS, vol. 111, no. 3, 17 Juillet 1989 Columbus, Ohio, US; abstract no. 20318, SOUNDARARAJAN, SOUNDARAMANI ET AL: "Boronic acids for affinity chromatography: spectral methods for determinations of ionization and diol-binding constants" XP002000557 & ANAL. BIOCHEM. (1989), 178(1), 125-34 , 1989,**
- **CHEMISCHE BERICHTE, vol. 113, no. 11, 1980, WEINHEIM DE, pages 3610-20, XP000567807 U. BROTZELLER ET AL.: "1H-NMR-Spektren mesitylsubstituierter 2,6-Dimethylbenzol-Derivate"**
- **CHEMICAL ABSTRACTS, vol. 92, no. 5, 4 Février 1980 Columbus, Ohio, US; abstract no. 41603, KARAMYSHEVA, L. A. ET AL: "p-Alkyl(alkoxy)biphenyl-p'-carboxamides as intermediates for the synthesis of p-alkyl(alkoxy)-p'-cyanobiphenyls" XP002000558 & SU 681 052 A (USSR)**
- **CHEMICAL ABSTRACTS, vol. 52, no. 17, 10 Septembre 1958 Columbus, Ohio, US; abstract no. 14552, K. TORSSELL: "Arylboronic acids" XP002000559 & ARKIV KEMI, vol. 10, 1957, pages 473-82,**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

- **DATABASE WPI Section Ch, Week 9327 Derwent Publications Ltd., London, GB; Class B05, AN 93-216733 XP002000560 & JP 05 140 062 A ( ONO PHARM CO LTD) , 8 Juin 1993**

## Description

La présente invention concerne de nouveaux composés biphényles, leur procédé de préparation et les intermédiaires de ce procédé, leur application à titre de médicament et les compositions pharmaceutiques les contenant.

WO-A-9303012, WO-A-9324442 et JP-A-05140062 décrivent des composés, ayant une activité inhibitrice vis à vis de la 5-α-reductase, structurellement différents de ceux de notre demande. WO-A-9303012 décrit des dérivés de l'indole, WO-A-9324442 décrit des dérivés biphényles, les 2 groupes phényles étant séparés par un hétéroatome et JP-A-05140062 décrit des dérivés de benzylaminophénoxybutarate.

L'invention a pour objet , à titre de médicaments, les composés de formule générale (I) :

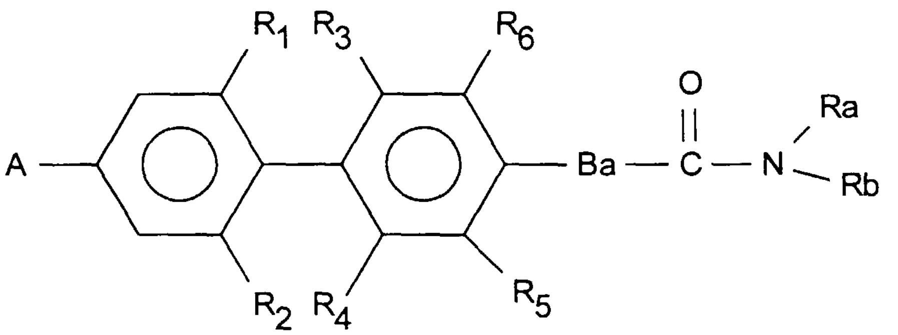

(I)

dans laquelle A représente un radical carboxy, cyano, nitro, HONHCO- ou un hétérocycle azoté, $B_a$ représente une simple liaison, un groupement $(CH_2)_n$, n étant un entier pouvant varier de 1 à 6, $(CH_2)_{n-1}$-CH($Alk_1$), $Alk_1$ étant un radical alkyle linéaire ou ramifié renfermant de 1 à 8 atomes de carbone, ou O-$(CH_2)_{n'}$, n' étant un entier pouvant varier de 1 à 6, $R_a$ et $R_b$, identiques ou différents, représentent un atome d'hydrogène, un radical alkyle linéaire ou ramifié renfermant de 1 à 8 atomes de carbone, un radical acyle renfermant de 1 à 12 atomes de carbone, un radical phényle, un radical benzyle, un radical diphénylméthyle, un radical trityle ou forment ensemble avec l'atome d'azote auquel ils sont liés un hétérocycle azoté saturé ou insaturé à 5 ou 6 chaînons, pouvant renfermer un second hétéroatome choisi parmi les atomes d'azote, d'oxygène et de soufre, $R_1$, $R_2$, $R_3$ et $R_6$ identiques ou différents, représentent un atome d'hydrogène, un atome d'halogène, un radical alkyle linéaire ou ramifié renfermant de 1 à 8 atomes de carbone éventuellement substitué, un radical alkoxy ou alkylthio renfermant de 1 à 8 atomes de carbone, un radical nitro, cyano, -$CF_3$, amino, alkylamino ou dialkylamino dans lesquels chaque radical alkyle renferme de 1 à 8 atomes de carbone, un radical -$NR'_aR'_b$ dans lequel $R'_a$ et $R'_b$ forment ensemble avec l'atome d'azote auquel ils sont liés un hétérocycle azoté saturé ou insaturé à 5 ou 6 chaînons pouvant renfermer éventuellement un second hétéroatome choisi parmi les atomes d'azote, d'oxygène et de soufre et éventuellement substitué par un radical alkyle renfermant de 1 à 4 atomes de carbone, $R_4$ et $R_5$, identiques ou différents, représentent les mêmes valeurs que $R_1$, $R_2$, $R_3$ ou $R_6$ ou forment ensemble un groupement -CH=CH-CH=CH-, ainsi que les sels d'addition de ceux-ci avec les acides ou les bases.

Les radicaux $R_1$, $R_2$, $R_3$, $R_4$, $R_5$ ou $R_6$ peuvent représenter un radical alkyl substitué par un ou plusieurs radicaux identiques ou différents choisis parmi les radicaux halogènes, hydroxy, alkoxy renfermant de 1 à 4 atomes de carbone, alkylthio renfermant de 1 à 4 atomes de carbone, acyloxy renfermant de 1 à 12 atomes de carbone, alkoxycarbonyle renfermant de 2 à 12 atomes de carbone, acyle renfermant de 1 à 12 atomes, amino, alkylamino ou dialkylamino dans lesquels chaque radical alkyle renferme de 1 à 8 atomes de carbone, un radical -$NR''_aR''_b$ dans lequel R"a et R"b forment ensemble avec l'atome d'azote auquel ils sont liés un hétérocycle azoté saturé ou insaturé à 5 ou 6 chaînons renfermant éventuellement un second hétéroatome choisi parmi les atomes d'azote, d'oxygène et de soufre.

Par radical alkyle linéaire ou ramifié renfermant de 1 à 8 atomes de carbone on entend les radicaux méthyle, éthyle, propyle, isopropyle, butyle, isobutyle, tert-butyle, n-pentyle, n-hexyle, 2-méthyl pentyle, 2,3-diméthyl butyle, n-heptyle, 2-méthylhexyle, 2,2-diméthylpentyle, 3,3-diméthyl pentyle, 3-éthylpentyle, n-octyle, 2,2-diméthylhexyle, 3,3-diméthylhexyle, 3-méthyl-3-éthylpentyle. Il s'agit de préférence du radical méthyle, éthyle, propyle ou isopropyle.

Parmi les radicaux acyle renfermant de 1 à 12 atomes, on peut citer les radicaux formyle, acétyle, propionyle, butyryle, isobutyryle, valéryle, isovaléryle, oxalyle, succinyle ou pivaloyle.

Lorsque $R_a$ et $R_b$, $R'_a$ et $R'_b$ ou $R''_a$ et $R''_b$ forment ensemble avec l'atome d'azote auquel ils sont liés un hétérocycle à 5 ou 6 chaînons, il s'agit soit d'un hétérocycle saturé, de préférence une pyrrolidine ou une pipéridine, soit d'un hétérocycle insaturé, de préférence un pyrrole ou une pyridine, soit d'un hétérocycle saturé ou insaturé renfermant le cas échéant un autre hétéroatome choisi parmi l'azote, l'oxygène et le soufre. Il s'agit notamment de la morpholine, la pipérazine ou la pyrimidine, le deuxième atome d'azote étant éventuellement substitué par un radical

alkyle, de préférence méthyle, éthyle, propyle ou isopropyle.

$R_a$ et $R_b$ forment ensemble avec l'azote auquel ils sont liés de préférence un hétérocycle saturé à 5 ou 6 chaînons renfermant éventuellement un second hétéroatome.

Lorsque A représente un hétérocycle azoté, il s'agit de préférence du radical tétrazole.

Par radical alkoxy renfermant de 1 à 8 atomes de carbone il peut s'agir notamment des radicaux méthoxy, éthoxy, propyloxy, butyloxy. Par radical alkylthio renfermant de 1 à 8 atomes de carbone, il peut s'agir notamment des radicaux méthylthio, éthylthio, propylthio ou butylthio.

Par radical acyloxy renfermant de 1 à 12 atomes de carbone, il peut s'agir notamment du dérivé d'un acide aliphatique ou cycloaliphatique saturé ou insaturé et notamment,

- d'un acide alcanoique tel que par exemple l'acide acétique, propionique, butyrique ou isobutyrique, valérique ou undécyclique,
- d'un acide cycloalcoylcarboxylique ou cycloalcoylalcanoique tel que par exemple l'acide cyclopropyl, cyclopentyl ou cyclohexylcarboxylique, cyclopentyle ou cyclohexyle acétique ou propionique. Il s'agit de préférence du dérivé de l'acide acétique, propionique ou butyrique.

Par atome d'halogène, on entend le fluor, le chlore, le brome ou l'iode.

L'invention s'étend aux sels des composés de formule (I), c'est-à-dire aux sels formés, lorsque les composés de formule (I) comportent une fonction amino, avec par exemple les acides chlorhydrique, bromhydrique, nitrique, sulfurique, phosphorique, acétique, formique, propionique, benzoique, maléique, fumarique, succinique, tartrique, citrique, oxalique, glyoxylique, aspartique, alcane sulfonique tels que les acides méthane ou éthane sulfonique, arylsulfonique, tels que les acides benzène ou paratoluène sulfonique et arylcarboxylique, ou lorsque les composés de formule (I) comportent une fonction acide, avec les métaux alcalins ou alcalinoterreux ou le radical ammonium éventuellement substitué. Il s'agit de préférence du sel de sodium.

L'invention a particulièrement pour objet , à titre de médicaments, les composés de formule générale (I) telle que définie précédemment dans laquelle A représente un groupement carboxyle et -$B_a$- représente une simple liaison, un groupement -$CH_2$-, -CH(Me)- ou -O-$CH_2$- ainsi que les sels d'addition de ceux-ci.

L'invention a particulièrement pour objet , à titre de médicaments, les composés de formule générale (I) telle que définie précédemment dans laquelle A représente un groupement tétrazole et -$B_a$- représente une simple liaison, un groupement -$CH_2$-, -CH(Me)- ou -O-$CH_2$- ainsi que les sels d'addition de ceux-ci.

L'invention a plus particulièrement pour objet , à titre de médicaments, les composés de formule générale (I) telle que définie précédemment dans laquelle $R_a$ et $R_b$ représentent chacun un groupement alkyle linéaire ou ramifié renfermant de 1 à 8 atomes de carbone ainsi que les sels d'addition de ceux-ci. $R_a$ et $R_b$ représentent de préférence un groupement méthyle, éthyle, n-propyle ou isopropyle. Il s'agit tout particulièrement du groupement isopropyle.

L'invention a plus particulièrement pour objet , à titre de médicaments, les composés de formule générale (I) telle que définie précédemment dans laquelle $R_a$ est un atome d'hydrogène et $R_b$ est un groupement trityle ainsi que les sels d'addition de ceux-ci.

L'invention a plus particulièrement pour objet , à titre de médicaments, les composés de formule générale (I) telle que définie précédemment dans laquelle $R_1$, $R_2$, $R_3$ et $R_6$ identiques ou différents, représentent un atome d'hydrogène, de chlore, d'iode, de brome, de fluor, un radical méthyle, éthyle, propyle, isopropyle, cyano, nitro, amino, diméthylamino, méthoxy, ethoxy, méthylthio, éthylthio, trifluorométhyle, et $R_4$ et $R_5$, identiques ou différents, représentent les mêmes valeurs que $R_1$, $R_2$, $R_3$ ou $R_6$ ou forment ensemble un groupement -CH=CH-CH=CH- ainsi que les sels d'addition de ceux-ci.

L'invention a tout particulièrement pour objet , à titre de médicaments, les composés de formule générale (I) telle que définie précédemment dans laquelle, A représente un groupement carboxyle, $B_a$ représente une simple liaison, un groupement -$CH_2$- ou -O-$CH_2$-,

<u>soit</u> $R_a$ et $R_b$ représentent chacun un groupement alkyle linéaire ou ramifié renfermant de 1 à 8 atomes de carbone, <u>soit</u> $R_a$ représente un atome d'hydrogène et $R_b$ représente un groupement trityle, $R_1$, $R_2$, $R_3$, $R_4$, $R_5$ et $R_6$ identiques ou différents, représentent un atome d'hydrogène, de chlore, d'iode, de brome, de fluor, un radical méthyle, éthyle, propyle, isopropyle, cyano, nitro, amino, diméthylamino, méthoxy, éthoxy, méthylthio, éthylthio, trifluorométhyle ainsi que les sels d'addition avec ceux-ci.

L'invention a plus particulièrement pour objet , à titre de médicaments, les composés de formule générale (I) telle que définie précédemment dans laquelle A représente un groupement carboxyle et $B_a$ représente un groupement -O-$CH_2$- ainsi que les sels d'addition de ceux-ci.

L'invention a tout particulièrement pour objet , à titre de médicaments, les composés de formule générale (I) suivants :

- Acide 4'-[2-[bis(1-méthyléthyl)amino]-2-oxoéthoxy)-(1,1'-biphényl)-4-carboxylique,
- Acide 4'-[2-[bis(1-méthyléthyl)amino]-2-oxoéthoxy]-3',5'-bis(1-méthyléthyl)-(1,1'-biphényl)-4-carboxylique,
- Acide 4'-[2-[bis(1-méthyléthyl)amino]-2-oxoéthoxy]-3'-fluoro-5'-nitro-(1,1'-biphényl)-4-carboxylique,
- Acide 4'-[2-oxo-2-([triphénylméthyl)amino]éthoxy]-(1,1'-biphényl)-4-carboxylique,
- Acide 3'-fluoro-5'-nitro-4'-[2-oxo-2-[(triphénylméthyl) amino]éthoxy]-(1,1'-biphényl)-4-carboxylique,
- Acide 4'-[2-[bis(1-méthyléthyl)amino)-2-oxoéthoxy]-2'-éthyl-(1,1'-biphényl)-4-carboxylique,
- Acide 4'-[2-[bis(1-méthyléthyl)amino)-2-oxoéthoxy]-3'-éthyl-(1,1'-biphényl)-4-carboxylique,
- Acide 4'-[2-[bis(1-méthyléthyl)amino]-2-oxoéthoxy]-2-chloro (1,1'-biphényl)-4-carboxylique,
- Acide 4'-[2-[bis(1-méthyléthyl)amino]-2-oxoéthoxy]-2-(trifluorométhyl)-(1,1'-biphényl)-4-carboxylique,

ainsi que les sels d'addition avec ceux-ci.

L'invention a également pour objet un procédé de préparation des composés de formule générale (I) telle que définie ci-dessus caractérisé en ce que l'on soumet un composé de formule (II) :

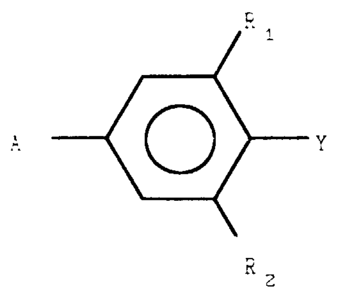

(II)

à l'action en présence d'un catalyseur, d'un composé de formule générale (III) :

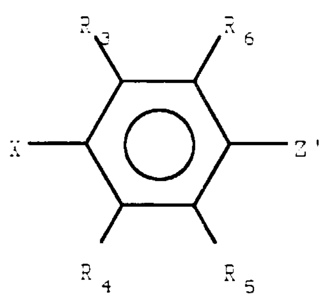

(III)

dans lesquelles, $R_1$, $R_2$, $R_3$, $R_4$, $R_5$ ou $R_6$ sont tels que définis plus haut, A' représente les groupements formyle éventuellement protégé, carboxy éventuellement estérifié, cyano, hétérocycle azoté, nitro, méthyle ou $CH_2OH$ éventuellement protégé, Z' représente un groupement $B_a$-CO-$NR_aR_b$ ou un groupement OH éventuellement protégé et dans lesquels :

(a) soit Y représente un groupement $B(OH)_2$ ou $SnBu_3$ et X représente un groupement $OSO_2CF_3$, un atome de brome ou un atome d'iode,
(b) soit Y représente un groupement $OSO_2CF_3$, un atome de brome ou un atome d'iode et X représente un groupement $B(OH)_2$ ou $SnBu_3$,
(c) soit Y représente un atome d'iode et X représente un atome de chlore,
(d) soit Y représente un atome de chlore et X représente un atome d'iode,
(e) soit Y représente un atome de brome et X représente un atome d'hydrogène,
(f) soit Y représente un atome d'hydrogène et X représente un atome de brome,

afin d'obtenir un produit de formule (I') :

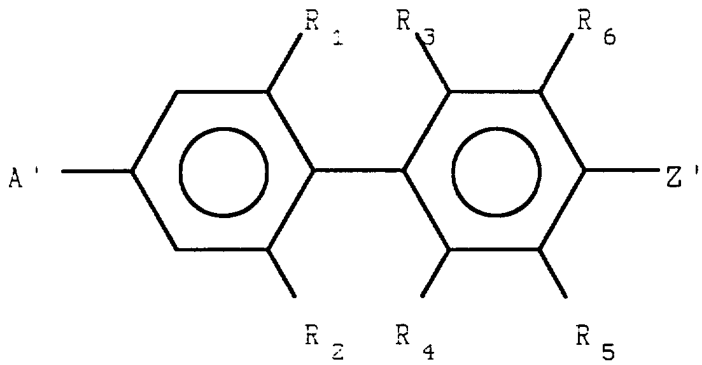

(I')

dans laquelle $R_1$, $R_2$, $R_3$, $R_4$, $R_5$, $R_6$, A' et Z' sont tels que définis précédemment, pouvant, le cas échéant, correspondre à certains produits de formule (I), produit de formule (I') que si désiré ou si nécessaire l'on soumet dans un ordre approprié à l'une ou, le cas échéant, plusieurs des réactions suivantes afin d'obtenir le produit de formule (I) :

- oxydation du groupement formyle que peut représenter A',
- oxydation du groupement méthyle que peut représenter A',
- oxydation du groupement $CH_2OH$ que peut représenter A',
- saponification de l'ester que peut représenter A',
- action de l'azido terbutyl étain ($Bu_3SnN_3$) puis de l'acide chlorhydrique lorsque A' représente un groupement cyano afin d'obtenir le produit de formule (I) dans lesquels A est un radical tétrazole,
- action du chlorhydrate de benzylhydroxylamine lorsque A' représente $CO_2H$ afin d'obtenir les produits de formule (I) dans lesquels A est un groupement CONHOH,
- protection des groupements $CH_2OH$, CHO, $CO_2H$ que peut représenter A',
- protection du groupement OH que peut représenter Z',
- déprotection des groupements $CH_2OH$, CHO, $CO_2H$ protégés que peut représenter A',
- déprotection du groupement OH protégé que peut représenter Z',
- action d'un groupement Hal-$(CH2)_n$-CO-$NR_aR_b$ lorsque Z' représente un hydroxyle,
- action de $Alk_1$-X en présence d'une base forte lorsque Z ou Z' représentent un groupement $(CH2)_n$-CO-$NR_aR_b$ afin d'obtenir les produits de formule (I) dans lesquels A est un groupement $(CH2)_{n-1}$-CH($Alk_1$)-CO-$NR_aR_b$,
- réduction totale ou partielle des groupements $NO_2$ que peuvent représenter $R_1$, $R_2$, $R_3$, $R_4$, $R_5$ ou $R_6$,
- substitution du radical $NH_2$ que peuvent représenter $R_1$, $R_2$, $R_3$, $R_4$, $R_5$, $R_6$ par un atome de brome ou d'iode,
- et salification par un acide ou une base.

La formation des biphényles de formule (I') par couplage du composé aromatique de formule (II) avec le composé aromatique de formule (III) s'effectue :
soit en présence d'un catalyseur choisi parmi les dérivés du palladium dans les cas où :

(a) Y représente un groupement $B(OH)_2$ ou $SnBu_3$ et X représente un groupement $OSO_2CF_3$, un atome de brome ou un atome d'iode,
(b) Y représente un groupement $OSO_2CF_3$ ou un atome de brome ou un atome d'iode et X représente un groupement $B(OH)_2$ ou $SnBu_3$,

et peut ainsi s'effectuer dans les conditions décrites dans les articles suivants lorsque X ou Y représentent un groupement $B(OH)_2$ :

- A. Huth, I. Beetz and I. Schumann, Tetrahedron (1989) 45 6679 : Conditions : $Na_2CO_3$ 2M/Pd($P\Phi_3$)$_4$/Toluène/LiCl/EtOH/Δ
- J. K. Stille et al. Ang. Chem. Int. Ed. (1986) 25 508 : Conditions : Pd($P\Phi_3$)$_4$/LiCl/Dioxane/Δ
- T. Oh-e, N. Migawa and A. Suzuki, J. Org. Chem. (1993) 58 2201-2208 : Conditions : $K_3PO_4$/KBr/Pd($P\Phi_3$)$_4$/Dioxane/Δ
- Suzuki et al., Synlett (1992) 208

Conditions : Pd($P\Phi_3$)$_4$/Ba$(OH)_2$/DMEaq ;
ou bien lorsque X ou Y représentent un groupement $SnBu_3$, dans les conditions décrites dans les articles suivants :

- J. K. Still et al, J. Am. Chem. Soc. (1987) 5478-5480 ou par V. Farina, J. Org. Chem. (1993) 58 5434 ;

soit en présence de cuivre dans le cas où :

(c) Y représente un atome d'iode et X représente un atome de chlore,
(d) Y représente un atome de chlore et X représente un atome d'iode,

et ainsi peut s'effectuer dans les conditions décrites dans l'article suivant :

- P. E. Fanta Chem. Rev. (1964) 38 139 ou synthesis (1974) 9 : Conditions : Cu/DMF/120°C ;

soit en présence d'une base forte et de $ZnCl_2$ puis d'un catalyseur choisi parmi les dérivés du palladium dans le cas où :

(e) Y représente un atome de brome et X représente un atome d'hydrogène,
(f) Y représente un atome d'hydrogène et X représente un atome brome,

et ainsi peut s'effectuer dans les conditions suivantes :

1) nBuLi/THF/-78°C
2) ZnCl2
3) $ArBr/Pd(P\Phi_3)_4/\Delta$
4) HCl/MeOH.

Les réactions d'orthométallation sont décrites par exemple dans les documents suivants :
T. KRESS Synthesis (1983) 803,
N. IWAO J. Org. Chem. (1990) 55 3623.

Par ailleurs, la réaction d'échange avec $ZnCl_2$ suivie d'une réaction de couplage a été décrite par E. Negishi dans J. Org. Chem. (1977) 42 182.

La réaction d'oxydation du groupement CHO que peut représenter A' en un groupement $CO_2H$ peut s'effectuer par action du réactif de Jones (acide chromique/acide sulfurique) dans un solvant neutre tel que l'acétone, par action d'oxyde d'argent ($Ag_2O$) dans le tétrahydrofurane et la soude 2N ou par action du chlorite de sodium en présence d'acide aminosulfonique.

La réaction d'oxydation du groupement méthyle que peut représenter A' en un groupement $CO_2H$ peut s'effectuer par action de $KMnO_4$ en milieu basique.

La réaction d'oxydation du groupement $CH_2OH$ que peut représenter A' en un groupement $CO_2H$ peut s'effectuer par action du réactif de Jones dans l'acétone.

La réaction de saponification de la fonction ester que peut représenter A' en l'acide correspondant s'effectue par exemple par action d'une base alcaline telle que la soude ou la potasse dans le tétrahydrofurane ou un alcool inférieur tel que le méthanol ou l'éthanol.

L'action de $Bu_3SnN_3$ puis de HCl lorsque A' représente un groupement cyano, afin d'obtenir le produit de formule (I) dans lesquels A est un tétrazole, s'effectue selon la réaction décrite par Barraclough et al. dans J. Chem. Soc. Perkin I (1989), 1815.

L'action de chlorhydrate de benzylhydroxylamine lorsque A' représente un $CO_2H$, afin d'obtenir Les produits de formule (I) dans lesquels A est un groupement CONHOH, s'effectue en présence d'une base telle que la triéthylamine, d'hydroxybenzotriazole et de dicyclohexyl carbodiimide.

Lorsque Z' est un groupement OH, éventuellement protégé par un groupement protecteur P, P est de préférence un radical alkyle renfermant de 1 à 4 atomes de carbone, un groupement benzyle, un groupement $R_CR_DR_ESi$, dans lequel $R_C$, $R_D$ et $R_E$ identiques ou différents, représentent chacun un radical alkyle renfermant de 1 à 4 atomes de carbone ou un groupement aryle et en particulier phényle.

Par ailleurs, le groupement $CO_2H$ que peut représenter A' peut être estérifié, le groupement formyle que peut représenter A' peut être protégé sous la forme d'un acétal tel que le dioxolane par action du glycol en présence d'acide paratoluène sulfonique, le groupement $CH_2OH$ que peut représenter A' peut être protégé sous la forme d'un groupement tétrahydropyrranyloxy par action du dihydropyrrane.

Les réactions de déprotection sont les méthodes de déprotection classiques connues de l'homme du métier. Une revue assez complète se trouve dans l'ouvrage suivant :
Protective groups in organic synthesis T.W greene, John Wiley & sons (1981).

A titre d'exemple les réactions de déprotection lorsque P est un radical méthyle peuvent s'effectuer par action de tribromoborane dans le dichlorométhane ou d'acide chlorhydrique dans la pyridine, les réactions de déprotection

lorsque P est un groupement benzyle peuvent s'effectuer par action d'hydrogène en présence de palladium sur charbon dans l'acétate d'éthyle ou par action d'acide trifluoroacétique, les réactions de déprotection lorsque P est un groupement tertbutyldiphénylsilyle peuvent s'effectuer par action de fluorure de tétrabutyl d'ammonium en solution dans le tétrahydrofurane.

Lorsque P est un groupement tetrahydropyrannyl, la déprotection s'effectue en présence d'un acide aqueux dans un solvant alcoolique et de préférence par action de l'acide chlorhydrique dans le méthanol.

L'action d'un groupement Hal-$(CH_2)_n$-CO-$NR_aR_b$, Hal représentant un halogène et de préférence le brome, lorsque Z' représente un hydroxyle, s'effectue en présence de soude dans un solvant dipolaire aprotique tel que le diméthylsulfoxyde ou en présence d'hydrure de potassium ou de sodium dans le diméthylformamide ou le diméthylsulfoxyde.

L'action de $Alk_1$-X en présence d'une base forte telle que le lithium diisopropylamide (LDA), lorsque A ou A' représentent un groupement $(CH_2)_n$-CO-$NR_aR_b$ permet d'obtenir les produits de formule (I) dans lesquels A est un groupement $(CH_2)_{n-1}$-CH($Alk_1$)-CO-$NR_aR_b$.

La réaction de réduction du radical $NO_2$ que peuvent représenter $R_1$, $R_2$, $R_3$, $R_4$, $R_5$ ou $R_6$ en radical $NH_2$ peut s'effectuer par action de dichlorure d'étain dans l'éthanol au reflux et la réaction de monoréduction s'effectue de préférence par action du cyclohexène en présence du dihydroxyde de palladium dans l'éthanol au reflux.

La réaction de substitution de $NH_2$ que peuvent représenter $R_1$, $R_2$, $R_3$, $R_4$, $R_5$ ou $R_6$ par le brome s'effectue de préférence par action d'acide bromhydrique en présence de nitrite de sodium et de bromure de cuivre dans l'eau à 0°C.

La réaction de substitution de $NH_2$ que peuvent représenter $R_1$, $R_2$, $R_3$, $R_4$, $R_5$ ou $R_6$ par l'iode s'effectue de préférence par action de iodure de potassium en présence de nitrite de sodium et d'acide sulfurique.

La salification peut être effectuée dans des conditions usuelles. On opère par exemple en présence de soude éthanolique. On peut également utiliser un sel de sodium tel que le carbonate ou le carbonate acide de sodium ou de potassium.

De même, la salification par un acide est réalisée dans les conditions usuelles. On opère par exemple avec l'acide chlorhydrique, par exemple en solution éthérée.

L'invention a plus particulièrement pour objet un procédé de préparation, tel que décrit plus haut, des produits de formule (I) telle que décrite précédemment, caractérisé en ce que l'on soumet un composé de formule ($II_1$) :

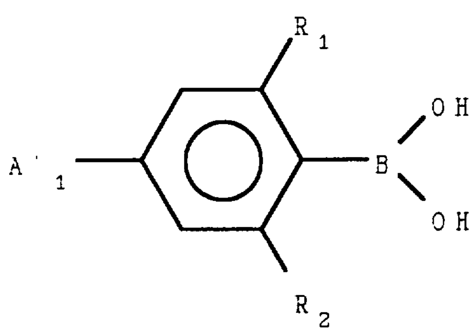

($II_1$)

à l'action, en présence d'un catalyseur choisi parmi les dérivés du Pd, d'un composé de formule générale $(III)_1$ :

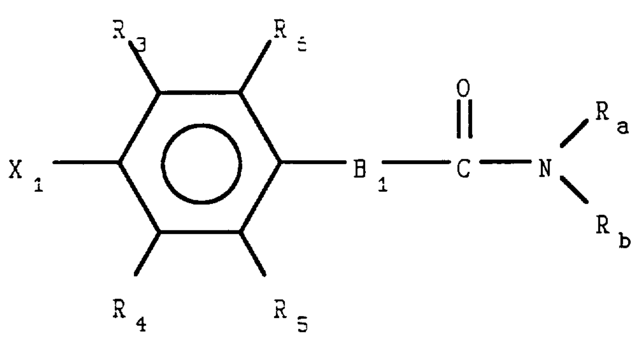

($III_1$)

dans lesquelles $R_a$, $R_b$, $R_1$, $R_2$, $R_3$, $R_4$, $R_5$ ou $R_6$ sont tels que définis plus haut, $A'_1$ représente les groupements formyle, carboxy éventuellement estérifié, $X_1$ représente un groupement $OSO_2CF_3$, un atome de brome ou un atome d'iode et

$B_1$ représente une simple liaison, un groupement $CH_2$ ou un groupement $OCH_2$, afin d'obtenir un produit de formule ($I'_1$) :

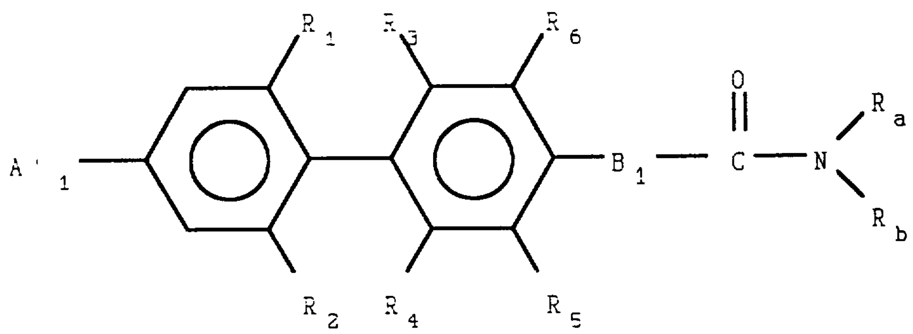

($I'_1$)

dans laquelle $R_a$, $R_b$, $R_1$, $R_2$, $R_3$, $R_4$, $R_5$, $R_6$, $B_1$ et $A'_1$ sont tels que définis précédemment, pouvant, le cas échéant, correspondre à certains produits de formule (I), produit de formule ($I'_1$) que si désiré ou si nécessaire l'on soumet dans un ordre approprié à l'une ou, le cas échéant, plusieurs des réactions suivantes afin d'obtenir le produit de formule (I) :

- oxydation du groupement formyle que peut représenter $A'_1$,
- saponification de l'ester que peut représenter $A'_1$,
- réduction totale ou partielle des groupements $NO_2$ que peuvent représenter $R_1$, $R_2$, $R_3$, $R_4$, $R_5$ ou $R_6$,
- substitution du radical $NH_2$ que peuvent représenter $R_1$, $R_2$, $R_3$, $R_4$, $R_5$, $R_6$ par un atome de brome ou d'iode,
- et salification par un acide ou une base.

Les composés de formule (I) présentent d'intéressantes propriétés pharmacologiques.
Les composés de formule (I) répondent de manière significative comme inhibiteur de l'enzyme testostérone 5-α-réductase, une enzyme clé dans la biosynthèse de certains androgènes, en particulier la 4,5-α-dihydrotestostérone (DHT).
C'est la première fois que l'on détermine que des composés biphényles, donc non stéroïdiens, agissent de manière significative en tant qu'inhibiteur de l'enzyme testostérone 5-α-réductase ce qu'en aucune manière l'art antérieur ne laissait prévoir.
L'art antérieur enseigne notamment qu'en dehors des produits en série stéroïde tels que la Finasteride (MK906) il existe quelques dérivés inhibiteurs de l'enzyme testostérone 5-α-réductase, en série indole ou quinoline.
A titre d'exemple, on peut citer :
En série Quinoline:

la 1,2,3,4,5,6-hexahydrobenzo[f]quinolin-3-one décrite dans la demande de brevet EP-531026,
le Trans-DL-8-chloro-4-méthyl-1,2,3,4,4a,5,,10b-octahydro-benzo[f]quinolin-3-ones (LY191704) décrit dans la demande de brevet EP-532190.

En série Indole :

l'acide 4-[3-[4-[1-(4-isobutylphényl)pentyloxy]benzoyl]indol-1-yl]butyrique décrit dans la demande de brevet WO9305019,
l'acide (R,S)-4-(3-[4-(2-méthylpropyl)phényl]éthoxy)-phényl-éthanoyl]indol-1-yl)butanoique décrit dans la demande de brevet WO9302051.

Les composés de formule (I) présentent une activité spécifique en tant qu'inhibiteur de la 5-α-réductase. La 5-α-réductase est une enzyme responsable de la conversion de la Testostérone en 5α-dihydrotestostérone (DHT), plus affine que la Testostérone pour le récepteur des androgènes (RA).
Cette hormone est essentielle, chez le mâle, au développement in utéro des organes sexuels externes et ensuite dès la puberté, à l'apparition de certaines caractéristiques sexuelles secondaires (système pileux, visage et corps).
On retrouve la 5-α-réductase essentiellement dans la prostate et dans la peau, là où il semble que la Testostérone doive être transformée en DHT pour agir.
Un excès de DHT peut être responsable d'acné ou d'alopécie androgénique, d'hirsutisme chez la femme, et son accumulation au niveau de la prostate, d'hyperplasie bénigne (HBP) ou de cancer de la prostate. On estime que

80 % des hommes de plus de 80 ans présentent une HBP, et 70 % des foyers cancéreux.

L'inhibition de cette étape enzymatique dans le métabolisme stéroïdien entraîne une chute du niveau de DHT in vivo. Cette réduction du niveau de DHT a pour effet de réduire la taille de la prostate chez l'homme et donc a un effet bénéfique dans le traitement de l'hyperplasie bénigne de la prostate, du cancer de la prostate, et de certaines maladies androgéno-dépendantes.

Un traitement inhibant la 5-α-réductase est donc très utile et ne présente pas les inconvénients d'un antiandrogène qui bloque le récepteur.

Cette propriété inhibitrice de la 5-α-réductase rend donc les composés de formule (I) aptes à être utilisés dans le traitement des troubles liés à l'hyperandrogénie, notamment celui de l'hyperplasie bénigne de la prostate, du cancer de la prostate, de l'acné, de l'alopécie androgénique, de la séborrhée, ou encore de l'hirsutisme féminin.

La présente invention a donc pour objet, les composés de formule (I) à titre de médicaments et notamment de médicaments destinés au traitement des troubles liés à l'hyperandrogénie.

La posologie varie en fonction de l'affection à traiter et de la voie d'administration.

Elle peut varier par exemple de 5 à 100 mg/kg et, de préférence, de 10 à 20 mg/kg et par jour chez l'adulte par voie orale.

Les composés de formule (I) peuvent donc être employés pour préparer des compositions pharmaceutiques les contenant à titre de principe actif et l'invention a aussi pour objet lesdites compositions pharmaceutiques.

Les composés de formule (I) selon l'invention peuvent être utilisés par voie digestive, parentérale ou locale, par exemple par voie transdermique. Ils peuvent être prescrits sous forme de comprimés simples ou dragéifiés, de gélules, de granulés, de suppositoires, de préparations injectables, d'ovules, de pommades, de crèmes, de gels, de patchs, lesquels sont préparés selon les méthodes usuelles.

Le principe actif peut y être incorporé à des excipients habituellement employés dans ces compositions pharmaceutiques tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention a également pour objet les composés de formule (I) telle que définie plus haut étant entendu que sont exclus les composés de formule (I) dans laquelle Ba est une simple liaison.

Les produits de formule (II) dans laquelle Y est un groupement $OSO_2CF_3$ ou les produits de formule (III) dans laquelle X est un groupement $OSO_2CF_3$, sont obtenus à partir des alcools correspondants de formule générale (II') ou (III') :

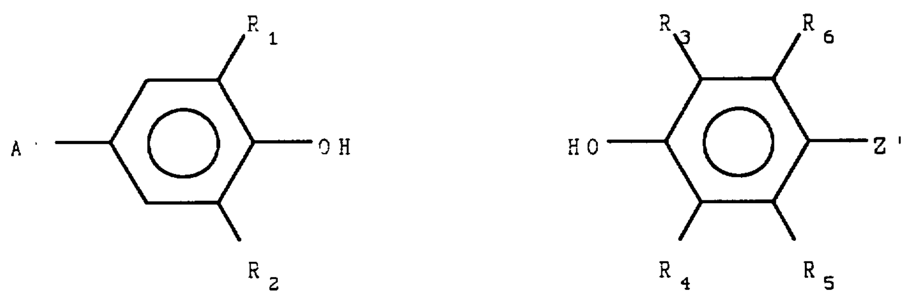

(II') (III')

par action d'anhydride triflique dans la pyridine à 0°C selon la méthode décrite par Scott W. J., Stille J. K., J. Am. Chem. Soc. (1986) 108 3033.

Les produits de formule (II) avec Y représentant un atome d'iode ou les produits de formule (III) avec X représentant un atome d'iode, peuvent être obtenus par orthométallation respectivement à partir produits aromatiques non iodés correspondants de formule générale (II") et (III"), correspondant respectivement aux produits de formule (II) avec Y représentant un atome d'hydrogène et aux produits de formule (III) avec X représentant un atome d'hydrogène :

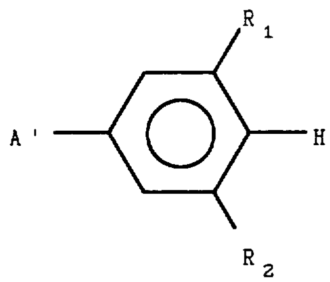

(II")

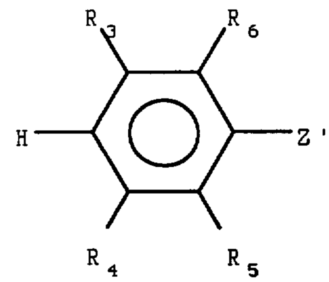

(III")

notamment par action de N-iodosuccinimide ou d'iode en présence d'une base forte telle que le n-Butyllithium dans le tétrahydrofuranne à -78°C.

Les produits de formule (II) avec Y représentant le groupement $B(OH)_2$ ou (III) avec X représentant le groupement $B(OH)_2$, peuvent être obtenus respectivement à partir des produits de formule (II''') ou (III'''), correspondant au produit de formule (II) avec Y représentant un atome de brome et au produit de formule (III) avec X représentant un atome de brome, étant entendu qu'en ce qui concerne le produit de formule (III'''), le groupement OH que peut représenter Z' est protégé comme décrit précédemment,

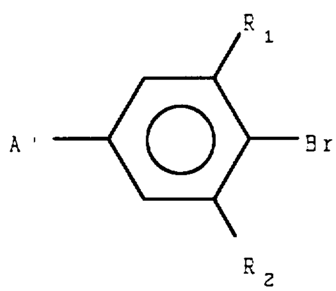

(II"')

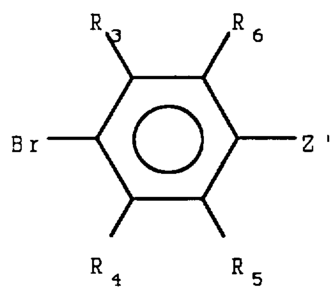

(III"')

notamment par action du magnésium en tournures dans l'éther diéthylique anhydre au reflux, puis par action du triéthylborate dans l'éther diéthylique anhydre à -70°C, puis d'une hydrolyse avec un acide minéral fort tel que l'acide sulfurique ou encore par échange brome-métal au moyen du n butyl lithium puis traitement au trialkylborate et hydrolyse aqueuse.

Ils peuvent également être obtenus par action du produit bromé correspondant, muni des protections nécessaires, en présence de n-butyllithium dans le tétrahydrofuranne à -78°C suivi d'une hydrolyse avec un acide minéral fort tel que l'acide sulfurique ou avec de l'eau.

Les produits de formule (II) avec Y représentant le groupement $SnBu_3$, ou les produits de formule (III) avec X représentant le groupement $SnBu_3$ peuvent être obtenus à partir du produit bromé correspondant (II''') ou (III'''), étant entendu qu'en ce qui concerne le produit de formule (III'''), le groupement OH que peut représenter Z' est protégé comme décrit précédemment, notamment avec le groupement tertbutyldiphénylsilyle, par action avec du chlorure de tributyl stanyle en présence de n-butyllithium dans le tétrahydrofuranne à -78°C.

Les composés de formules générales (III), (III'), (III") et (III''') dans lesquelles Z' est un groupement Ba-CO-$NR_aR_b$ sont préparés :

- soit par action d'un groupement Hal-$(CH2)_n$-CO-$NR_aR_b$ sur un composé de formule générale ($III'_a$) ou ($III_a$) en présence d'une base,
- soit par action d'un groupement H-$NR_aR_b$ en présence de $SOCl_2$ sur un composé de formule générale ($III'_b$) ou ($III_b$),
- soit par action d'un groupement H-$NR_aR_b$ en présence de $SOCl_2$ sur un composé de formule générale ($III'_c$) ou ($III_c$).

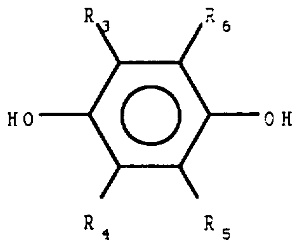

(III'$_a$)

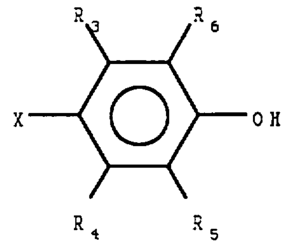

(III$_a$)

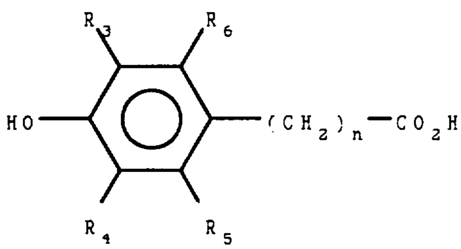

(III'$_b$)

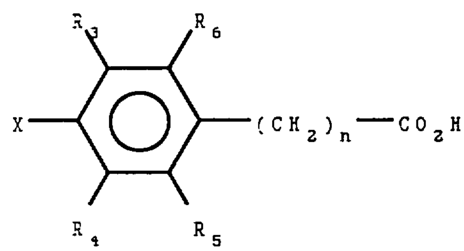

(III$_b$)

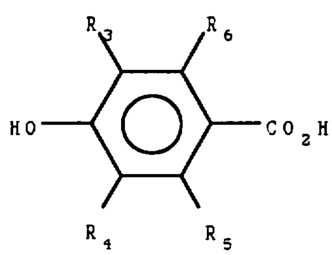

(III'$_c$)

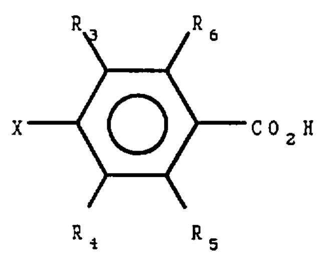

(III$_c$)

$R_1$, $R_2$, $R_3$, $R_4$, $R_5$ ou $R_6$ étant tels que définis précédemment,
X étant un atome d'hydrogène, de brome ou de chlore.

Les produits de formules générales (II), (II'), (II"), (II'"), (III$_a$), (III'$_a$), (III$_b$), (III'$_b$), (III$_c$), (III'$_c$), sont en général connus ou sont accessibles en appliquant les principes généraux de la chimie des composés aromatiques connus de l'homme du métier. On peut citer entre autre la référence suivante : RODD'S CHEMISTRY OF CARBON COMPOUNDS Vol III Aromatic compounds Ed. M. F. ANSELL Elsevier Scientific Publishing Company (1981).

Les produits de formules générales (III), (III'), (III") et (III'") dans lesquelles Z' est un groupement hydroxyle éventuellement protégé, sont également connus ou sont accessibles par l'homme du métier.

Les produits de formules générales (III) et (III'), (III") et (III'") dans lesquelles Z' est un groupement Ba-CO-$NR_aR_b$ et X est un atome d'hydrogène, d'iode, de brome ou de chlore sont également connus ou sont accessibles par l'homme du métier.

Les composés de formule générale Hal-$(CH_2)_nCONR_aR_b$ sont formés par action de $HNR_aR_b$ sur le composé de formule Hal-$(CH_2)_n$-$CO_2H$ en présence d'une base. Hal est de préférence un atome de brome ou chlore et n est de préférence égal à 1.

Les produits de formule générale (I)' dans laquelle Z' est un groupement OH éventuellement protégé sont

connus ou accessibles pour l'homme du métier qui met en oeuvre les réactions de couplage telle que décrite dans les différents documents cités plus haut par la demanderesse.

Les composés de formule (III) dans laquelle Z' est un groupement Ba-CO-$NR_aR_b$ et X est un groupement $OSO_2CF_3$, $B(OH)_2$ ou $SnBu_3$ sont utiles notamment pour la mise en oeuvre du procédé de l'invention. Ils sont nouveaux et font l'objet de la présente invention, étant entendu que les composés de formule (III) dans laquelle Ba est une simple liaison ou un groupement -$CH_2$- sont exclus , de même que les composés de formule (I') dans laquelle Z' est un groupement Ba-CO-$NR_aR_b$ étant entendu que les composés de formule (I') dans laquelle Ba est une simple liaison sont exclus.

Les exemples suivants illustrent l'invention sans toutefois la limiter.

**<u>PREPARATION 1</u> : Acide (4-méthoxyphényl)-boronique**

On ajoute goutte à goutte au reflux 100 ml d'une solution de 10 g de p-bromoanisole dans l'éther diéthylique anhydre à une suspension, sous gaz inerte, de 1,3 g de Mg en tournure dans 5 ml d'éther diéthylique anhydre, et laisse le mélange refluer pendant 2 heures. Le mélange est ensuite versé dans une solution de 9,02 ml de triéthylborate dans 60 ml d'éther anhydre refroidi à -70°C. Après agitation durant 1 heure à -70°C, puis 1 heure à température ambiante, on verse la solution dans un mélange comprenant 11 ml d'acide anhydre à une suspension, sous gaz inerte, de 1,3 g de Mg en tournure dans 5 ml d'éther diéthylique anhydre, et laisse le mélange refluer pendant 2 heures. Le mélange est ensuite versé dans une solution de 9,02 ml de triéthylborate dans 60 ml d'éther anhydre refroidi à -70°C. Après agitation durant 1 heure à -70°C, puis 1 heure à température ambiante, on verse la solution dans un mélange comprenant 11 ml d'acide sulfurique et 50 g de glace et d'eau et agite pendant 1 heure. On extrait la phase organique avec 100 ml d'une solution aqueuse saturée en bicarbonate de soude, réunit les phases aqueuses, les réacidifie avec de l'acide chlorhydrique 6N, extrait avec de l'éther, séche et évapore sous pression réduite. On obtient 3,9 g du produit attendu.

**Spectre I.R.** : (Nujol)

Absorption complexe région OH/NH , 1609, 1573 et 1518 $cm^{-1}$

| **RMN** : DMSO-$d_6$, 300MHz | |
|---|---|
| 3,76 s | C<u>H</u>$_3$O |
| 6,88 (d) J=9Hz | $H_3$ et $H_5$ |
| 7,78 (d) J=9Hz | $H_2$ et $H_6$ |
| 7,86 | $B(OH)_2$ |

**<u>PREPARATION 2</u> : Acide [4-(phénylméthoxy)-phényl]-boronique**

**<u>Stade A</u> : 1-bromo-4-(phénylméthoxy)-benzène**

On ajoute, à 0°C, 15,26 g d'hydrure de sodium à 50 % dans l'huile à une solution sous gaz inerte de 50 g de parabromophénol dans 320 ml de diméthylformamide, agite pendant 30 minute à 0°C, puis ajoute 37,7 ml de bromure de benzyle. On agite pendant 2 heures 30 en laissant remonter la température à 20°C, puis coule le mélange réactionnel sur de l'eau glacée, filtre le précipité, et séche. On obtient 73,35 g de produit attendu.

Rf : 0,85 (chromatographie sur couche mince, support : silice, éluant : cyclohexane/acétate d'éthyle 7/3).

**Spectre I.R.** : ($CHCl_3$)

Absence d'OH

Aromatique 1592, 1580 et 1488 $cm^{-1}$

**<u>Stade B</u> : Acide [4-(phénylméthoxy)-phényl]-boronique**

On ajoute, goutte à goutte, sous gaz inerte et à -78°C, 143 ml d'une solution de n-Butyllithium à 47,08 g du produit obtenu au stade A dans 375 ml de tétrahydrofurane, agite 1 heure à -78°C, puis ajoute 36,5 ml de triéthylborate. On agite 1 nuit, en laissant remonter la température à 20°C, et on hydrolyse le milieu réactionnel au moyen d'une solution d'eau glacée contenant 45 ml d'acide sulfurique concentré, pendant 1 heure à 20°C. La phase aqueuse est extraite avec de l'acétate d'éthyle, les phases organiques sont lavées par de la soude 2N et la phase aqueuse est acidifiée à pH=1 à l'aide d'une solution d'acide chlorhydrique 1N afin de précipiter l'acide boronique. Après filtration et séchage du précipité on obtient 28,54 g du produit attendu.

Rf: 0,16 (chromatographie sur couche mince, support : silice, éluant : cyclohexane/acétace d'éthyle 7/3)

| **Spectre I.R.** : (Nujol) | |
|---|---|
| Absorption générale région OH/NH<br>Aromatique<br>B-O | 3650, 3615, 3510 et 3420 $cm^{-1}$<br>1605, 1570 et 1510 $cm^{-1}$<br>1410, 1340 $cm^{-1}$ |

**PREPARATION 3 : Acide 4-[[(1,1-diméthyléthyl)diphénylsilyl) oxy]phényl]-boronique.**

**Stade A : 1-Bromo-4-[[(1,1-diméthyléthyl)diphénylsilyl]oxy]-benzène.**

On agite 2 heures à température ambiante 80,89 g de parabromophénol, 400 ml de diméthylformamide, 31,18 g d'imidazole et 125,89 g de 1,1-diméthyl-éthyl-diphényl-chlorosilane. On verse dans 2 litres d'eau, extrait avec de l'acétate d'éthyle, décante, sèche et évapore sous pression réduite. Après cristallisation dans le pentane on essore, sèche et obtient 179,24 g du produit attendu.
Rf : 0,53 (chromatographie sur couche mince, support : silice, éluant Cyclohexane/AcOEt 95/5).
Point de fusion : 56°C

| **RMN** ($CDCl_3$, 300MHz) | |
|---|---|
| 1,09 (s)<br>6,63 (m)<br>7,17 (m)<br>7,69 (dd)<br>7,4 | SitBu<br>$H_3$, $H_5$<br>$H_2$, $H_6$<br>4H pour SiΦ2<br>6H pour SiΦ2 |

**Stade B : Acide 4-[[(1,1-diméthyléthyl)diphénylsilyl)oxy]-phényl]-boronique**

A une solution de 30 g du produit du stade précédent dans 100 ml de tétrahydrofurane anhydre, on ajoute, goutte à goutte, à -78°C et sous gaz inerte, 60 ml d'une solution de n-butyl-lithium (1,24M) dans l'hexane puis après agitation durant 30 minutes à -78°C puis ajoute goutte à goutte, 9,95 ml de triméthylborate. Après 2 heures 30 d'agitation, en laissant la température du bain évoluer jusqu'à 12°C, on introduit goutte à goutte 20 ml d'eau et agite pendant 72 heures à température ambiante. Après évaporation sous pression réduite du tétrahydrofurane, on extrait la phase aqueuse à l'éther, sèche et concentre sous pression réduite jusqu'à obtention d'une huile (26,35 g) que l'on purifie par chromatographie filtrante sur silice avec le mélange hexane/acétate d'éthyle 1/1 pour obtenir 7,73 g du produit attendu sous forme de trimère et de monomère.

| **IR** ($CHCl_3$) | |
|---|---|
| OSi<br>B-O<br>Aromatiques | 915 et 1255 $cm^{-1}$<br>1350 et 1370 $cm^{-1}$<br>1515, 1570 et 1602 $cm^{-1}$ |

| **RMN** ($CDCl_3$) | |
|---|---|
| 1,11<br>6,81 et 7,88<br>7,3 à 7,5 (6H) et 7,72 (4H) | tBu<br>(Ph-O)<br>(PhSi) |

**PREPARATION 4 : [4-[[(1,1-diméthyléthyl)diphénylsilyl]oxy] phényl]-tributylétain**

On ajoute, goutte à goutte, à -50°C et sous gaz inerte, 123,89 ml d'une solution de sec-butyl-lithium à une solution de 50 g du produit obtenu au stade A de la préparation 3 dans 300 ml de tétrahydrofurane anhydre, puis après agitation durant 1 heure à -50°C, ajoute 36,29 ml de chlorure de tributyl étain. Après 30 minutes d'agitation, la température ayant évolué jusqu'à température ambiante, on verse le mélange réactionnel dans de l'eau glacée, extrait avec de l'acétate d'éthyle, sèche et évapore à sec sous pression réduite. On obtient 38,5 g du produit attendu sous forme d'une huile ($T_{eb}$: 230°C sous $10^{-2}$ mbar)

Rf : 0,36 (chromatographie sur couche mince, support : silice, éluant cyclohexane).
**IR** ($CHCl_3$)
Aromatique 1569, 1583, 1510, 1493 $cm^{-1}$

**PREPARATION 5 : Acide (4-formylphényl)-boronique**

Ce produit est préparé selon la méthode décrite dans la demande de brevet Européenne EP 0606065.

**PREPARATION 6 :Acide [4-(hydroxyméthyl)phényl] boronique**

On ajoute à une solution de 1,5 g d'acide (4-formyl phényl) boronique dans 40 ml de méthanol à 0°C et sous atmosphère inerte 380 mg de borohydrure de sodium puis après 30 minutes d'agitation 200 ml d'acide chlorhydrique 2N. Après évaporation sous pression réduite, on extrait le brut obtenu à l'acétate d'éthyle, sèche la phase organique sur sulfate de magnésium, filtre et évapore sous pression réduite jusqu'à obtention de 1,5 g du produit attendu. (Rf=0,15 cyclohexane/acétate d'éthyle 50/50)

| IR ($CHCl_3$) | |
|---|---|
| Aromatiques | 1614, 1565, 1518$cm^{-1}$ |

| RMN ($CDCl_3$) | |
|---|---|
| 4,50 (d)<br>7,92 (s)<br>7,26 et 7,74<br>5,14 (t) | $C\underline{H}_2OH$<br>$B(O\underline{H})_2$<br>H aromatiques<br>$CH_2O\underline{H}$ |

**PREPARATION 7 : 4-iodo-benzoate de méthyle**

A une suspension de 10 g d'acide 4-iodo-benzoïque dans 100 ml de méthanol, on ajoute 2,95 ml de $SOCl_2$, chauffe au reflux pendant 24 heures puis évapore sous pression réduite jusqu'à obtention de 10,49 g d'un extrait sec correspondant au produit attendu (Rf 0,55 cyclohexane/acétate d'éthyle 80/20).

| IR ($CHCl_3$) | |
|---|---|
| CO ester<br>Aromatique | 1724 $cm^{-1}$<br>1588 $cm^{-1}$ |

**PREPARATION 8 : N,N-bis(1-méthyléthyl)-2-bromo-acétamide**

On ajoute goutte à goutte, sous atmosphère inerte et à -40°C 12,95 ml de bromure de bromoacétyle dans 160 ml d'éther à une solution de 52 ml de diisopropylamine dans 200 ml d'éther puis on agite 1 heure à -40°C. Après filtration du précipité le filtrat est évaporé sous pression réduite et chromatographié sur silice en éluant avec le mélange acétate d'éthyle/ cyclohexane 9/1). On obtient 12,92 g d'amide attendue sous forme d'une huile.

| RMN ($CDCl_3$) | |
|---|---|
| 1,26 (d) 1,39 (d)<br>3,43 et 3,96 (m)<br>3,8 (s) | $CH(C\underline{H}_3)_2$<br>$C\underline{H}(CH_3)_2$<br>$Br-C\underline{H}_2-CO$ |

IR ($CHCl_3$)
1641, 1633 $cm^{-1}$

**PREPARATION 9 : 2-bromo-N-(triphénylméthyl) acetamide**

On ajoute goutte à goutte, sous atmosphère inerte et à 0°C 3,22 ml de bromure de bromoacétyle à 19,23 g

de tritylamine dans 50 ml de Chloroforme puis on agite 1 heure en laissant remonter la température à l'ambiante. Après filtration du précipité, la phase organique est lavée avec de la soude 1N, séchée, évaporée sous pression réduite jusqu'à obtention d'un résidu qui est repurifié par cristallisation dans de l'éther. On obtient 8,5 g d'amide attendue.

| RMN ($CDCl_3$) | |
|---|---|
| 3,9 (s)<br>7,18 à 7,34 (m)<br>7,70 (s, large) | Br-C$\underline{H}_2$-CO<br>H aromatiques<br>NH |

IR ($CHCl_3$)
3408, 1684, 1598, 1585, 1514, 1496 $cm^{-1}$

**<u>Exemple 1</u> : Acide 4'-[[bis(1-méthyléthyl)amino]carbonyl]-(1,1'-biphényl)-4-carboxylique.**

<u>Stade A</u> : N,N-bis(1-méthyléthyl)-4-hydroxy-benzamide.

On additionnne, sous atmosphère inerte, goutte à goutte 5,92 ml de chlorure de thionyle puis 4 gouttes de diméthylformamide à une suspension de 5,6 g d'acide 4-hydroxybenzoïque dans 100 ml de dichlorométhane et on porte au reflux pendant 50 minutes, concentre à sec sous pression réduite, ajoute 100 ml de dichlorométhane, puis en refroidissant à l'aide d'un bain glacé. On additionne ensuite goutte à goutte 28,36 ml de diisopropylamine et laisse 1 heure à température ambiante. On lave avec de l'acide chlorhydrique 2N, puis on concentre sous pression réduite la phase organique, rajoute 100 ml de méthanol, 10 ml de soude concentrée (32 %), agite 30 minutes et évapore sous pression réduite afin d'éliminer le méthanol et permettre au produit de cristalliser. Après filtration on obtient 7,15 g de l'amide attendue.

| RMN ($CDCl_3$, 250MHz) | |
|---|---|
| 1,6<br>3,74<br>6,80 et 7,16 (AA'BB')<br>8,92 (s) | CH(C$\underline{H}_3$)$_2$<br>C$\underline{H}$(CH$_3$)$_2$<br>H aromatiques<br>OH |

IR (Nujol)
3240, 1609, 1588 et 1520 $cm^{-1}$

<u>Stade B</u> : Trifluorométhanesulfonate de 4-[[bis(1-méthyléthyl)amino)carbonyl]phényle.

On additionne sous atmosphère inerte, goutte à goutte 3,9 ml d'anhydride triflique à une solution de 3,4 g du produit préparé au stade A dans 30 ml de pyridine refroidie à 0°C, agite 1 heure à cette température et verse le mélange dans environ 150 ml d'eau glacée. Le produit cristallisé est filtré puis séché. On obtient 5,07 g de triflate attendu sous forme d'une poudre blanche.

| IR ($CHCl_3$) | |
|---|---|
| 1427 et 1140 $cm^{-1}$<br>1627 $cm^{-1}$<br>1603 et 1502 $cm^{-1}$ | (OTf)<br>(CO)<br>(aromatiques) |

<u>Stade C</u> : N,N-bis(1-méthyléthyl)-4'-méthyle-(1,1'-biphényl)-4-carboxamide.

A une solution sous atmosphère inerte de 700 mg de produit préparé au stade précédent dans 18 ml de toluène, on additionne successivement 76 mg de tetrakis triphénylphosphine palladium, 2,6 ml d'une solution de carbonate de sodium 2M, 8 ml d'éthanol 99 %, 298 mg d'acide 4-Méthylphénylboronique (Commercial : LANCASTER) et 170 mg de chlorure de lithium anhydre, porte l'ensemble à 95°C pendant 3 heures, additionne 20 ml d'eau distillée, lave la solution organique avec une solution saturée en chlorure d'ammonium, sèche sur sulfate de magnésium et évapore sous pression réduite. On obtient une huile marron qui cristallise et on purifie par chromatographie avec comme éluant un mélange acétate d'éthyle-cyclohexane 1-1 pour obtenir 594 mg de produit attendu.

| RMN ($CDCl_3$, 250MHz) | |
|---|---|
| 1,35 (m large) | CH(C<u>H</u>$_3$)$_2$ |
| 3,71 (m large) | C<u>H</u>(CH$_3$)$_2$ |
| 2,4 (s) | C<u>H</u>$_3$Ph |
| 7,25 et 7,59 (AA'BB') | Ar |
| 7,37 et 7,49 (AA'BB') | Ar |

Stade D : Acide 4'-[[bis(1-méthyléthyl)amino]carbonyl]-(1,1'-biphényl)-4-carboxylique.

A une solution de 293 mg de biphényle préparé au stade précédent dans 1 ml de pyridine et 4 ml d'une solution aqueuse de KOH 20 % à 95°C-100°C on additionne 466 mg de permanganate de potassium et maintient à cette température pendant 8 heures. Après refroidissement, et élimination par filtration d'un léger précipité, on acidifie le filtrat avec une solution d'acide chlorhydrique 2N. Le précipité obtenu est lavé à l'eau puis sèché. On obtient ainsi, 234 mg du produit attendu.

| RMN (DMSO) | |
|---|---|
| 1,08 à 1,6 | CH(C<u>H</u>$_3$)$_2$ |
| 3,76 (m large) | C<u>H</u>(CH$_3$)$_2$ |
| 7,40 et 7,78 | Ar |
| 7,83 et 8,04 | Ar |
| 13,02 | COO<u>H</u> |

IR (Nujol)
1688, 1624, 1606, 1572 $cm^{-1}$

**Exemple 2 : Acide 4'-[2-(bis(1-méthyléthyl)amino]-2-oxoéthoxy)-(1,1'-biphényl)-4-carboxylique.**

Stade A : N,N-bis(1-méthyléthyl)-2-(4-iodophénoxy)-acetamide

A une solution de 5,2 g de paraiodophénol dans 30 ml de tétrahydrofuranne on additionne sous atmosphère inerte 1,38 g d'hydrure de sodium à 50 % (en suspension dans l'huile), agite à 0°C puis ajoute 6,30 g de N,N-bis (1-méthyléthyl)-2-bromo-acétamide (preparation 8). Après agitation pendant 2 heures en laissant remonter la température à 20°C, on verse le milieu réactionnel sur de l'eau glacée, filtre puis sèche pour obtenir 9,3 g de produit attendu.

| RMN ($CDCl_3$, 250MHz) | |
|---|---|
| 1,20 (d) et 1,39 (d) | CH(C<u>H</u>$_3$)$_2$ |
| 3,42 (m) et 4,03(m) | C<u>H</u>(CH$_3$)$_2$ |
| 4,58 (s) | CO-C<u>H</u>$_2$-O |
| 6,73 et 7,55 | Ar |

| IR ($CHCl_3$) | |
|---|---|
| Absence d'OH (CO) | 1638 $cm^{-1}$ |
| Aromatique | 1586, 1576 et 1486 $cm^{-1}$ |

Stade B : N,N-bis(1-méthyléthyl)-2-[[4'-formyl-(1,1'-biphényl)-4-yl]oxy]-acetamide

A une solution sous atmosphère inerte de 3,14 g de produit préparé au stade précédent dans 40 ml de dioxane, on additionne successivement 0,5 g de tetrakis triphénylphosphine palladium, 3,0 g de tri-potassium phosphate 1-hydrate, 1,43 g d'acide 4-formylphénylboronique (préparation 5), porte l'ensemble au reflux pendant 2 heures, puis verse le mélange réactionnel dans de l'eau glacée. La phase organique est séchée sur sulfate de magnésium puis concentrée sous pression réduite. On obtient 3,94 g de produit que l'on purifie par chromatographie en éluant avec un mélange

acétate d'éthyle/cyclohexane 2/8 pour obtenir 833 mg de produit attendu.

| RMN ($CDCl_3$, 250MHz) | |
|---|---|
| 1,24 (d) et 1,43 (d) | CH(C<u>H</u>3)2) |
| 3,46 (m) et 4,09 (m) | C<u>H</u>($CH_3$)$_2$ |
| 4,69 (s) | OC<u>H</u>$_2$CO |
| 7,06 (d) et 7,59 (d) | H aromatique (Ar-O) |
| 7,71 (d) et 7,93 (d) | H aromatique (Ar-CHO) |
| 10,04 (s) | C<u>H</u>O |

<u>Stade C</u> : Acide 4'-[2-[bis(1-méthyléthyl)amino]-2-oxoéthoxy)-(1,1'-biphényl)-4-carboxylique.

A une solution sous atmosphère inerte de 741 mg du produit préparé au stade précédent dans 10 ml d'acétone, on introduit 0,85 ml de réactif de Jones, agite pendant 7 heures et filtre le précipité formé que l'on sèche. On obtient ainsi 620 mg de de produit attendu.
Rf = 0,5 (dichlorométhane/méthanol 9/1)

| RMN ($CDCl_3$, 250MHz) | |
|---|---|
| 1,18 (d) et 1,31 (d) | CH(C<u>H</u>$_3$)$_2$ |
| 3,48 (m) et 3,99 (m) | C<u>H</u>($CH_3$)$_2$ |
| 4,77 (s) | OC<u>H</u>$_2$CO |
| 7,02 (d) et 7,68 (d) | H aromatique (Ar-O) |
| 7,75 (d) et 7,99 (d) | H aromatique (Ar-COOH) |
| 12,85 (étalé) | $CO_2$<u>H</u> |

| IR ($CHCl_3$) | |
|---|---|
| Absorption région OH/NH | |
| 1712 $cm^{-1}$ | (CO) |
| 1622 $cm^{-1}$ | (CO) + |
| 1606, 1587, 1530 et 1498 $cm^{-1}$ | (aromatiques) |

| Microanalyse | | | |
|---|---|---|---|
| % calculé | C 70,96 | H 7,09 | H 3,94 |
| % trouvé | C 70,9 | H 7,2 | H 3,9 |

**<u>Exemple 3 :</u> Acide 4'-[2-[bis(1-méthyléthyl)amino]-2-oxoéthoxy]-3',5'-bis(1-méthyléthyl)-(1,1'-biphényl)-4-carboxylique.**

On opère de la même façon qu'à l'exemple 2 stades A et B en partant de 2 g de 2',6'-diisopropyl-4-bromophénol et l'oxydation du groupement formyle (stade C) s'effectue avec $Ag_2O$ comme décrit plus loin dans l'exemple 10 stade B. On obtient ainsi 54 mg de produit attendu
(Rf=0,23 (dichlorométhane-Méthanol 9-1))
IR ($CHCl_3$)
présence d'acide jugé d'après la région OH
Carbonyle 1728, 1691, 1655, 1639 cm-1
Aromatique 1609, 1565, 1515 cm-1

| RMN ($CDCl_3$, 300MHz) | |
|---|---|
| 1,30 (d) 1,49(d) | CH(C<u>H</u>$_3$)$_2$ |
| 3,54 (ml) et 4,10(ml) | C<u>H</u>($CH_3$)$_2$ |

(suite)

| RMN ($CDCl_3$, 300MHz) | |
|---|---|
| 3,39 (m) | Ph-C<u>H</u>($CH_3$)$_2$ |
| 4,45 (s) | O-C<u>H</u>$_2$-CO |
| 7,34 (s) | H'$_2$, H'$_6$ aromatiques (Ar-O) |
| 7,64 (d) 8,05 (d) | H aromatiques (Ar-COOH) |

**Exemple 4 : Acide 4'-[2-[bis(1-méthyléthyl)amino]-2-oxoéthoxy]-3'-fluoro-5'-nitro-(1,1'-biphényl)-4-carboxylique.**

On opère de la même façon qu'à l'exemple 2 stades A, B et C en partant de 5 g de 2-fluoro-4-bromo-6-nitrophénol. On obtient 1,8 g de produit attendu.
F° = 202°C

| IR ($CHCl_3$) | |
|---|---|
| présence d'acide jugé d'après la région OH | |
| Carbonyle | 1703, 1648 cm-1 |
| Aromatique | 1611, 1569, 1543, 1518 cm-1 |

| RMN ($CDCl_3$, 250MHz) | |
|---|---|
| 1,16 (d) 1,27(d) | CH(C<u>H</u>$_3$)$_2$ |
| 3,48 (ml) et 3,77(ml) | C<u>H</u>($CH_3$)$_2$ |
| 5,05 (s) et 5,06 (s) | O-C<u>H</u>$_2$-CO |
| 7,95 à 8,10 | H'$_2$, H'$_6$ aromatiques (Ar-O) |
| 7,88 (m) 8,03 (m) (AA'BB') | H aromatiques (Ar-COOH) |

**Exemple 5 : Acide 4'-[2-oxo-2-([triphénylméthyl)amino] éthoxy]-(1,1'-biphényl)-4-carboxylique.**

Stade A : 2-(4-bromophénoxy)-N-(triphénylméthyl)-acetamide

En suivant la même méthode qu'à l'exemple 2 stade A, mais à partir de 560 mg de parabromophénol et de 1,3 g de 2-bromo-N-(triphénylméthyl)acetamide (préparation 9), on obtient 1,81 g de produit attendu.

| IR ($CHCl_3$) | |
|---|---|
| Absence d'OH | |
| =C-NH | 3420 $cm^{-1}$ |
| C=O | 1689 $cm^{-1}$ |
| Amide, aromatique : | 1592, 1585, 1510, et 1490$cm^{-1}$ |

| RMN ($CDCl_3$) 300MHz | |
|---|---|
| 4,47 (s) | O-C<u>H</u>$_2$-CO |
| 6,79 et 7,41 (AA' BB') : | 4H aromatiques |
| 7,15 à 7,32 | trityl |
| 7,74 (sl) | N<u>H</u>CO |

Stade B : 4'[[4'-formyl(1,1'-biphényl)-4-yl]oxy])-N-(triphénylméthyl)-acetamide.

En suivant la même méthode qu'à l'exemple 2 stade B (en ajoutant 1,1 équivalent de bromure de potassium), à partir de 1,5 g de produit précédent et 525 mg d'acide boronique on obtient 160 mg de produit attendu pur :

| RMN ($CDCl_3$) 300MHz | |
|---|---|
| 4,56 (s) | O-C<u>H</u>$_2$-CO |
| 7,71 et 7,95 (AA' BB') : | 4H aromatiques (Ar-CHO) |
| 7,03 et 7,61 (AA' BB') : | 4H aromatiques (Ar-O) |
| 7,20 à 7,33 (m) | trityl |
| 7,83 (sl) | NHCO |
| 10,05 | CHO |
| 4,56 (s) | O-$CH_2$-CO |

Stade C : Acide 4'-[2-oxo-2-([triphénylméthyl)amino]éthoxy]-(1,1'-biphényl)-4-carboxylique.

En suivant la même méthode qu'à l'exemple 3 stade C, mais à partir de 270 mg de produit précédent on obtient 60 mg du produit attendu :
Rf=0,43 (dichlorométhane/méthanol 98/02)

| IR ($CHCl_3$) | |
|---|---|
| Absorption générale OH/NH | |
| C=O | 1692, 1672 $cm^{-1}$ |
| aromatique + Amide II: | 1608, 1585, 1525, et 1492$cm^{-1}$ |

| RMN ($CDCl_3$) 300MHz | |
|---|---|
| 4,76 (s) | O-$CH_2$-CO |
| 6,96 et 7,65 (AA' BB') : | 4H aromatiques Ar-O |
| 7,75 et 7,99 (AA' BB') : | 4H aromatiques ArCOOH |
| 7,18 à 7,35 (m) | trityl |
| 8,72 (s) | NHCO |
| 12,95 | $CO_2H$ |

**Exemple 6 : Acide 3'-fluoro-5'-nitro- 4'-[2-oxo-2-[(triphénylméthyl) amino]éthoxy] - (1,1'-biphényl) -4-carboxylique.**

En suivant la même méthode qu'à l'exemple 5 stades A et B en partant de 0,638 g de 2-fluoro-4-bromo-6-nitro-phénol, et l'oxydation du groupement formyle (stade C) s'effectuant avec NaClO/$H_2NSO_3H$ comme décrit plus loin dans l'exemple 9 stade C. On obtient 43 mg de produit attendu après recristallisation dans le chloroforme.
F° = 236-7°C

| IR ($CHCl_3$) | |
|---|---|
| NH | 3410 $cm^{-1}$ |
| C=O | 1730, 1695 $cm^{-1}$ |
| Amide II, aromatique, $NO_2$ | 1613, 1583, 1566,1545,1516 et 1492 $cm^{-1}$ |

| RMN ($CDCl_3$) 300MHz | |
|---|---|
| 4,78 (d) | O-$CH_2$-CO |
| 8,01 (m) : | $H'_2$ Ar-O |
| 7,69 (dd) : | $H'_6$ Ar-O |
| 7,68 et 8,23 (AA' BB') : | 4H aromatiques ArCOOH |
| 7,31 (m) | trityl |
| 8,06 (sl) | NHCO |
| 12,95 | $CO_2H$ |

**Exemple 7 : Acide 4'-[2-[bis(1-méthyléthyl)amino]-2-oxoéthoxy]-2',3'-dicyano-(1,1'-biphényl)-4-carboxylique.**

Stade A : N,N-bis(1-méthyléthyl)-2-(2,3-dicyano-4-hydroxyphénoxy)-acetamide

On ajoute à une solution de 1,6 g de 2,3-dicyano-4-hydroxyphénol dans 100 ml de diméthylformamide 960 mg d'hydrure de sodium (50 % dans l'huile), agite 30 minutes à température ambiante puis ajoute 2,3 g de N,N-bis (1-méthyléthyl)-2-bromoacétamide (préparation 8). Après avoir agité pendant 48 heures, on évapore sous pression réduite le solvant, ajoute 200 ml d'acide chlorhydrique 2N, extrait à l'acétate d'éthyle, sèche la phase organique sur sulfate de magnésium, filtre et évapore sous pression réduite. On chromatographie le résidu en éluant avec le mélange cyclohexane-acétate d'éthyle 50/50, puis l'acétate d'éthyle pur, et enfin le mélange acétate d'éthyle-méthanol 98-2. On obtient 1,8 g du produit attendu que l'on utilise tel quel pour le stade suivant.

Stade B : Trifluorométhanesulfonate de [4-[2-[bis(1-méthyléthyl)amino]-2-oxoéthoxy]-2,3-dicyanophényle].

A une solution de 900 mg du produit obtenu au stade A dans 50ml d'acétonitrile, on ajoute 0,58 ml d'éthyl diisopropylamine, 0,59 ml d'anhydride triflique, porte au reflux pendant 1 heure puis évapore le solvant sous pression réduite. Le produit brut obtenu est chromatographié sur silice en éluant avec un mélange cyclohexane-acétate d'éthyle 50-50. On obtient ainsi 600 mg du produit attendu.

| IR ($CHCl_3$) | |
|---|---|
| Nitrile<br>Carbonyle<br>Aromatique | 2245 $cm^{-1}$<br>1656, 1644 $cm^{-1}$<br>1594 $cm^{-1}$ |

| RMN ($CDCl_3$, 250MHz) | |
|---|---|
| 1,28 (d) 1,38(d)<br>3,49 (m) 3,94 (m)<br>4,90 (s)<br>7,38 (d) 7,62 (d) | CH(C$\underline{H}_3$)$_2$<br>C$\underline{H}$(CH$_3$)$_2$<br>O-C$\underline{H}_2$-CO<br>H aromatiques |

Stade C : N,N-bis(1-méthyléthyl)-2-[[2,3-dicyano-4'-(hydroxyméthyl)-(1,1'-biphényl)-4-yl]oxy]-acetamide.

On mélange sous atmosphère inerte 1,1 g du produit préparé au stade B, 875 mg de Tripotassium phosphate monohydrate, 330 mg de bromure de potassium, 75 mg de Palladium tétrakis, 425 mg d'acide boronique (préparation 6), 50 ml de dioxane et agite 16 h au reflux. Après évaporation sous pression réduite, on chromatographie sur silice en éluant avec un mélange 50-50 de cyclohexane-acétate d'éthyle puis de l'acétate d'éthyle. On obtient 140 mg de produit attendu.

| IR (CHCl3) | |
|---|---|
| Nitrile<br>Alcool<br>Carbonyle<br>Aromatique | 2240 $cm^{-1}$<br>3612 $cm^{-1}$<br>1655, 1642 $cm^{-1}$<br>1595, 1562 $cm^{-1}$ |

| RMN ($CDCl_3$, 250MHz) | |
|---|---|
| 1,28-1,39(d)<br>1,84<br>3,48-4,08 (m)<br>4,88 (s)<br>7,39-7,64 (d)<br>7,5 (s) | CH(C$\underline{H}_3$)$_2$<br>OH<br>C$\underline{H}$(CH$_3$)$_2$<br>CO-C$\underline{H}_2$-O<br>H aromatiques Ar-O<br>H aromatiques Ar-CH$_2$OH |

(suite)

| RMN ($CDCl_3$, 250MHz) | |
|---|---|
| 4,78 (d) | C_H_$_2$-OH |

Stade D : Acide 4'-[2-[bis(1-méthyléthyl)amino]-2-oxoéthoxy]-2',3'-dicyano-(1,1'-biphényl)-4-carboxylique.

On mélange sous atmosphère inerte, pendant 15 minutes 30 mg du produit préparé au stade précédent, 0,1 ml de réactif de Jones et 10 ml d'acétone puis on coule dans l'eau. Le solide est filtré, séché puis recristallisé dans 2 ml d'acétate d'éthyle. On obtient 12 mg du produit attendu (Rf=0,2 dans l'acétate d'éthyle).

| IR ($CHCl_3$) | |
|---|---|
| Nitrile<br>Carbonyle<br>Aromatique | 2240 cm-1<br>1700, 1656 cm-1<br>1612, 1595, 1558, 1515 cm-1 |

| RMN ($CDCl_3$, 250MHz) | |
|---|---|
| 1,25 (d) 1,39(d) | CH(C_H_$_3$)$_2$ |
| 3,48 (m) 4,07 (m) | C_H_(CH$_3$)$_2$ |
| 4,93 (sl) | O-C_H_$_2$-CO |
| 7,44 (d) 7,68 (d) | H aromatiques (Ar-O) |
| 7,62 (d) 8,22 (d) | H aromatiques (Ar-COOH) |

**Exemple 8 : Acide 4-[4-[2-[bis(1-méthyléthyl)amino]-2-oxoéthoxy]-1-naphtalenyl]-benzoïque.**

En suivant la même méthode qu'à l'exemple 7 stades A, B, C et D en partant de 3,2 g de 1,4-dihydroxy naphtalène. On obtient 20 mg du produit attendu.
(Rf=0,14 (Cyclohexane-acétate d'éthyle 50-50))

| IR ($CHCl_3$) | |
|---|---|
| Carbonyle<br>Aromatique | 1725, 1692, 1653, 1638 $cm^{-1}$<br>1609, 1596, 1585, 1564, 1510, 1507 $cm^{-1}$ |

| RMN ($CDCl_3$, 250MHz) | |
|---|---|
| 1,24 (d) 1,47(d) | CH(C_H_$_3$)$_2$ |
| 3,48 (m) 4,25 (m) | C_H_(CH$_3$)$_2$ |
| 4,87 (s) | O-C_H_$_2$-CO |
| 6,97 (d) J=8 | |
| 7,34 (d) J=8 | |
| 7,84 (m) | H aromatique du naphtalène |
| 8,41 (m) | |
| 7,50 (m) | |
| 7,59-8,22 | H aromatiques Ar-$CO_2H$ |

**Exemple 9 : Acide 4'-[2-[bis(1-méthyléthyl)amino]-2-oxoéthyl)-(1,1'-biphényl)-4-carboxylique**

Stade A : N,N-bis(1-méthyléthyl)-4-bromo-benzeneacetamide.

On ajoute 4,21 g de 1-éthyl 3-(3-diméthylamino propyl) carbodiimide (EDAC) et 3,08 ml de diisopropylamine à une solution de 4,3 g d'acide 4-bromo-benzeacetique ($BrPhCH_2COOH$) dans 15 ml de dichlorométhane et agite 10 minutes à température ambiante. Après évaporation sous pression réduite, le produit brut obtenu est chromatographié

sur silice en éluant avec le mélange acétate d'éthyle/cyclohexane 2/8. On obtient ainsi 3,7 g de produit sous forme d'huile.
(Rf : 0,21 acétate d'éthyle/cyclohexane 2/8)

Stade B : N,N-bis(1-méthyléthyl)-4'-formyl-(1,1'-biphényl)-4-acetamide.

On porte au reflux pendant 12 heures le mélange constitué de 3 g du produit préparé au stade précédent, 1,66 g d'acide boronique (Préparation 5), 3,5 g de phosphate de potassium monohydraté, 1,32 g de bromure de potassium et 300 mg de Pd(PPh3)4 (palladium tétrakis) dans 50 ml de dioxane. Après avoir versé le milieu réactionnel dans un mélange eau-glace, et extrait à l'acétate d'éthyle, la phase organique est sèchée, filtrée et évaporée sous pression réduite. Le produit brut est purifié par chromatographie en éluant avec le mélange cyclohexane/acétate d'éthyle 85/15. On obtient 1,95 g de produit attendu.
(Rf = 0,05 cyclohexane/acétate d'éthyle 85/15)

| IR ($CHCl_3$) | |
|---|---|
| aldehyde et amide | 1702, 1632 $cm^{-1}$ |
| aromatiques | 1606, 1579, 1560, 1524, 1490$cm^{-1}$ |

Stade C : Acide 4'-[2-[bis(1-méthyléthyl)amino]-2-oxoéthyl)-(1,1'-biphényl)-4-carboxylique.

A une solution de 600 mg de produit préparé au stade précédent dans 5 ml de tétrahydrofurane, on ajoute 418 mg de chlorite de sodium monohydrate dans 1,5 ml d'eau et 497 mg d'acide aminosulfonique et agite 10 minute à température ambiante. On ajoute de l'acétate d'éthyle et extrait le mélange à la soude 2N. La phase aqueuse est acidifiée avec de l'acide chlorhydrique 2N, extraite à l'acétate d'éthyle, sèchée puis évaporée sous pression réduite pour fournir le produit brut attendu que l'on chromatographie sur silice en éluant avec le mélange acétonitrile/eau 8/2. On obtient 40 mg du produit attendu.

| RMN (DMSO) | |
|---|---|
| 1,02 (d) et 1,32 (d) | $CH(C\underline{H}_3)_2$ |
| 3,42 (m) et 4,05 (m) | $C\underline{H}(CH_3)_2$ |
| 3,71 (s) | $C\underline{H}_2CO$ |
| 7,34 (d) 7,69 (d) | Aromatiques |
| 7,80 (d) 8,01 (d) | Aromatiques |
| 12,86 (étalé) | H mobiles |

| IR (Nujol) | |
|---|---|
| CO | 1708 cm-1 |
| Aromatiques, amide | 1608,1584, 1562, 1530 et 1499 cm-1 |

**Exemple 10 : Acide 4'-[2-[bis[1-méthyléthyl)amino]-1-méthyl-2-oxoéthyl]-(1,1'-biphényl)-4-carboxylique**

Stade A : N,N-bis(1-méthyléthyl)-4'-formyl-alpha-méthyl-(1,1'-biphényl)-4-acetamide.

On chauffe au reflux 1,29 g du produit obtenu à l'exemple 9 stade B dans 0,42 ml d'éthylène glycol, 35 mg d'acide paratoluène sulfonique et 40 ml de toluène pendant 3 heures, rajoute 0,42 ml d'éthylène glycol et chauffe 2 heures supplémentaires au reflux. Le milieu réactionnel est ensuite versé sur une solution saturée en hydrogéno carbonate de sodium, puis on extrait au dichlorométhane, sèche puis évapore sous pression réduite pour obtenir 1,23 g de produit protégé. Après dissolution de ce produit dans 20 ml de tétrahydrofurane on ajoute à -78°C 1,2 équivalent de lithium diisopropylamine (préparé à partir de 0,54 ml de diisopropylamine et de 3,3 ml de n-butyllithium dans l'hexane) agite 1 heure à cette température et ajoute 0,31 ml de iodure de méthyle. Après agitation pendant 30 minutes à température ambiante, le mélange est versé sur de la glace et on extrait à l'acétate d'éthyle. La phase organique est séchée, évaporée sous pression réduite et chromatographiée sur silice en éluant avec le mélange cyclohexane/acétate d'éthyle 7/3. On obtient 922 mg de cétal intermédiaire.
On porte au reflux pendant 12 heures un mélange constitué de 460 mg du cétal obtenu précédemment, 1,6 ml d'acide

chlorhydrique 6N et 5 ml d'éthanol puis on verse sur de la glace, extrait à l'acétate d'éthyle, sèche la phase organique et évapore sous pression réduite pour obtenir 393 mg d'aldehyde attendu, utilisé tel quel pour le stade suivant.

Stade B : Acide 4'-[2-[bis[1-méthyléthyl)amino]-1-méthyl-2-oxoéthyl]-(1,1'-biphényl)-4-carboxylique.

On ajoute 18,04 ml de soude 1N et 3,14 g d'Ag20 à une solution de 380 mg du produit obtenu au stade précédent dans 8 ml de tétrahydrofurane et agite 24 heures à température ambiante. Après avoir ajouté de l'acide chlorhydrique 6N, on extrait à l'acétate d'éthyle. On obtient ainsi 380 mg du produit attendu que l'on recristallise dans l'isopropanol.
Rf = 0,11 Acétate d'éthyle/cyclohexane 1/1

| RMN (DMSO) | |
|---|---|
| 0,59 (d)<br>1,1 (d) et 1,26 (d)<br>1,28 (d) et 1,35 (d)<br>3,33 (masqué,m) et 4,07 (m)<br>7,36 (d) 7,71 (d)<br>7,79 (d) 8,00 (d)<br>12,97 (étalé) | CH-C<u>H</u>$_3$<br>CH(C<u>H</u>$_3$)$_2$<br><br>C<u>H</u>(CH$_3$)$_2$ et C<u>H</u>-CH$_3$<br>Aromatiques<br>Aromatiques<br>H mobiles |

| IR (Nujol) | |
|---|---|
| CO<br>Aromatiques, amide | 1716 cm-1<br>1606, 1580 et 1492 cm-1 |

**Exemple 11 : Acide 4'-[2-[bis(1-méthyléthyl)amino)-2-oxoéthoxy]-2'-éthyl-(1,1'-biphényl)-4-carboxylique.**

Stade A :

Produit A :2-éthyl-4-(phénylméthoxy)-phénol.
Produit B :3-éthyl-4-(phénylméthoxy)-phénol

1) A une solution, sous atmosphère inerte, de 5 g de 2-éthyl-3-hydroxyphénol, dans 100 ml de diméthylformamide, on ajoute 7,5 g d'imidazole et 9,3 ml de diphényl terbutyl chlorosilane, agite 48 heures à température ambiante et évapore sous pression réduite le solvant. On reprend le produit brut par de l'eau, extrait avec de l'acétate d'éthyle, sèche sur sulfate de magnésium et évapore sous pression réduite et purifie par chromatographie sur silice en éluant avec le mélange cyclohexane/acétate d'éthyle 95/5 pour obtenir 10 g d'un mélange de deux produits brut (Rf = 0,12 cyclohexane/ acétate d'éthyle 98/2).
2) On ajoute à une solution du mélange de produits précédent dans 200 ml de diméthylformamide, 1,4 g d'hydrure de sodium (50 % dans l'huile) puis 3,6 ml de bromure de benzyle et agite pendant 24 heures à température ambiante. Après évaporation sous pression réduite du solvant, le produit brut est repris dans de l'eau, extrait à l'acétate d'éthyle, la phase organique est séchée sur sulfate de magnésium, filtrée, puis évaporée sous pression réduite pour obtenir 12 g d'un mélange de deux produits brut.
3) On mélange ensuite à température ambiante, pendant 1 heure le mélange de produits précédent avec 40 ml de fluorure de tétrabutyl ammonium et 300 ml de tétrahydrofurane puis évapore sous pression réduite le solvant. Après chromatographie sur silice, éluant cyclohexane-acétate d'éthyle (95-5), on obtient 900 mg de produit A et 1,9 g de produit B.

Produit A : 2-éthyl-4-(phénylméthoxy)-phénol
(Rf produit 0,45 cyclohexane-acétate d'éthyle 80-20)

| IR ($CHCl_3$) | |
|---|---|
| 3605 $cm^{-1}$<br>1600, 1506$cm^{-1}$ | Phénol<br>Aromatiques |

| RMN (250MHz, $CDCl_3$) | |
|---|---|
| 7,25 7,47 (m) | H aromatique de benzyle |
| 5,00 (s) | C<u>H</u>$_2$ du benzyle |
| 6,68 (m) 2H | H aromatiques du phénol |
| 6,80 (sl) 1H | H aromatiques du phénol |
| 2,60 (q) | C<u>H</u>$_2$CH$_3$ |
| 1,25 (t) | CH$_2$C<u>H</u>$_3$ |
| 4,37 (s) | OH |

Produit B : 3-éthyl-4-(phénylméthoxy)-phénol (Rf produit 0,40 cyclohexane-acétate d'éthyle 80-20)

| IR ($CHCl_3$) | |
|---|---|
| 3603 $cm^{-1}$ | Phénol |
| 1599, 1502$cm^{-1}$ | Aromatiques |

| RMN (250MHz, $CDCl_3$) | |
|---|---|
| 7,25 7,47 (m) | H aromatique de benzyle |
| 5,01 (s) | C<u>H</u>$_2$ du benzyle |
| 6,59 (dd), 6,69 (d) 6,77 (d) | H aromatiques du phénol |
| 2,66 (q) | C<u>H</u>$_2$CH$_3$ |
| 1,20 (t) | CH$_2$C<u>H</u>$_3$ |
| 4,42 (s) | OH |

<u>Stade B :</u> 2'-éthyl-4'-(phénylméthoxy)-(1,1'-biphényl)-4-carboxaldehyde.

1) Formation du triflate

A 900 mg du produit A obtenu au stade précédent en solution dans 50 ml de dichlorométhane, on ajoute sous atmosphère inerte 1,64 g de diterbutyl méthyl pyridine et 1,3 ml d'anhydride triflique et agite 3 heures à température ambiante. Après lavage à l'eau, la phase organique est séchée sur sulfate de magnésium, filtrée puis évaporée à sec sous pression réduite. On chromatographie le résidu sur silice (cyclohexane/acétate d'éthyle 90/10). On obtient 1,4 g du triflate que l'on engage dans la réaction de couplage.
(Rf=0,5 cyclohexane/acétate d'éyhyle 90/10)

2) Couplage

On mélange sous atmosphère inerte 1,4 g du triflate précédent, 150 ml de dioxane, 2 g de monohydrate de phosphate tripotassique, 920 mg de bromure de potassium, 200 mg de palladium Tetrakis, 1040 mg d'acide boronique, et porte au reflux pendant 4 heures. Après évaporation sous pression réduite, on chromatographie le brut obtenu sur silice en éluant avec un mélange cyclohexane-acétate d'éthyle 95-5 et obtient 830 mg du produit attendu (Rf=0,5 cyclohexane/acétate d'éthyle 80-20).

| IR ($CHCl_3$) | |
|---|---|
| Carbonyle | 1701 $cm^{-1}$ |
| Aromatiques | 1606, 1573, 1563, 1516, 1487 $cm^{-1}$ |

| RMN (300MHz, $CDCl_3$) | |
|---|---|
| 7,2 à 7,5 (m) | H aromatique du benzyle |
| 5,12 (s) | O-C<u>H</u>$_2$Ph |

(suite)

| RMN (300MHz, $CDCl_3$) | |
|---|---|
| 6,97 (d) | H'$_3$ |
| 6,88 (dd) | H'$_5$ |
| 7,07 (d) | H'$_6$ |
| 2,58 (q) | $C\underline{H}_2CH_3$ |
| 1,1 (t) | $CH_2C\underline{H}_3$ |
| 7,46, 7,91 | H aromatiques (Ar-CHO) |
| 10,24 (s) | CHO |

Stade C : Acide 2'-éthyl-4'-(phénylméthoxy)-(1,1'-biphényl)-4-carboxylique.

On ajoute à une solution, sous atmosphère inerte, de 400 mg du produit préparé précédemment dans 20 ml d'acétone, 0,3 ml de réactif de Jones et agite 30 minutes à température ambiante. Le milieu est ensuite repris dans un mélange de 100 ml d'eau et 100 ml d'acétate d'éthyle et la phase organique est séchée sur sulfate de magnésium, filtrée puis évaporée sous pression réduite jusqu'à obtention d'un produit brut qui est recristallisé dans 20 ml d'un mélange 50/50 d'éther/ pentane.
On obtient 400 mg du produit attendu. (Rf=0,1 cyclohexane/ acétate d'éthyle 80-20).

| IR ($CHCl_3$) | |
|---|---|
| Carbonyle | 1676 $cm^{-1}$ |
| Aromatiques | 1610, 1566, 1518, 1496 $cm^{-1}$ |

Stade D : Acide 2'-éthyl-4'-hydroxy-(1,1'-biphényl)-4-carboxylique.

400 mg du produit précédent sont ajoutés à 100 mg de palladium sur charbon dans 50 ml d'acétate d'éthyle. Après 24 heures d'agitation à température ambiante sous atmosphère d'hydrogène, le milieu réactionnel est filtré puis évaporé sous pression réduite jusqu'à obtention d'un produit brut que l'on purifie par chromatographie sur silice en éluant avec un mélange $CH_2Cl_2$/AcOH 997/003. On obtient 110 mg de produit attendu. (Rf=0,1 $CH_2Cl_2$/MeOH 95/5)

| IR ($CHCl_3$) | |
|---|---|
| hydroxyle | 3600 $cm^{-1}$ |
| Carbonyle | 1723, 1693 $cm^{-1}$ |
| Aromatiques | 1611, 1582, 1566, 1514 $cm^{-1}$ |

Stade E : Acide 4'-[2-[bis(1-méthyléthyl)amino)-2-oxoéthoxy]-2'-éthyl-(1,1'-biphényl)-4-carboxylique.

On procède de la même façon qu'à l'exemple 2 stade A mais à partir de 70 mg de produit préparé précédemment. On obtient 40 mg de produit attendu (Rf=0,18 Cyclohexane/Acétate d'éthyle 80/20)

| IR ($CHCl_3$) | |
|---|---|
| Hydroxyle | 3500 $cm^{-1}$ |
| Carbonyle | 1734, 1720, 1692, 1654, 1637 $cm^{-1}$ |
| Aromatiques | 1575, 1564, 1518, 1487 $cm^{-1}$ |

| RMN (300MHz, $CDCl_3$) | |
|---|---|
| 1,25 (d) 1,44 (d) | $CH(CH_3)_2$ |
| 3,47 (m) 4,11 (m) | $CH(CH_3)_2$ |
| 4,68 (s) | O-$CH_2$-CO |
| 6,83 (dd) | H'$_3$ |

(suite)

| RMN (300MHz, $CDCl_3$) | |
|---|---|
| 7,12 (d) | H'$_5$ |
| 6,93 (d) | H'$_6$ |
| 2,57 (q) | $C\underline{H}_2CH_3$ |
| 1,09 (t) | $CH_2C\underline{H}_3$ |
| 7,39 8,14 | H aromatiques (Ph-COOH) |
| 12,96 (s) | COOH |

**Exemple 12 : Acide 4'-[2-[bis(1-méthyléthyl)amino)-2-oxoéthoxy]-3'-éthyl-(1,1'-biphényl)-4-carboxylique.**

On procède de la même façon qu'à l'exemple 11 stades B, C, D et E en partant de 1,9 g du produit B préparé au stade A de l'exemple 11.

On obtient 40 mg du produit attendu
(Rf=0,5 éthanol)

| IR ($CHCl_3$) | |
|---|---|
| Carbonyle | 1690, 1636 $cm^{-1}$ |
| Aromatiques | 1607, 1565, 1520, 1493 $cm^{-1}$ |

| RMN (300MHz, $CDCl_3$) | |
|---|---|
| 1,24 (d) 1,42 (d) | $CH(C\underline{H}_3)_2$ |
| 3,46 (m) 4,19 (m) | $C\underline{H}(CH_3)_2$ |
| 4,70 (s) | $O-C\underline{H}_2-CO$ |
| 6,97 (d J=8,5) | H'$_5$ |
| 7,44 (m) | H'$_2$, H'$_6$ |
| 2,75 (s) | $C\underline{H}_2CH_3$ |
| 1,24 (m) | $CH_2C\underline{H}_3$ |
| 7,65 8,14 | H aromatiques (Ph-COOH) |

| Microanalyse | | | | |
|---|---|---|---|---|
| % calculé | C 72,04 | H 7,62 | N 3,65 | O 16,69 |
| % trouvé | C 71,7 | H 7,7 | N 3,3 | |

**Exemple 13 : Acide 4'-[2-[bis(1-méthyléthyl)amino]-2-oxoéthoxy]-2-chloro-(1,1'-biphényl)-4-carboxylique.**

Stade A : 3-chloro-4-[[(trifluorométhyl)sulfonyl]oxy]-benzoate de méthyle .

On agite pendant 6 heures sous atmosphère inerte à 0°C, le mélange constitué de 3 g de 3-chloro-4-hydroxybenzoate de méthyle, 5,3 ml d'anhydride triflique et 30 ml de pyridine puis on verse sur de l'eau glacée. Après extraction avec de l'acétate d'éthyle, et sèchage de la phase organique sur sulfate de magnésium, on concentre sous pression réduite jusqu'à obtention de 6,84 g de produit attendu.

| RMN (DMSO, 300MHz) | |
|---|---|
| 3,90 (s) | $CO_2C\underline{H}_3$ |
| 8,09 (dd) | $H_6$ |
| 7,83 (d) | $H_5$ |
| 8,23 (d) | $H_2$ |

| IR (Nujol) | |
|---|---|
| 1740 $cm^{-1}$<br>1660 $cm^{-1}$<br>1640, 1628, 1603(ép),1546 et 1492 $cm^{-1}$ | (CO)<br>(CO)<br>(aromatiques) |

Stade B : 2-chloro-4'-(phénylméthoxy)-(1,1'-biphényl)-4-carboxylate de méthyle.

On chauffe au reflux pendant 3 heures le mélange constitué par 1,58 g du triflate préparé précédemment, 1,13 g de l'acide (4-(phénylméthoxy)-phényl) boronique (Préparation 2), 0,29 g de tetrakis(triphénylphosphine)-palladium, 1,72 g de phosphate tripotassique-mono-hydrate, 0,65 g de bromure de potassium et 15 ml de dioxane, puis on verse sur de l'eau glacée. Après extraction avec de l'acétate d'éthyle, et sèchage de la phase organique sur sulfate de magnésium, on concentre sous pression réduite jusqu'à obtention de 1,83 g d'huile brune que l'on purifie par chromatographie sur silice en éluant avec un mélange 98/2 de cyclohexane/acétate d'éthyle puis on isole 0,956 g du produit attendu.

| RMN (DMSO, 300MHz) | |
|---|---|
| 3,95 (s) | $CO_2C\underline{H}_3$ |
| 7,95 (dd) | $H_5$ |
| 7,3, 7,5 (m) | 6H arom.: $H_6$ + 5H du benzyle |
| 8,13 (d) | $H_3$ |
| 7,04 et 7,41 (AA'BB') | H aromatique (Ar-O) |
| 5,12 (s) | $C\underline{H}_2$ du benzyle |

Stade C : 2-chloro-4'-hydroxy-(1,1'-biphényl)-4-carboxylate de méthyle.

On place sous atmosphère d'hydrogène le mélange constitué par 286 mg de produit préparé au stade précédent, 91 mg de palladium à 9,5 % sur charbon et 20 ml d'acétate d'éthyle et on agite à 20°C pendant 24 heures. Après filtration et concentration sous pression réduite, on isole 211 mg du produit attendu.

| RMN ($CDCl_3$, 200MHz) | |
|---|---|
| 3,9 (s) | $CO_2C\underline{H}_3$ |
| 7,85 (d) | $H_5$ |
| 7,75 (m) 3H | $H_6$, $H'_2$, $H'_6$ |
| 8,05 (s) | $H_3$ |
| 6,9 (d) 2H | $H'_3$, $H'_5$ |

Stade D : Acide 4'-[2-[bis(1-méthyléthyl)amino]-2-oxoéthoxy]-2-chloro -(1,1'-biphényl)-4-carboxylique.

1) Alkylation

A un mélange constitué par 260 mg du produit préparé au stade précédent dans 5 ml de tetrahydrofurane on additionne sous atmosphère inerte à 0°C 48 mg d'hydrure de sodium (à 50 % en suspension dans l'huile), 220 mg de N,N-bis(1-méthyléthyl)-2-bromo-acétamide (préparation 8) et on agite 3 heures 30 en laissant remonter la température à 20°C. On verse sur 50 ml d'eau glacée, extrait avec de l'acétate d'éthyle, sèche les phases organiques sur sulfate de magnésium et après concentration sous pression réduite isole 385 mg d'huile engagée telle quelle dans la saponification.

2) Saponification

On agite au reflux pendant 24 heures le mélange constitué de 203 mg de l'huile obtenue au stade précédent, 1 ml de soude 2N et 5 ml de tetrahydrofurane, coule le mélange réactionnel sur 50 ml d'eau, extrait avec de l'acétate d'éthyle,lave les phases organique avec de l'acide chlorhydrique 2N, sèche les phases organiques sur sulfate de magnésium et concentre sous pression réduite. On obtient 169 mg du produit attendu.

Rf= 0,39 dichlorométhane/méthanol 9/1

| RMN ($CDCl_3$, 250MHz) | |
|---|---|
| 1,23 1,42 | $CH(C\underline{H}_3)_2$ |
| 3,45 4,05 | $C\underline{H}(CH_3)_2$ |
| 4,64 (s) | $OC\underline{H}_2CO$ |
| 6,95 7,28 | H aromatiques (Ar-O) |
| 8,16 (sl) | $H_3$ |
| 7,28 7,98 | $H_5$, $H_6$ |

| IR (Nujol) | |
|---|---|
| Absorption complexe région OH/NH | |
| 1665 $cm^{-1}$ | CO |
| 1628 $cm^{-1}$ | CO |
| 1610, 1542, 1520 $cm^{-1}$ | Aromatiques |

**<u>Exemple 14</u> : Acide 4'-[2-[bis(1-méthyléthyl)amino]-2-oxoéthoxy]-2, 6-diméthyl-(1,1'-biphényl)-4-carboxylique.**

<u>Stade A</u> : Trifluorométhanesulfonate de (2,6-diméthyl-4-formylphényle).

A un mélange, sous atmosphère inerte à 0°C, constitué de 3,0 g de 3,6-diméthyl-4-hydroxy-benzaldéhyde et 20 ml de pyridine, on ajoute 4,3 ml d'anhydride triflique et agite pendant 3 heures à température ambiante puis on verse sur de l'eau glacée. Après extraction avec du dichlorométhane, et sèchage de la phase organique sur sulfate de magnésium, on concentre à sec sous pression réduite et chromatographie le résidu sur silice en éluant avec le mélange cyclohexane/ acétate d'éthyle (9/1). On obtient ainsi 4,11 g du produit attendu.

| RMN ($CDCl_3$, 300MHz) | |
|---|---|
| 2,49 (s) | 6H Ph-$C\underline{H}_3$ |
| 7,66 (s) | 2H Aromatiques |
| 9,97 (s) | |

<u>Stade B</u> : 2, 6-diméthyl-4'-[[(1,1-diméthyléthyl)diphénylsilyl]oxy]-(1,1'-biphényl)-4-carboxaldehyde.

On agite 16 heures à 100°C sous atmosphère inerte le mélange constitué par 4 g du produit obtenu au stade précédent, 1,58 g de chlorure de lithium, 600 mg de tetrakis (triphénylphosphine)-palladium, 15,56 g de dérivé stanylé (préparation 4) et 50 ml de dioxane. Après avoir évaporé le dioxane sous pression réduite, on ajoute du dichlorométhane et lave la phase organique avec une solution saturée en fluorure de potassium, sèche sur sulfate de magnésium, évapore le dichlorométhane et purifie par chromatographie sur silice en utilisant comme éluant le mélange cyclohexane/ acétate d'éthyle 99/1.
On obtient 0,392 g de produit pur attendu.

| IR ($CHCl_3$) | |
|---|---|
| CO | 1693 $cm^{-1}$ |
| Aromatique | 1607, 1591, 1571, 1510 $cm^{-1}$ |

| RMN ($CDCl_3$, | 250MHz) |
|---|---|
| 1,14 (s) | SitBu |
| 2,03 (s) | Ph-$C\underline{H}_3$ |
| 7,56 (s) | H aromatiques en ortho du CO |
| 6,83 (m) | H aromatiques |

(suite)

| RMN ($CDCl_3$, | 250MHz) |
|---|---|
| 7,30 à 7,80 | H Aromatiques |
| 9,95 (s) | CHO |

Stade C : 2, 6-diméthyl-4'-hydroxy-(1,1'-biphényl)-4-carboxaldehyde.

A une solution de 1,8 g de biphényle protégé préparé au stade précédent dans 10 ml de tétrahydrofurane, on ajoute 3,5 ml de fluorure de tétrabutylammonium et agite 10 minutes à température ambiante. Après évaporation sous pression réduite du tétrahydrofurane on chromatographie sur silice en utilisant comme éluant le mélange cyclohexane/acétate d'éthyle 9/1.
On obtient 634 mg de produit attendu.
IR (CHCl3)
OH : 3597 $cm^{-1}$
CO : 1695 $cm^{-1}$
Aromatique : 1613, 1603, 1592, 1518 $cm^{-1}$

Stade D : N,N-bis(1-méthyléthyl)-2-[[2',6'-diméthyl-4'-formyl-(1,1'biphényl)-4-yl]oxy]-acétamide.

La réaction d'alkylation s'effectue de la même manière qu'au stade D de l'exemple 13, à partir de 189 mg du produit préparé au stade précédent. On obtient 231 g de produit attendu

| IR (CHCl3) | |
|---|---|
| Absence d'OH | |
| CO | 1695, 1656 et 1637 $cm^{-1}$ |
| Aromatiques | 1610, 1577, 1568, 1514 $cm^{-1}$ |

Stade E : Acide 4'-[2-[bis(1-méthyléthyl)amino]-2-oxoéthoxy]-2, 6-diméthyl-(1,1'-biphényl)-4-carboxylique.

La réaction d'oxydation s'effectue de la même manière qu'au stade C de l'exemple 9, à partir de 160 mg du produit préparé au stade D ci-dessus. On obtient 118 mg de produit attendu que l'on recristallise dans l'éther.

| RMN ($CDCl_3$, 250MHz) | |
|---|---|
| 1,25 (d) 1,43 (d) | $CH(C\underline{H}_3)_2$ |
| 3,45 (m) 4,14 (m) | $C\underline{H}(CH_3)_2$ |
| 4,69 (s) | $OC\underline{H}_2CO$ |
| 2,08 (s) | $Ph-C\underline{H}_3$ |
| 7,04 (AA'BB') | Ph-O |
| 7,85 | $H_3$, $H_5$ en ortho du CO |

| IR ($CHCl_3$) | |
|---|---|
| Acide | 3520 $cm^{-1}$ |
| CO : | 1690,1653 et 1636 $cm^{-1}$ |
| Aromatiques : | 1611, 1576, 1514 $cm^{-1}$ |

**Exemple 15 : Acide 4'-[2-[bis(1-méthyléthyl)amino]-2-oxoéthoxy]-2-(trifluorométhyl)-(1,1'-biphényl)-4-carboxylique.**

Stade A : 4'-(phénylméthoxy)-2-trifluorométhyl-(1,1'-biphényl)-4-carboxaldehyde.

On mélange sous atmosphère inerte, 0,911 g de 3-trifluorométhyl-4-bromo-benzaldehyde, 0,903 g d'acide boronique de la préparation 2, 0,208 g de tetrakis (triphénylphosphine)-palladium, 1,24 g de phosphate tripotassique

monohydrate et 10 ml de dioxane, puis agite au reflux pendant 3 heures. Après dilution dans l'eau, extraction avec de l'acétate d'éthyle, de l'éther et du dichlorométhane, les phases organiques sont séchées sur sulfate de magnésium, évaporées sous pression réduite afin d'obtenir 1,584 g de produit brut attendu. Après purification par chromatographie sur silice en éluant avec le mélange cyclohexane/ acétate d'éthyle 95/5, on isole 1,093 g de produit pur. (Rf = 0,33 cyclohexane/acétate d'éthyle 8/2).

| IR ($CHCl_3$) | |
|---|---|
| 2736, 1706 $cm^{-1}$<br>1611 (F), 1580, 1565 (ep.), 1520$cm^{-1}$ | CHO<br>Aromatiques |

Stade B : 4'-hydroxy-2-trifluorométhyl-(1,1'-biphényl)-4-méthanol.

1) Débenzylation

On mélange 1 nuit à 20°C, sous atmosphère d'hydrogène, 308 mg du produit obtenu au stade précédent et 290 mg de palladium à 9,5 % sur charbon actif dans 15 ml d'acétate d'éthyle, puis on filtre le mélange réactionnel. Après évaporation sous pression réduite on obtient 215,7 mg d'un produit brut que l'on engage directement dans la réaction de réduction suivante.

2) Réduction

On mélange à 20°C pendant 3 heure sous atmosphère d'argon 215,7 mg du produit obtenu précédemment et 48 mg d'hydrure de bore et de sodium à 95 % dans 3 ml de méthanol puis évapore sous pression réduite. Le résidu obtenu est repris dans du dichlorométhane et la phase organique est lavée avec une solution saturée en chlorure d'ammonium, séchée sur sulfate de magnésium et évaporée sous pression réduite afin d'obtenir 113 mg d'une poudre blanche que l'on purifie par chromatographie sur colonne de silice en éluant avec le mélange cyclohexane/acétate d'éthyle 8/2. On obtient 99 mg du produit attendu. (Rf= 0,20 cyclohexane/acétate d'éthyle 7/3). mp : 132-134°C
IR (Nujol)
Absence de C=O
Absorption générale région OH/NH
Aromatique : 1615, 1600, 1520 (ép), 1492 $cm^{-1}$

Stade C : Acide-4'-hydroxy-2-trifluorométhyl-(1,1'-biphényl)-4-carboxylique.

On effectue une oxydation à partir de 99 mg du produit obtenu au stade précédent avec le réactif de jones dans les conditions telles que décrites à l'exemple 2 stade C. On obtient 98,5 mg du produit attendu. (Rf = 0,33 dichlorométhane/méthanol 9/1)

Stade D : Acide 4'-[2-[bis(1-méthyléthyl)amino]-2-oxoéthoxy]-2-(trifluorométhyl)-(1,1'-biphényl)-4-carboxylique.

On agite à 80°C pendant 30 minute le mélange constitué de 505 mg du produit obtenu au stade précédent, 1,8 ml de soude 2N, 8 ml de diméthylsulfoxyde et ajoute 397 mg de N,Nbis(1-méthyl éthyl)-2-bromo acétamide. Après avoir agité pendant 2 heures à 80°C, le mélange réactionnel est versé sur 80 ml d'eau, on extrait à l'acétate d'éthyle, lave à l'eau jusqu'à élimination du diméthylsulfoxyde, puis lave à la soude 2N, sèche et évapore sous pression réduite afin d'obtenir 424 mg de produit brut que l'on purifie par chromatographie sur silice en éluant avec le mélange dichlorométhane/méthanol 95/5. On obtient 124 mg de produit pur attendu.
(Rf = 0,26 dichlorométhane/méthanol 9/1)

| RMN ($CDCl_3$, 250MHz) | |
|---|---|
| 1,24 (d) 1,43 (d)<br>3,46 (m) 4,11 (m)<br>4,69 (s)<br>7,01 et 7,26 (AA' BB')<br>7,46 (d)<br>8,25 (dd) | $CH(C\underline{H}_3)_2)$<br>$C\underline{H}(CH_3)_2)$<br>$OC\underline{H}_2CO$<br>H aromatique Ar-O<br>$H_6$ de ArCOOH<br>$H_5$ de ArCOOH |

(suite)

| RMN ($CDCl_3$, 250MHz) | |
|---|---|
| 8,47 (d) | $H_3$ de ArCOOH |

| IR (Nujol) | |
|---|---|
| OH acide | 3520 $cm^{-1}$ + absorption générale |
| C=O acide | 1738 (ép) 1702 $cm^{-1}$ (max) |
| C=O amide | 1657 (ép) 1638 $cm^{-1}$ (max) |
| Aromatique : | 1618 (F), 1582, 1570, 1520, 1490 $cm^{-1}$ |

**Exemple 16 : Acide 4'-[2-[bis(1-méthyléthyl)amino]-2-oxoéthoxy]-2, 6-dichloro-(1,1'-biphényl)-4-carboxylique.**

Stade A : 3,5-dichloro-4-hydroxy-benzaldéhyde.

On agite au reflux pendant 36 heures 15 g d'hydroxy-4-benzaldehyde dans 75 ml de chlorure de thionyle puis évapore sous pression réduite pour obtenir le produit brut attendu que l'on recristallise dans un mélange acétone/n-pentane. On obtient 20 g d'un mélange 85/15 de produit dichloré/ monochloré. Une recristallisation dans le mélange cyclohexane/acétone 4/1 permet d'obtenir le produit dichloré pur.
(Rf=0,15 dichlorométhane/méthanol 99/1)

| IR ($CHCl_3$) | |
|---|---|
| -OH | 3511$cm^{-1}$ |
| C=O | 1600$cm^{-1}$ |
| Aromatiques | 1600, 1588, 1574$cm^{-1}$ |

Stade B : Trifluorométhanesulfonate de (2,6-dichloro-3-formylphényle).

A une solution, sous atmosphère inerte, de 14,6 g du mélange de produits obtenu au stade précédent dans 100 ml de pyridine, on ajoute goutte à goutte, à 0-5°C, 15 ml d'anhydride triflique et agite 1 heure trente. Le milieu réactionnel est versé sur de l'eau glacée puis extrait à l'acétate d'éthyle. La phase organique est séchée sur sulfate de magnésium, filtrée puis évaporée sous pression réduite on obtient 25 g de produit brut que l'on chromatographie sur silice en éluant avec le mélange cyclohexane/acétate d'éthyle 9/1. On obtient alors 20,22 g du mélange de produit attendu : dichloré 92/monochloré 8 (Dichloré : Rf=0,53 cyclohexane/ acétate d'éthyle 7/3).

| IR ($CHCl_3$) (dichloré) | |
|---|---|
| Absence d'OH | |
| C=O | 1711 $cm^{-1}$ |
| $OSO_2CF_3$ | 1434, 1133 $cm^{-1}$ |
| Aromatiques | 1573 $cm^{-1}$ |

Stade C : 2, 6-dichloro-4'-[[(1,1-diméthyléthyl)diphénylsilyl]oxy]-(1,1'-biphényl)-4-carboxaldéhyde.

A une solution sous atmosphère inerte de 20,2 g du produit préparé au stade précédent dans 125 ml de dioxane, on ajoute 8,2 g de bromure de potassium, 15,8 g de phosphate de potassium monohydraté, 22,4 g d'acide boronique ( préparation 3) et 3,6 g de tetrakistriphénylphosphine palladium et porte au reflux pendant 15 heures. Le milieu réactionnel est ensuite filtré puis chromatographié sur silice en éluant avec le mélange cyclohexane/acétate d'éthyle 92/8, 9/1 et enfin 8/2. On obtient 12,5 g de produit comprenant 38 % de monochloré et 62 % de dichloré.
(Rf= 0,63 cyclohexane/acétate d'éthyle 7/3)

| IR ($CHCl_3$) | |
|---|---|
| C=O | 1708 $cm^{-1}$ |

(suite)

| IR ($CHCl_3$) | |
|---|---|
| Aromatiques | 1609, 1592,1548, 1516 $cm^{-1}$ |

Stade D : 2, 6-dichloro-4'-hydroxy-(1,1'-biphényl)-4-carboxaldehyde.

A une solution sous atmosphère inerte de 1,5 g du produit dichloré obtenu au stade précédent dans 5 ml de tétrahydrofurane, on ajoute 2 ml d'une solution 1M de fluorure de tétrabutylammonium et agite à température ambiante pendant 30'

| IR ($CHCl_3$) | |
|---|---|
| CHO<br>Ar | 1707 $cm^{-1}$<br>1612, 1593, 1549, 1519 $cm^{-1}$ |

| RMN ($CDCl_3$) | |
|---|---|
| 4,97 (s)<br>6,97 et 7,16 (AA'BB')<br>7,89 (s)<br>9,96 (s) | OH<br>H aromatiques (Ph-OH)<br>$H_4$, $H_6$ (PhCHO)<br>CHO |

Stade E : N,N-bis(1-méthyléthyl)-2-[[2',6'-dichloro-4'-formyl-(1,1'-biphényl)-4-yl]oxy]-acétamide.

On effectue une alkylation à partir de 116,4 mg du produit dichloré obtenu au stade précédent avec le bromoacétamide (préparation 9) dans les conditions telles que décrites à l'exemple 2 stade A.
On obtient 124 mg du produit attendu. (Rf = 0,26 cyclohexane/ acétate d'éthyle 8/2)

| IR (Nujol) | |
|---|---|
| C=O acide, amide<br>Aromatique : | 1709 $cm^{-1}$ , 1640 $cm^{-1}$<br>1610, 1580, 1550, 1518 $cm^{-1}$ |

Stade F : Acide 4'-[2-[bis(1-méthyléthyl)amino]-2-oxoéthoxy]-2, 6-dichloro-(1,1'-biphényl)-4-carboxylique.

On mélange 124 mg du produit obtenu au stade précédent avec 2 ml de tétrahydrofurane, 3,5 ml d'eau, 98 mg de chlorite de sodium monohydrate et 87 mg d'acide amidosulfonique, agite 1 heure 30 à température ambiante et évapore sous pression réduite jusqu'à obtention d'un résidu que l'on reprend dans de l'acétate d'éthyle. La phase organique est lavée à la soude 1N, séchée sur sulfate de magnésium et évaporée sous pression réduite, on obtient 97,8 mg de produit attendu.
(Rf = 0,26 cyclohexane/acétate d'éthyle 8/2)

| RMN ($CD_3OD$, 300MHz) | |
|---|---|
| 1,27 (d,J=6,5) 1,42 (d,J=6,5)<br>3,57 (sept,J=6,5) 4,13 (sept,J=6,5)<br>4,77 (s)<br>7,07 et 7,18 AA'BB'<br>8,02 (s) | $CH(C\underline{H}_3)_2$<br>$C\underline{H}(CH_3)_2$<br>$OC\underline{H}_2CO$<br>H aromatique (Ar-O)<br>$H_3$, $H_5$ (Ar-COOH) |

| IR ($CHCl_3$) | |
|---|---|
| C=O acide<br>C=O amide | 1704 $cm^{-1}$<br>1636 $cm^{-1}$ |

(suite)

| IR ($CHCl_3$) | |
|---|---|
| Aromatique : | 1610, 1581, 1543, 1517 $cm^{-1}$ |

**Exemple 17 : Acide 4'-[2-[bis(1-méthyléthyl)amino]-2-oxoéthoxy]-2, 6-difluoro-(1,1'-biphényl)-4-carboxylique**.

Stade A : Protection

2-[(3,5-difluorophényl)méthoxy]-tetrahydropyranne. On agite sous atmosphère d'argon, à température ambiante, pendant 2 heure 30, 1,28 g de 3,5-difluorobenzylalcool, 1,3 ml de 3,4-dihydropyrane et 155 mg d'acide paratoluène sulfonique dans 20 ml de dioxane puis verse le mélange réactionnel sur une solution saturée en bicarbonate de sodium. Le dioxane est évaporé sous pression réduite et on extrait la phase aqueuse avec de l'acétate d'éthyle. la phase organique est séchée sur du sulfate de magnésium et évaporée sous pression réduite on obtient 1,81 g de produit attendu.
(Rf=0,42 cyclohexane/ acétate d'éthyle 9/1).

| IR ($CHCl_3$) | |
|---|---|
| Absence d'OH<br>1632, 1602 $cm^{-1}$ | Aromatiques |

Stade B : 2,6-difluoro-4'-phénylméthoxy-(1,1'-biphényl)-4-méthanol.

A une solution de 1,045 g du produit obtenu au stade précédent dans 20 ml de tétrahydrofurane, on ajoute sous atmosphère inerte à -78°C goutte à goutte 3,7 ml d'une solution (1,5M) de n-butyllithium dans l'hexane, agite 15 minutes à -78°C et ajoute 5,5 ml d'une solution (1,0M) de chlorure de Zinc dans le tétrahydrofurane. Au bout de 30 minutes à -78°C, on laisse la température remonter à 20°C et ajoute 1,44 g de 1-bromo 4-(phénylméthoxy) benzène (Préparation 2 stade A) et 265 mg de Tetrakis(triphénylphosphine)-palladium. On chauffe au reflux pendant 5 heures puis verse dans une solution saturée en chlorure d'ammonium, extrait avec du dichlorométhane, sèche la phase organique sur sulfate de magnésium et évapore sous pression réduite jusqu'à obtention d'une huile jaune que l'on engage directement dans la réaction d'hydrolyse.

On ajoute 2,3 ml d'acide chlorhydrique 2N à l'huile brute dissoute dans 30 ml de méthanol et agite à 20°C pendant 24 heures. Le milieu reactionnel est versé sur une solution saturée en bicarbonate de sodium et le méthanol est évaporé sous pression réduite. La phase aqueuse est extraite avec du chlorure de méthylène et la phase organique est séchée sur sulfate de magnésium, filtrée et evaporée sous pression réduite pour obtenir 1,85 g de produit que l'on purifie par chromatographie sur silice en éluant avec le mélange cyclohexane/acétate d'éthyle 9/1. On obtient ainsi 873 mg de produit recherché. (Rf= 0,32 cyclohexane/acétate d'éthyle 7/3)

| IR (Nujol) | |
|---|---|
| Absorption générale OH/NH<br>1640, 1612, 1582, 1569, 1528, 1492 $cm^{-1}$ | Aromatiques |

Stade C : Acide 2, 6-difluoro-4'-(phénylméthoxy)-(1,1'-biphényl)-4-carboxylique.

On effectue une oxydation à partir de 107 mg du produit obtenu au stade précédent avec le réactif de jones dans les conditions telles que décrites à l'exemple 2 stade C. On obtient 86 mg du produit attendu. (Rf = 0,06 cyclohexane/ acétate d'éthyle 7/3)

| IR (Nujol) | |
|---|---|
| Absorption générale OH/NH<br>1704 $cm^{-1}$<br>1610, 1580, 1564, 1522 $cm^{-1}$ | <br>C=O<br>Aromatiques |

Stade D : Acide 2, 6-difluoro-4'-hydroxy-(1,1'-biphényl)-4-carboxylique.

On agite 1 nuit à 20°C, sous atmosphère d'hydrogène, 342 mg du produit obtenu au stade précédent dans 30 ml d'acétate d'éthyle et avec 113 mg de palladium à 9,5 % sur charbon actif. On filtre le mélange réactionnel. Après évaporation sous pression réduite on obtient 232,6 mg du produit attendu.

| IR (Nujol) | |
|---|---|
| Absorption générale OH/NH | |
| 1705 $cm^{-1}$ | C=O |
| 1615, 1597, 1572, 1529 $cm^{-1}$ | Aromatiques |

Stade E : Acide 4'-[2-[bis(1-méthyléthyl)amino]-2-oxoéthoxy]-2,6-difluoro-(1,1'-biphényl)-4-carboxylique.

On effectue une alkylation à partir de 370 mg du produit obtenu au stade précédent avec 428 mg de bromoacétamide (préparation 9) dans les conditions telles que décrites à l'exemple 13 stade D. On obtient 282 mg du produit attendu.
(Rf = 0,35 dichlorométhane/méthanol/acide acétique 95/4/1)

| RMN ($CD_3OD$, 300MHz) | |
|---|---|
| 1,25 (d) 1,44 (d) | $CH(C\underline{H}_3)_2$ |
| 3,47 (m) 4,09 (m) | $C\underline{H}(CH_3)_2$ |
| 4,70 (s) | $OC\underline{H}_2CO$ |
| 7,06 (d) et 7,45 (d) | H aromatique (Ph-O) |
| 7,70 (d, J=8) | $H_3$, $H_5$ (Ph-COOH) |
| 5,20 (étalé) | H mobile $COO\underline{H}$ |

| IR ($CHCl_3$) | |
|---|---|
| C=O acide | 1700 $cm^{-1}$ |
| C=O amide | 1637 $cm^{-1}$ |
| Aromatique : | 1610, 1583, 1568, 1523 $cm^{-1}$ |

**Exemple 18 : Acide 4'-[2-[bis(1-méthyléthyl)amino]-2-oxoéthoxy]-2',6'-dichloro-(1,1'-biphényl)-4-carboxylique.**

Stade A : 3,5-dichloro-1-[[(1,1-diméthyléthyl)diméthylsilyl]-oxy]-benzène.

On agite à 20°C pendant 3 heures un mélange de 5,02 g de 3,5-dichlorophénol, 5,57 g de chlorure de tert-butyldiméthylsilyle, 5,24 g d'imidazole et 50 ml de N,N-Diméthylformamide, puis verse ce mélange sur une solution saturée en chlorure d'ammonium et extrait avec du dichlorométhane. La phase organique est séchée sur du sulfate de magnésium puis concentrée sous pression réduite, on obtient 9 g de résidu que l'on chromatographie sur silice, éluant essence G à 1/00 de triéthylamine. On obtient 7,45 g du produit attendu.

| RMN ($CDCl_3$, 200MHz) | |
|---|---|
| 7,05 (s) 1H | $H_4$ |
| 6,8 (s) 2H | $H_2$, $H_6$ |
| 1,05 (s) 9H | $Si-C(C\underline{H}_3)_3$ |
| 0,3 (s) 6H | $Si-(C\underline{H}_3)_2$ |

Stade B : 2', 6'-dichloro-4'-hydroxy-(1,1'-biphényl)-4-carboxylate de méthyle.

1) Couplage

A une solution de 1,04 g du dérivé chloré préparé au stade A dans 10 ml de tetrahydrofurane, on introduit à

-78°C sous atmosphère inerte 3,9 ml de n-butyllithium (1,16M) dans l'hexane puis 4,5 ml de chlorure de zinc, en solution (1M) dans le tétrahydrofuranne, agite 30 minutes à cette température puis après avoir laissé la température évoluer progressivement vers 20°C, on ajoute 0,966 g de 4-bromobenzoate de méthyle, 0,217 g de Tetrakis (triphénylphosphine) palladium et chauffe au reflux pendant 24 heures. Le mélange réactionnel est ensuite versé sur une solution saturée en chlorure d'ammonium, on extrait avec du dichlorométhane, sèche les phases organiques et concentre sous pression réduite jusqu'à sec on obtient 1,93 g d'une huile que l'on engage telle quelle dans l'étape suivante.

2) Déprotection

On ajoute à 0°C sous atmosphère inerte 5 ml de fluorure de tetrabutylammonium à 1,709 g de produit brut issu de l'étape précédente dans 10 ml de tétrahydrofurane et agite pendant 1 heure 30. Le mélange réactionnel est ensuite versé sur une solution saturée en chlorure d'ammonium, on extrait avec du dichlorométhane, sèche les phases organiques avec du sulfate de magnésium et concentre sous pression réduite jusqu'à sec, on obtient 2,56 g d'huile que l'on chromatographie sur silice avec un mélange éluant cyclohexane/acétate d'éthyle 90/10. On obtient 260 mg d'un mélange comprenant le produit attendu et l'ester butylique correspondant.

Stade C : Acide 4'-[2-[bis(1-méthyléthyl)amino]-2-oxoéthoxy]-2', 6'-dichloro-(1,1'-biphényl)-4-carboxylique.

Après avoir porté au reflux pendant 1 heure 30 le mélange constitué par 194,3 mg du mélange d'ester obtenu au stade précédent, 0,65 ml de soude 2N et 4ml de Diméthylsulfoxyde, on additionne 144 mg de N,N-bis(1-méthyléthyl)-2-bromo-acétamide (preparation 8) et on agite une nuit au reflux. Le mélange réactionnel est ensuite versé sur de l'eau, on extrait avec de l'acétate d'éthyle, sèche les phases organiques avec du sulfate de magnésium et concentre sous pression réduite jusqu'à obtention de 196 mg d'une huile que l'on que l'on purifie par chromatographie sur colonne de silice avec un mélange éluant dichlorométhane/méthanol 90/10. On obtient 51 mg de produit attendu.
Rf=0,5 dichlorométhane/méthanol 9/1 IR (Nujol)

| IR (Nujol) | |
|---|---|
| Absorption OH/NH<br>C=O<br>C=O amide + aromatiques | <br>1712cm$^{-1}$<br>1628, 1614, 1600 (ép), 1552, 1516cm$^{-1}$ |

| RMN (DMSO, 250MHz) | |
|---|---|
| 13,07 (large) | $CO_2H$ |
| 7,38 (d) 8,02 (d) | H aromatique (Ar-COOH) |
| 7,17 (s) 2H | $H'_3$ et $H'_5$ |
| 4,88 (s) 2H | $OC\underline{H}_2CO$ |
| 3,49 (m) 3,91 (m) | $C\underline{H}(CH_3)_2$ |
| 1,19 (d) 1,31 (d) | $CH(C\underline{H}_3)_2$ |

**Exemple 19 : Acide 4'-[[bis(1-méthyléthyl)amino]carbonyl]-2'-iodo, 6'-nitro-(1,1'-biphényl)-4-carboxylique.**

Stade A : N,N-bis(1-méthyléthyl)-4-chloro-3,5-dinitrobenzenecarboxamide.

A une solution, sous atmosphère inerte de 10 g d'acide 4-chloro-3,5-dinitro-benzene-1-carboxylique dans 300 ml de dichlorométhane on additionne goutte à goutte 3 ml de $SOCl_2$ et environ 5 ml de diméthylformamide et on chauffe au reflux pendant 20 heures. On ajoute ensuite 300 ml de dichlorométhane, refroidit à 0°C, ajoute 18 ml de diisopropylamine et agite à température ambiante pendant 12 heures. On dilue le mélange réactionnel dans 250 ml d'acide chlorhydrique 2N, extrait avec du dichlorométhane, évapore sous pression réduite, rajoute de l'éther et filtre le précipité ainsi obtenu ($iPr_2NH_2^+Cl^-$). La phase étherée est lavée avec de l'hydrogénocarbonate de sodium puis concentrée sous pression réduite afin d'obtenir 5,31 g d'extrait sec correspondant au produit attendu.
(Rf=0,5 cyclohexane/acétate d'éthyle 70/30)
IR ($CHCl_3$)
C=O, Aromatiques,$NO_2$ 1637 cm$^{-1}$ , 1610 cm$^{-1}$, 1550 cm$^{-1}$

| RMN (200MHz $CDCl_3$) | |
|---|---|
| 1,2 à 1,5<br>3,5 à 3,7<br>7,9 | $CH(C\underline{H}3)_2$<br>$C\underline{H}(CH3)_2$<br>H Aromatiques |

Stade B : 2'-amino-4'-[[bis(1-méthyléthyl)amino]carbonyl]-6'-nitro-(1,1'-biphényl)-4-carboxylate de méthyle (Produit A) et 4'-[[bis(1-méthyléthyl)amino]carbonyl]-2',6'-dinitro-(1,1'-biphényl)-4-carboxylate de méthyle (Produit B).

On mélange dans un autoclave à 200°C pendant 1 heure 30 1,67 g de l'amide préparée au stade A, 1,39 g de l'iodo benzoate de méthyle (préparation 7), 2,5 g de cuivre et 4 ml de diméthylformamide. Après évaporation du diméthylformamide on extrait avec du chlorure de méthylène, concentre sous pression réduite et purifie par chromatographie sur silice en éluant avec le mélange cyclohexane/Acétate d'éthyle 7/3. On récupère 337,5 mg de produit A et 766 mg de produit B.

| **Produit A** | |
|---|---|
| IR ($CHCl_3$)<br>1723cm$^{-1}$<br>1625cm$^{-1}$<br>1610, 1535cm$^{-1}$<br>3494, 3404cm$^{-1}$ | <br>CO<br>Amide<br>Aromatique + $NO_2$<br>$-NH_2$ |

| **Produit B** | |
|---|---|
| IR ($CHCl_3$)<br>1724cm$^{-1}$<br>1636cm$^{-1}$<br>1612, 1546cm$^{-1}$ | <br>CO<br>Amide<br>Aromatique + $NO_2$ |

Stade C : Acide 2'-amino-4'-[[bis(1-méthyléthyl)amino]-carbonyl]- 6'-nitro-(1,1'-biphényl)-4-carboxylique.

A une solution de 235 mg de produit A préparé précédemment dans 4 ml de tétrahydrofuranne, on ajoute 0,64 ml de soude 2N et chauffe 2 heures au reflux. On dilue ensuite le mélange réactionnel avec 10 ml d'eau, ajuste le pH à 9 avec de l'acide chlorhydrique 1N, lave avec de l'acétate d'éthyle, ajuste le pH à 5 avec de l'acide chlorhydrique 1N et extrait avec de l'éther, sèche les phases éthérées avec du MgSO4, évapore sous pression réduite et effectue une recristallisation du produit dans l'éther pour obtenir 191,7 mg du produit attendu.

| IR ($CHCl_3$) | |
|---|---|
| 1735, 1696, 1624cm$^{-1}$<br>1536, 1487cm$^{-1}$<br>3500, 3410cm$^{-1}$ | CO<br>Aromatique + $NO_2$<br>$-NH_2$ |

Stade D : Acide 4'-[[bis(1-méthyléthyl)amino]carbonyl]-2'-iodo, 6'-nitro-(1,1'-biphényl)-4-carboxylique.

A une solution de 76,6 mg du produit obtenu précédemment et 0,6 ml d'acide sulfurique concentré refroidie à 0°C, on ajoute goutte à goutte 2,7 mg de $NaNO_2$ en solution dans de l'eau, maintient la température à 0°C pendant 3 heures, verse dans 20 g de glace et enfin additionne goutte à goutte 100 mg de iodure de potassium dans 1,5ml d'eau. Après avoir chauffé le mélange à 80°C pendant 15 minutes et refroidit à température ambiante, on extrait avec 10 ml de dichlorométhane, sèche la phase organique, concentre sous pression réduite et purifie par chromatographie sur silice avec un mélange éluant cyclohexane/acétate d'éthyle 50/50 additionné d'1 % d'acide acétique. On obtient 82,2 mg du produit attendu (Rf : 0,34 dichlorométhane/méthanol)

| RMN ($CDCl_3$, 250MHz) | |
|---|---|
| 1,2 à 1,4<br>3,7<br>7,32; 8,20<br>7,84 (d) 8,14 (d) | CH(C<u>H</u>3)$_2$<br>C<u>H</u>(CH3)$_2$<br>H aromatique (Ar-COOH)<br>2H couplés en méta (Ar-CONiPr$_2$) |

**<u>Exemple 20</u> : Acide 4'-[[bis(1-méthyléthyl)amino]carbonyl]-2',6'-dinitro-(1,1'-biphényl)-4-carboxylique.**

On opère de la même façon qu'à l'exemple 19 stade C mais à partir de 244,4 mg de produit B préparé à l'exemple 19 stade B. On obtient 244,5 mg du produit attendu.(Rf 0,146 cyclohexane/acétate d'éthyle 1/1)

| IR ($CHCl_3$) | |
|---|---|
| CO (acide)<br>Aromatique,amide,$NO_2$ | 1696 $cm^{-1}$<br>1628(F), 1609,1567 (ép.),1555 (ép.)<br>1541 (F),1530 $cm^{-1}$(ép) |

| RMN ($CDCl_3$, 250MHz) | |
|---|---|
| 1,2 à 1,7<br>3,75<br>7,39; 8,20<br>8,03 (s) | CH(C<u>H</u>3)$_2$<br>C<u>H</u>(CH3)$_2$<br>Ar-COOH<br>H Aromatiques (ArCONiPr$_2$) |

**<u>Exemple 21</u> : Acide 4'-[[bis(1-méthyléthyl)amino]carbonyl]-2',6'-diamino-(1,1'-biphényl)-4-carboxylique.**

On chauffe au reflux pendant 1 heure, sous atmosphère inerte, le mélange constitué par 349,7 mg du produit de l'exemple 20, 1,55 g de chlorure stanneux et 10 ml d'éthanol puis on ajoute successivement 30 ml d'eau puis de l'ammoniac afin d'ajuster le pH à 7. On extrait avec de l'acétate d'éthyle, ajuste la phase aqueuse à un pH de 5 ce qui entraine la formation d'un précipité qui est extrait avec de l'éther. Les phases organiques sont séchées sur sulfate de magnésium, filtrées et concentrées afin d'obtenir 349,6 mg du produit attendu.
(Rf 0,13 dichlorométhane/méthanol 9/1)

| IR ($CHCl_3$) | |
|---|---|
| CO<br>=C-$NH_2$<br>Aromatique + $NH_2$ | 1696, 1613 $cm^{-1}$<br>3490, 3400 $cm^{-1}$<br>1569 ,1464 $cm^{-1}$ |

| RMN ($CDCl_3$, 250MHz) | |
|---|---|
| 1,2 à 1,51 (s,l)<br>3,5 à 4,05 (1)<br>7,44 (d) ; 8,19 (d)<br>6,13 (s) | CH(C<u>H</u>3)$_2$<br>C<u>H</u>(CH3)$_2$<br>H Aromatiques (Ar-COOH)<br>H Aromatiques $\alpha$ NH2 |

Exemple 22 : 2'-amino-4'-[[bis(1-méthyléthyl)amino]carbonyl]-6'-nitro-(1,1'-biphényl)-4-carboxylate de sodium.

A une solution de 235,3 mg de biphényle, sous atmosphère inerte, préparé au stade B (Produit A) de l'exemple 19 dans 4 ml de tétrahydrofuranne, on ajoute 0,64 ml d'une solution de NaOH 2N et agite pendant 4 heures à température ambiante puis 1 heure 30 au reflux. Après refroidissement on ajuste le pH à pH3 par addition d'acide chlorhydrique 1N, extrait à l'éther, lave la solution éthérée avec 3,7 ml de soude 0,1 N. Après séparation et lyophilisation de la phase aqueuse, on obtient 74,5 mg de produit attendu.

| RMN ($D_2O$, 250MHz) | |
|---|---|
| 1,21 (d) à 1,5(d) | CH(C<u>H</u>3)$_2$ |
| 3,77 (m) et 3,9(m) | C<u>H</u>(CH3)$_2$ |
| 7,11 (d) | H'$_3$ |
| 7,37 (d) | H'$_5$ |
| 7,39 et 7,99 (AA'BB') | H aromatiques (Ar-COONa) |

**<u>Exemple 23</u> : Acide 4'-[[bis(1-méthyléthyl)amino]carbonyl]-2',6'-diiodo-(1,1'-biphényl)-4-carboxylique.**

On opére comme à l'exemple 19D mais à partir de 226 g du produit de l'exemple 21. On obtient 87,7 mg du produit attendu, (Rf. = 0,47 chlorure de méthylène/méthanol 90/10).

| IR (Nujol) | |
|---|---|
| 1690, 1628 $cm^{-1}$ | CO |
| 1612, 1565 ,1510$cm^{-1}$ | Aromatique |

| RMN ($CDCl_3$, 300MHz) | |
|---|---|
| 1,2 à 1,7 | CH(C<u>H</u>3)$_2$ |
| 3,4 à 4,0 | C<u>H</u>(CH3)$_2$ |
| 7,25, 8,24 | Ar-CO |
| 7,88 (s) | H Aromatiques $\alpha$ de I |

**<u>Exemple 24</u> : Acide 4'-[[bis(1-méthyléthyl)amino]carbonyl]-2',6'-dibromo-(1,1'-biphényl)-4-carboxylique.**

On prépare une solution, à 0°C, de 100 mg du produit de l'exemple 21, dans 2 ml d'eau et 0,34 ml d'acide bromhydrique, on ajoute 29 mg de nitrite de sodium en solution dans 0,5 ml d'eau puis ajoute cette solution à 0°C, goutte à goutte, sur le mélange agité à 0°C de 121 mg de bromure de cuivre dans 0,4 ml d'acide bromhydrique à 66 % et agite à température ambiante 16 heures. Après dilution dans 20 ml d'eau glacée, acidification par l'acide chlorhydrique 1N afin d'obtenir un pH de 1, et extraction par de l'éther,on sèche les phases organiques, concentre sous pression réduite et chromatographie sur silice avec un mélange éluant cyclohexane/acétate d'éthyle 50/50 à 1 % d'acide acétique, on obtient 43,9 mg du produit attendu. (Rf 0,47 : Chlorure de méthylène/Méthanol 90/10)

| RMN ($CDCl_3$, 250MHz) | |
|---|---|
| 1,2 à 1,6 | CH(C<u>H</u>3)$_2$ |
| 3,4 à 3,95 | C<u>H</u>(CH3)$_2$ |
| 7,35; 8,23 | Ar-CO |
| 7,59 (s) | H Aromatiques $\alpha$ Br |

**<u>Exemple 25</u> : 4'-[2-[bis(1-méthyléthyl)amino]-2-oxoéthoxy]-N-(hydroxy)-(1,1'-biphényl)-4-carboxamide.**

<u>Stade A</u> : 4'-[2-[bis(1-méthyléthyl)amino]-2-oxoéthoxy]-N-(phénylméthoxy)-(1,1'-biphényl)-4-carboxamide.

On mélange sous atmosphère inerte 170 mg du produit obtenu à l'exemple 2 avec 80 mg de chlorhydrate de benzylhydroxylamine, 0,071 ml de triéthylamine, 70 mg d'hydroxybenzotriazole, 120 mg de dicyclohexyl carbodiimide et 10 ml de chloroforme et agite 8 heure à température ambiante. La phase organique est séchée sur sulfate de magnésium, filtrée puis évaporée sous pression réduite jusqu'à sec, on obtient un produit brut que l'on chromatographie sur silice (en éluant avec le mélange cyclohexane/acétate d'éthyle 70/30).
On obtient 100 mg du produit attendu

| RMN ($CDCl_3$, | 300MHz) |
|---|---|
| 5,06 (s) | C<u>H</u>$_2$Ph |

Stade B : 4'-[2-[bis(1-méthyléthyl)amino]-2-oxoéthoxy]-N-(hydroxy)-(1,1'-biphényl)-4-carboxamide.

On mélange 80 mg de l'hydroxamate de benzyle obtenu au stade précédent avec 50 mg de palladium sur charbon, 5 ml d'éthanol, 40 ml d'acétate d'éthyle, on agite pendant 8 heures sous atmosphère d'hydrogène. Le milieu réactionnel est filtré, évaporé sous pression réduite et purifié par chromatographie sur silice en éluant avec le mélange cyclohexane/ acétate d'éthyle 50/50 puis l'acétate d'éthyle et enfin l'acétate d'éthyle/ acide acétique 99/1.Le produit est enfin recristallisé dans l'acétate d'éthyle. On obtient 5,2 mg du produit attendu.

| IR ($CHCl_3$) | |
|---|---|
| Carbonyle<br>Aromatique | $1630 cm^{-1}$<br>$1606, 1584, 1555, 1494 cm^{-1}$ |

| RMN ($CDCl_3$, 250 MHz) | |
|---|---|
| 1,24 (d) 1,43 (d)<br>3,46 (m) 4,08 (m)<br>4,67<br>7,00 (d) 7,49 (d)<br>7,53 (d) 7,57 (d) | $CH(C\underline{H}_3)_2$<br>$C\underline{H}(CH_3)_2$<br>$O-CH_2-CO$<br>Ph-0<br>Ph-CO |

**Exemple 26 : N,N-bis(1-méthyléthyl)-2[[4'-nitro-(1,1'-biphényl)-4-yl]oxy]-acetamide.**

On prépare le produit comme à l'exemple 2 stade A au départ de 900 mg de 4'-hydroxy-(4-nitro biphényl) (Aldrich) et de 958 mg de N,N-bis(1-méthyléthyl)-2-bromo-acétamide (préparation 8). On obtient 1,26 g de produit attendu.

| IR ($CHCl_3$) | |
|---|---|
| Absence d'OH<br>C0<br>Aromatique + $NO_2$ | <br>$1655, 1638 cm^{-1}$<br>$1600, 1580, 1520, 1490 cm^{-1}$ |

| RMN ($CDCl_3$) | |
|---|---|
| 1,24 (d) 1,42 (d)<br>3,46 (m) 4,18 (m)<br>4,69 (s)<br>7,07 (d) 7,57 (d)<br>7,69 (d) 8,27 | $CH(C\underline{H}_3)_2$<br>$C\underline{H}(CH_3)_2$<br>$O-C\underline{H}_2-CO$<br>H Aromatiques (Ar-O)<br>H Aromatiques (Ar-$NO_2$) |

| Microanalyse | | | |
|---|---|---|---|
| % calculé<br>% Trouvé | C 67,40<br>C 67,3 | H 6,79<br>H 6,8 | N 7,86<br>N 7,8 |

**Exemple 27 : N,N-bis(1-méthyléthyl)-2[[4'-cyano-(1,1'-biphényl)-4-yl]oxy]-acetamide.**

On prépare le produit comme à l'exemple 2 stade A au départ de 780 mg de 4'-hydroxy-(4-biphényl carbonitrile) (Aldrich) et de 1,33 g de N,N-bis(1-méthyléthyl)-2-bromo-acétamide (préparation 8). On obtient 1,2 g de produit attendu.

| IR ($CHCl_3$) | |
|---|---|
| CN | $2229 cm^{-1}$ |

(suite)

| IR ($CHCl_3$) | |
|---|---|
| CO<br>Aromatiques | 1656 et 1638 $cm^{-1}$<br>1606, 1584, 1518 et 1495$cm^{-1}$ |

| RMN ($CDCl_3$, 250MHz) | |
|---|---|
| 1,24 (d) 1,42 (d)<br>3,45 (m) 4,08 (m)<br>4,68 (s)<br>7,06 7,53<br>7,67 (AA'BB') | $CH(C\underline{H}_3)_2$<br>$C\underline{H}(CH_3)_2$<br>$O-CH_2-CO$<br>Aromatiques<br>Aromatiques |

**<u>Exemple 28</u> : N,N-bis(1-méthyléthyl)-2[(4'-1H-tétrazol-5-yl)-(1,1'-biphényl)-4-yl]oxy]-acetamide.**

On porte pendant 12 heures au reflux un mélange de 800 mg du produit de l'exemple 27 et 1,4 ml de azido terbutyl étain ($Bu_3SnN_3$) dans 4 ml de toluène anhydre. Après avoir fait barboter de l'acide chlorhydrique gazeux quelque secondes dans le mélange, on filtre le précipité obtenu. On obtient 931 mg de produit attendu.

| IR (Nujol) | |
|---|---|
| Absorption complexe OH/NH<br>CO<br>Aromatiques, tétrazole | <br>1626$cm^{-1}$<br>1603, 1585, 1567, 1558 et 1518$cm^{-1}$ |

| RMN (DMSO, 250MHz) | |
|---|---|
| 1,19 (d) 1,32 (d)<br>3,49 (m) 4,00 (m)<br>4,78 (s)<br>7,04 et 7,72<br>7,88 et 8,10 | $CH(C\underline{H}_3)_2$<br>$C\underline{H}(CH_3)_2$<br>$O-CH_2-CO$<br>Aromatiques<br>Aromatiques |

**<u>Etude pharmacologique des composés de formule (I)</u>**

Le test de mesure de l'activité 5-α-réductase se fait in-vitro en incubant l'enzyme et son substrat (la Testostérone) et en mesurant par chromatographie HPLC la quantité de métabolites 5-α-réduits formés (dihydrotestostérone (DHT) et 5-α-androstane-diol).

On utilise des homogénats de prostates humaines obtenues par prostatectomies radicales pour cause d'hyperplasie bénigne de la prostate (HBP) : PH6.

<u>Méthode</u>

<u>Préparation de l'homogénat</u> :

La prostate est recueillie dès la sortie du bloc opératoire et emballée dans du papier aluminium placé dans un récipient rempli de glace carbonique. Au laboratoire, si l'homogénat n'est pas préparé immédiatement, la prostate peut être conservée à -80°C.

On émince la prostate sur de la glace en très petits morceaux, que l'on homogénise dans un milieu A (20 mM de phosphate de potassium, pH 6,5 ; 0,32 M de sucrose ; 1 mM de dithiotréïtol (DTT) ; 50 µM de NADPH), au polytron puis au potter verre-verre. Après centrifugation à 140000 g pendant 60 mn à 4°C, on remet les culots obtenus en suspension dans 10 à 20 ml de milieu B (20 nM de phosphate de potassium, pH 6,5 ; 20 % de glycérol ; 1 mM de dithiotréïtol). On répartit dans des tubes Nunc et on conserve à -80°C.

On effectue un dosage des protéines sur un peu d'homogénat pour son utilisation future dans le test et on

s'assure ainsi d'avoir au moins 10 mg de protéines/ml d'homogénat.

Mesure de l'activité 5-α-réductase :

L'incubation se fait à pH 5,5 à raison de 1 ml de milieu/tube, en présence de citrate (milieu et pH normaux de la prostate).

1) Préparation du substrat (S) c'est-à-dire du mélange de Testostérone "froide" et de Testostérone "tritiée" avec une dilution isotopique de 100 dans un tampon citrate tri-sodique pH 5,0 :

a) tampon citrate tri-sodique 40 mM : On pèse 11,76 g de citrate-3Na, $2H_20$ (Merk ref. Art 6448) que l'on dissout dans 1 litre d'eau distillée (Milli-Q) et on ajuste à pH 5,0.
b) mélange de Testostérone "froide" et de Testostérone "tritiée" avec une dilution isotopique de 100 : Pour obtenir une solution de testostérone de 1 mM, on pèse 2,88 mg de Testostérone "froide" que l'on dissout dans 10 ml d'éthanol. Cette solution est diluée au 0,99/1000 soit 9,9 µl dans 10 ml de tampon citrate (= solution $10^{-6}$M). On ajoute 9 µl de Testostérone-$^3$H (NET-370) à 10 ml de la solution précédente (= dilution isotopique de 100). Le substrat (S) est prêt.

2) Préparation du mélange Enzyme +DTT +NADPH

a) Tampon phosphate de potassium, 40 mM, pH 6,5 :
On pèse 5,44 g de $KH_2PO_4$ (Riedel de Haen ref. 30407) que l'on dissout dans 1 l d'eau distillée (Milli-Q) et on ajuste à pH 6,5.
b) DTT 1 mM (Sigma)
On pèse 3,1 mg de DTT et on dissout dans 1 ml de tampon phosphate.
c) NADPH 500 µM (Sigma)
On pèse 8,33 mg de NADPH et on dissout dans 1 ml de tampon phosphate.
d) Mélange enzyme+DTT+NADPH (E) :
Pour un essai, on mélange à 4°C, 0,5 mg de protéines de l'homogénat de prostate, 50 µl de DTT et 50 µl de NADPH. On complète à 500 µl avec le tampon phosphate.

3) Incubation (5α-réduction)
On prépare autant de tubes que d'essais. Dans chaque tube, on met 500 µl de (S) et 10 µl d'inhibiteur à la concentration voulue (ou d'éthanol pour les témoins).
On préincube au bain-marie les tubes contenant le mélange (S) et la fiole contenant le mélange (E) pendant 3 à 5 mn.
Pour démarrer la réaction, on transfère rapidement 500 µl de E dans chaque tube contenant S + l'inhibiteur et on incube à 37°C avec une légère agitation pendant 30 mn. La réaction est stoppée à la fin de l'incubation en mettant les tubes dans de la glace.
4) Extraction
Aussitôt la réaction stoppée, on ajoute 2 ml d'acétate d'éthyle dans chaque tube et on agite au Multi-tubes vortexer pendant 1 mn. On laisse décanter pendant 10 mn puis on récupère dans d'autres tubes 1,6 ml de la phase organique (supérieure) de l'extrait (= 80 %). Après évaporation totale de l'acétate d'éthyle, on ajoute dans chaque tube 800 µl de la phase mobile de chromatographie HPLC.
5) Chromatographie HPLC :
La mesure des taux de métabolites produits au cours de l'incubation est effectuée en séparant les composés formés par chromatographie liquide à haute performance (HPLC) en phase inverse. On utilise une colonne ODS-Hypersil (diamètre des particules = 5 µm) et une phase mobile composée de $MeOH/THF/H_20$ dans un rapport de 45/15/40. L'extrait recueilli après extraction est injecté automatiquement au niveau de la chaîne HPLC et grâce au système de mesure de radioactivité en ligne (Berthold), on n'apprécie que les métabolites radioactifs, donc formés à partir de la Testostérone marquée du substrat. Cette mesure est très sensible et élimine tous les produits endogènes éventuels.

Expression des résultats et méthodes de calcul :

Les taux de métabolites formés sont calculés par rapport à une courbe étalon stockée dans le logiciel d'exploitation de la chaîne HPLC. Au niveau du calculateur, ils sont transformés en pmoles/µl injectés. Des courbes étalons existent pour la testostérone, la 5α DHT et le 5α androstanediol, les deux métabolites 5-α-réduits de la testostérone.

Les taux sont exprimés en moles de produits formés/mg de protéines d'homogénat, chaque essai étant effectué en double, et on calcule le pourcentage d'activité 5-$\alpha$-réductase résiduelle par rapport aux essais témoins :

$$\% = \frac{\text{Taux de produits formés en présence d'inhibiteur x 100}}{\text{Taux de produits formés pour les témoins}}$$

Résultat

La $CI_{50}$ d'inhibition de l'activité 5-$\alpha$-réductase de l'enzyme de prostate humaine (PH6) des produits de l'invention est rapportée dans le tableau suivant :

| Exemple | CI50 |
|---|---|
| Exemple 15 | $5{,}7.10^{-7}$M. |
| Exemple 3 | $7{,}1.10^{-8}$M |
| Exemple 4 | $9{,}6.10^{-9}$M |
| Exemple 13 | $8{,}7.10^{-7}$M |
| Exemple 5 | $9{,}2.10^{-8}$M |
| Exemple 12 | $2{,}0.\ 10^{-6}$M |
| Exemple 11 | $7{,}7.10^{-8}$M |
| Exemple 6 | $3{,}6.10^{-8}$M |

Conclusion :

Tous ces produits possèdent une $CI_{50}$ comprise entre 2,0 $10^{-6}$ et 9,6 $10^{-9}$. Cette activité inhibitrice de la 5$\alpha$-réductase, rend ces produits aptes à être utilisé dans le traitement des troubles liés à l'hyperandrogénie.

**Revendications**

1. A titre de médicaments les composés de formule (I) :

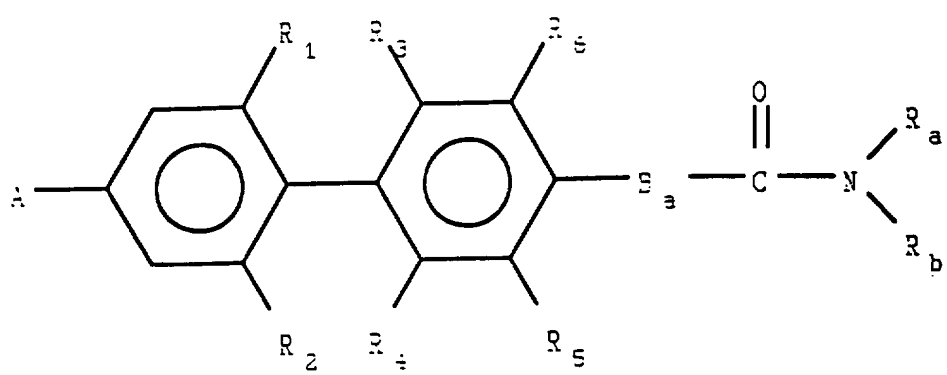

(I)

dans laquelle A représente un radical carboxy, cyano, nitro, HONHCO- ou un hétérocycle azoté, $B_a$ représente une simple liaison, un groupement $(CH_2)_n$, n étant un entier pouvant varier de 1 à 6, $(CH_2)_{n-1}$-$CH(Alk_1)$, $Alk_1$ étant un radical alkyle linéaire ou ramifié renfermant de 1 à 8 atomes de carbone, ou O-$(CH_2)_{n'}$, n' étant un entier pouvant varier de 1 à 6, $R_a$ et $R_b$, identiques ou différents, représentent un atome d'hydrogène, un radical alkyle linéaire ou ramifié renfermant de 1 à 8 atomes de carbone, un radical acyle renfermant de 1 à 12 atomes de carbone, un radical phényle, un radical benzyle, un radical diphénylméthyle, un radical trityle ou forment ensemble avec l'atome d'azote auquel ils sont liés un hétérocycle azoté saturé ou insaturé à 5 ou 6 chaînons pouvant renfermer un second hétéroatome choisi parmi les atomes d'azote, d'oxygène et de soufre, $R_1$, $R_2$, $R_3$ et $R_6$ identiques ou différents, indépendamment l'un de l'autre représentent un atome d'hydrogène, un atome d'halogène, un radical alkyle linéaire

ou ramifié renfermant de 1 à 8 atomes de carbone éventuellement substitué, un radical alkoxy ou alkylthio renfermant de 1 à 8 atomes de carbone, un radical nitro, cyano, $-CF_3$, amino, alkylamino ou dialkylamino dans lesquels chaque radical alkyle renferme de 1 à 8 atomes de carbone, un radical $-NR'_aR'_b$ dans lequel $R'_a$ et $R'_b$ forment ensemble avec l'atome d'azote auquel ils sont liés un hétérocycle azoté saturé ou insaturé à 5 ou 6 chaînons pouvant renfermer un second hétéroatome choisi parmi les atomes d'azote, d'oxygène et de soufre et éventuellement substitué par un radical alkyle renfermant de 1 à 4 atomes de carbone, $R_4$ et $R_5$, identiques ou différents, représentent les mêmes valeurs que $R_1$, $R_2$, $R_3$ ou $R_6$ ou forment ensemble un groupement -CH=CH-CH=CH- ainsi que les sels d'addition de ceux-ci.

**2.** A titre de médicaments, les composés de formule générale (I) telle que définie à la revendication 1 dans laquelle A représente un groupement carboxyle et $-B_a-$ représente une simple liaison, un groupement $-CH_2-$, -CH(Me)- ou $-O-CH_2-$ ainsi que les sels d'addition de ceux-ci, avec les acides et les bases.

**3.** A titre de médicaments, les composés de formule générale (I) telle que définie à la revendication 1 dans laquelle A représente un groupement tétrazole et $-B_a-$ représente une simple liaison, un groupement $-CH_2-$, -CH(Me)- ou $-O-CH_2-$ ainsi que les sels d'addition de ceux-ci.

**4.** A titre de médicaments, les composés de formule générale (I) telle que définie à l'une des revendications 1 à 3 dans laquelle $R_a$ et $R_b$ représentent chacun un groupement alkyle linéaire ou ramifié renfermant de 1 à 8 atomes de carbone ainsi que les sels d'addition de ceux-ci.

**5.** A titre de médicaments, les composés de formule générale (I) selon la revendication 4 dans laquelle $R_a$ et $R_b$ représenent chacun un groupement isopropyle.

**6.** A titre de médicaments, les composés de formule générale (I) telle que définie à l'une des revendications 1 à 3 dans laquelle $R_a$ est un atome d'hydrogène et $R_b$ est un groupement trityle ainsi que les sels d'addition de ceux-ci.

**7.** A titre de médicaments, les composés de formule générale (I) telle que définie à l'une des revendications 1 à 6 dans laquelle $R_1$, $R_2$, $R_3$ et $R_6$ identiques ou différents, représentent un atome d'hydrogène, de chlore, d'iode, de brome, de fluor, un radical méthyle, éthyle, propyle, isopropyle, cyano, nitro, amino, diméthylamino, méthoxy, éthoxy, méthylthio, éthylthio, trifluorométhyle, et $R_4$ et $R_5$, identiques ou différents, représentent les mêmes valeurs que $R_1$, $R_2$, $R_3$ ou $R_6$ ou forment ensemble un groupement -CH=CH-CH=CH- ainsi que les sels d'addition de ceux-ci.

**8.** A titre de médicaments, les composés de formule générale (I) telle que définie à l'une des revendications 1, 2 et 4 à 7 dans laquelle A représente un groupement carboxyle, $B_a$ représente une simple liaison, un groupement $-CH_2-$, ou $-O-CH_2-$,

<u>soit</u> $R_a$ et $R_b$ représentent chacun un groupement alkyle linéaire ou ramifié renfermant de 1 à 8 atomes de carbone,
<u>soit</u> $R_a$ représente un atome d'hydrogène et $R_b$ représente un groupement trityle, $R_1$, $R_2$, $R_3$, $R_4$, $R_5$ et $R_6$ identiques ou différents, indépendamment l'un de l'autre représentent un atome d'hydrogène, de chlore, d'iode, de brome, de fluor, un radical méthyle, éthyle, propyle, isopropyle, cyano, nitro, amino, diméthylamino, méthoxy, éthoxy, méthylthio, éthylthio, trifluorométhyle ainsi que les sels d'addition avec ceux-ci.

**9.** A titre de médicaments, les composés de formule générale (I) telle que définie à l'une des revendications 1, 2 et 4 à 8 dans laquelle A représente un groupement carboxyle et $B_a$ représente un groupement $-O-CH_2-$.

**10.** A titre de médicaments, les composés de formule générale (I) tels que définie à la revendication 1 dont les noms suivent :

- Acide 4'-[2-[bis(1-méthyléthyl)amino]-2-oxoéthoxy)-(1,1'-biphényl)-4-carboxylique,
- Acide 4'-[2- [bis(1-méthyléthyl)amino] -2-oxoéthoxy]-3',5'-bis(1-méthyléthyl)-(1,1'-biphényl)-4-carboxylique,
- Acide 4'-[2-[bis(1-méthyléthyl)amino] -2-oxoéthoxy] -3'-fluoro-5'-nitro-(1,1'-biphényl)-4-carboxylique,
- Acide 4'-[2-oxo-2-([triphénylméthyl)amino] éthoxy]-(1,1'-biphényl)-4-carboxylique,
- Acide 3'-fluoro-5'-nitro- 4'-[2-oxo-2-[(triphénylméthyl) amino]éthoxy]-(1,1'-biphényl)-4-carboxylique,
- Acide 4'-[2-[bis(1-méthyléthyl)amino)-2-oxoéthoxy]-2'-éthyl-(1,1'-biphényl)-4-carboxylique,
- Acide 4'-[2-[bis(1-méthyléthyl)amino)-2-oxoéthoxy]-3'-éthyl-(1,1'-biphényl)-4-carboxylique,

- Acide 4'-[2-[bis(1-méthyléthyl)amino]-2-oxoéthoxy]-2-chloro (1,1'-biphényl)-4-carboxylique,
- Acide 4'-[2-[bis(1-méthyléthyl)amino]-2-oxoéthoxy]-2-(trifluorométhyl)-(1,1'-biphényl)-4-carboxylique ainsi que les sels d'addition avec ceux-ci.

**11.** Les compositions pharmaceutiques renferment comme principe actif au moins l'un des médicaments tels que définis aux revendications 1 à 10.

**12.** Les composés de formule (I) telle que définie aux revendications 1 à10, étant entendu que sont exclus les produits de formule (I) dans laquelle Ba est une simple liaison.

**13.** Procédé de préparation des composés de formule générale (I) telle que définie à la revendication 1, caractérisé en ce que l'on soumet un composé de formule (II) :

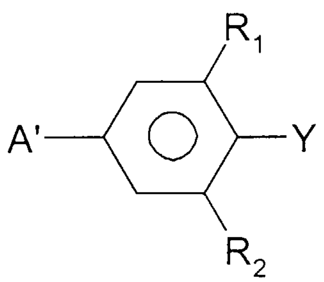

(II)

à l'action en présence d'un catalyseur, d'un composé de formule générale (III) :

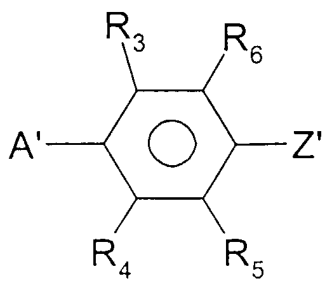

(III)

dans lesquelles, $R_1$, $R_2$, $R_3$, $R_4$, $R_5$ ou $R_6$ sont tels que définis à la revendication 1, A' représente les groupements formyle éventuellement protégé, carboxy éventuellement estérifié, cyano, hétérocycle azoté, nitro, méthyle ou $CH_2OH$ éventuellement protégé, Z' représente un groupement $B_a$-CO-$NR_aR_b$ ou un groupement OH éventuellement protégé et dans lesquels :

(a) soit Y représente un groupement $B(OH)_2$ ou $SnBu_3$ et X représente un groupement $OSO_2CF_3$, un atome de brome ou un atome d'iode,
(b) soit Y représente un groupement $OSO_2CF_3$, un atome de brome ou un atome d'iode et X représente un groupement $B(OH)_2$ ou $SnBu_3$,
(c) soit Y représente un atome d'iode et X représente un atome de chlore,
(d) soit Y représente un atome de chlore et X représente un atome d'iode,
(e) soit Y représente un atome de brome et X représente un atome d'hydrogène,
(f) soit Y représente un atome d'hydrogène et X représente un atome brome,

afin d'obtenir un produit de formule (I') :

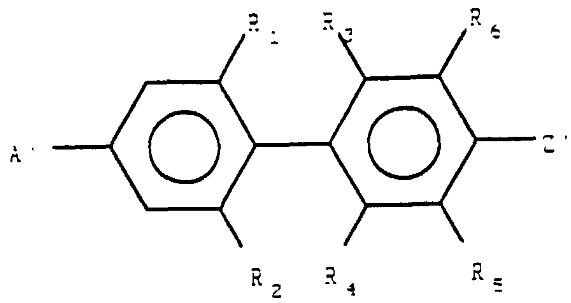

(I')

dans laquelle $R_1$, $R_2$, $R_3$, $R_4$, $R_5$, $R_6$, A' et Z' sont tels que définis précédemment, pouvant, le cas échéant, correspondre à certains produits de formule (I), produit de formule (I') que si désiré ou si nécessaire l'on soumet dans un ordre approprié à l'une ou, le cas échéant, plusieurs des réactions suivantes afin d'obtenir le produit de formule (I) :

- oxydation du groupement formyle que peut représenter A',
- oxydation du groupement méthyle que peut représenter A',
- Oxydation du groupement $CH_2OH$ que peut représenter A'
- saponification de l'ester que peut représenter A',
- action de l'azido terbutyl étain ($Bu_3SnN_3$) puis de l'acide chlorhydrique lorsque A' représente un groupement cyano afin d'obtenir le produit de formule (I) dans lequel A est un radical tétrazole,
- action du chlorhydrate de benzyl hydroxylamine lorsque A' représente $CO_2H$ afin d'obtenir les produits de formule (I) dans lesquels A est un groupement CONHOH,
- protection des groupements $CH_2OH$, CHO, $CO_2H$ que peut représenter A',
- protection du groupement OH que peut représenter Z',
- déprotection des groupements $CH_2OH$, CHO, $CO_2H$ protégés que peut représenter A',
- déprotection du groupement OH protégé que peut représenter Z',
- action d'un groupement Hal-$(CH2)_n$-CO-$NR_aR_b$ lorsque Z' représente un hydroxyle,
- action de $Alk_1$-X en présence d'une base forte lorsque Z ou Z' représentent un groupement $(CH2)_n$-CO-$NR_aR_b$ afin d'obtenir les produits de formule (I) dans lesquels A est un groupement $(CH_2)_{n-1}$-CH($Alk_1$)-CO-$NR_aR_b$,
- réduction totale ou partielle des groupements $NO_2$ que peuvent représenter $R_1$, $R_2$, $R_3$, $R_4$, $R_5$ ou $R_6$,
- substitution du radical $NH_2$ que peuvent représenter $R_1$, $R_2$, $R_3$, $R_4$, $R_5$, $R_6$ par un atome de brome ou d'iode,
- et salification par un acide ou une base.

**14.** Procédé selon la revendication 13 de préparation des produits de formule (I) telle que définie à la revendication 1, caractérisé en ce que l'on soumet un composé de formule ($II_1$) :

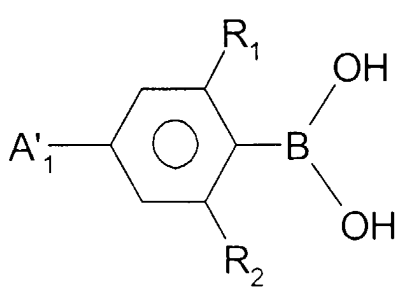

($II_1$)

à l'action, en présence d'un catalyseur choisi parmi les dérivés de Pd, d'un composé de formule générale ($III_1$) :

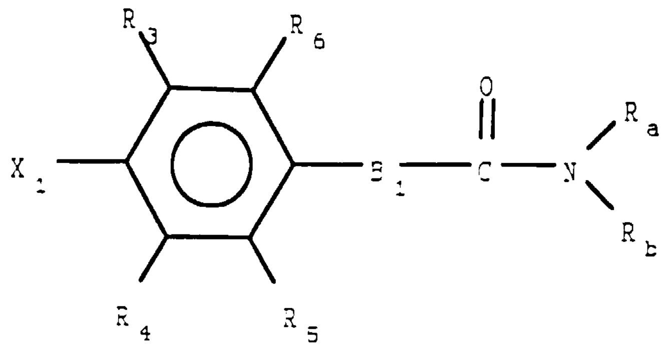

(III'1)

dans lesquelles $R_a$, $R_b$, $R_1$, $R_2$, $R_3$, $R_4$, $R_5$ ou $R_6$ sont tels que définis à la revendication 1, $A'_1$ représente les groupements formyle, carboxy éventuellement estérifié, $X_1$ représente un groupement $O\text{-}SO_2CF_3$, un atome de brome ou un atome d'iode et $B_1$ représente une simple liaison, un groupement $CH_2$ ou un groupement $OCH_2$, afin d'obtenir un produit de formule ($I'_1$) :

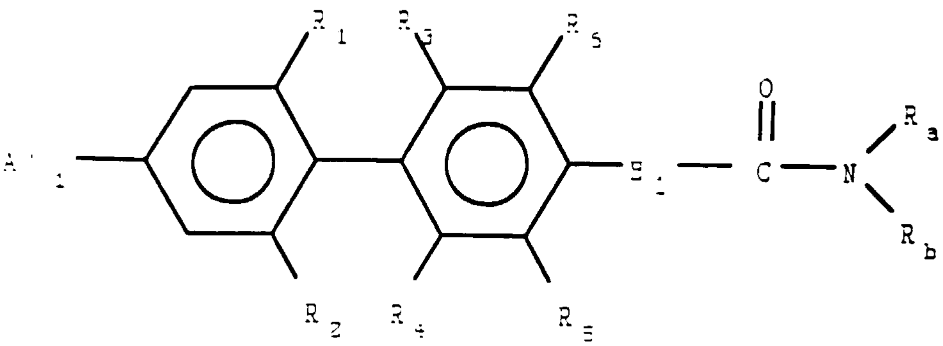

($I'_1$)

dans laquelle $R_a$, $R_b$, $R_1$, $R_2$, $R_3$, $R_4$, $R_5$, $R_6$, $B_1$ et $A'_1$ sont tels que définis précédemment, pouvant, le cas échéant, correspondre à certains produits de formule (I), produit de formule ($I'_1$) que si désiré ou si nécessaire l'on soumet dans un ordre approprié à l'une ou, le cas échéant, plusieurs des réactions suivantes afin d'obtenir le produit de formule (I):

- oxydation du groupement formyle que peut représenter $A'_1$,
- saponification de l'ester que peut représenter $A'_1$,
- réduction totale ou partielle des groupements $NO_2$ que peuvent représenter $R_1$, $R_2$, $R_3$, $R_4$, $R_5$ ou $R_6$,
- substitution du radical $NH_2$ que peuvent représenter $R_1$, $R_2$, $R_3$, $R_4$, $R_5$, $R_6$ par un atome de brome ou d'iode,
- et salification par un acide ou une base.

**15.** A titre de produits industriels, les composés de formule (III) telle que définie à la revendication 13, dans laquelle Z' est un groupement $B_a\text{-}CO\text{-}NR_aR_b$ et X est un groupement $OSO_2CF_3$, $B(OH)_2$ ou $SnBu_3$ étant entendu que les composés de formule (III) dans laquelle Ba est une simple liaison ou un groupement $-CH_2-$ sont exclus.

**16.** A titre de produits industriels, les composés de formule (I') telle que définie à la revendication 13, dans laquelle Z' est un groupement $B_a\text{-}CO\text{-}NR_aR_b$ étant entendu que les composés de formule (IV) dans laquelle Ba est une simple liaison sont exclus.

**Patentansprüche**

**1.** Als Medikamente die Verbindungen der Formel (I):

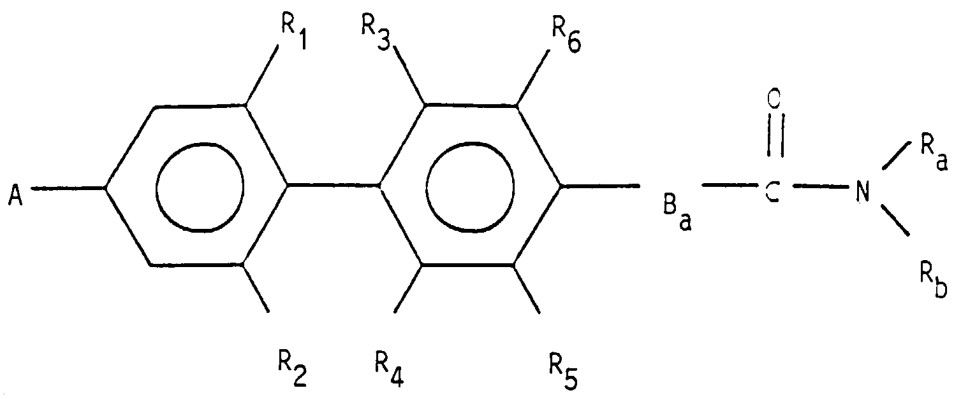

(I)

in der A einen Carboxy-, Cyano-, Nitro-, HONHCO-Rest oder einen Stickstoff-Heterocyclus darstellt, $B_a$ eine Einfachbindung, eine $(CH_2)_n$-Gruppe, wobei n eine ganze Zahl ist, die von 1 bis 6 variieren kann, eine $(CH_2)_{n-1}$-CH ($Alk_1$)-Gruppe, wobei $Alk_1$ ein linearer oder verzweigter Akylrest mit 1 bis 8 Kohlenstoffatomen ist, oder eine O-$(CH_2)_{n'}$-Gruppe, wobei n' eine ganze Zahl ist, die von 1 bis 6 variieren kann, darstellt, $R_a$ und $R_b$, die gleich oder verschieden sind, ein Wasserstoffatom, einen linearen oder verzweigten Alkylrest mit 1 bis 8 Kohlenstoffatomen, einen Acylrest mit 1 bis 12 Kohlenstoffatomen, einen Phenylrest, einen Benzylrest, einen Diphenylmethylrest, einen Tritylrest darstellen oder zusammen mit dem Stickstoffatom, an das sie gebunden sind, einen gesättigten oder ungesättigten Stickstoff-Heterocyclus mit 5 oder 6 Kettengliedern bilden, der ein zweites Heteroatom, das unter den Stickstoff-, Sauerstoff- und Schwefelatomen ausgewählt ist, enthalten kann, $R_1$, $R_2$, $R_3$ und $R_6$, die gleich oder verschieden sind, unabhängig voneinander ein Wasserstoffatom, ein Halogenatom, einen gegebenenfalls substituierten linearen oder verzweigten Alkylrest mit 1 bis 8 Kohlenstoffatomen, einen Alkoxy- oder Alkylthiorest mit 1 bis 8 Kohlenstoffatomen, einen Nitro-, Cyano-, $-CF_3$-, Amino-, Alkylamino- oder Dialkylaminorest, in denen jeder Alkylrest 1 bis 8 Kohlenstoffatome enthält, einen $-NR'_aR'_b$-Rest, in dem $R'_a$ und $R'_b$ zusammen mit dem Stickstoffatom, an das sie gebunden sind, einen gesättigten oder ungesättigten Stickstoff-Heterocyclus mit 5 oder 6 Kettengliedern bilden, der ein zweites Heteroatom, das unter den Stickstoff-, Sauerstoff- und Schwefelatomen ausgewählt ist, enthält und gegebenenfalls mit einem Alkylrest mit 1 bis 4 Kohlenstoffatomen substituiert ist, darstellen, $R_4$ und $R_5$, die gleich oder verschieden sind, dieselben Werte wie $R_1$, $R_2$, $R_3$ oder $R_6$ darstellen oder zusammen eine -CH=CH-CH=CH-Gruppe bilden, sowie deren Additionssalze.

2. Als Medikamente die Verbindungen der allgemeinen Formel (I) wie in Anspruch 1 definiert, in der A eine Carboxylgruppe darstellt und $-B_a-$ eine Einfachbindung, eine $-CH_2-$, -CH(Me)- oder $-O-CH_2$-Gruppe darstellt, sowie deren Additionssalze mit den Säuren und den Basen.

3. Als Medikamente die Verbindungen der allgemeinen Formel (I) wie in Anspruch 1 definiert, in der A eine Tetrazolgruppe darstellt und $-B_a-$ eine Einfachbindung, eine $-CH_2-$, -CH(Me)- oder $-O-CH_2$-Gruppe darstellt, sowie deren Additionssalze.

4. Als Medikamente die Verbindungen der allgemeinen Formel (I) wie in einem der Ansprüche 1 bis 3 definiert, in der $R_a$ und $R_b$ jeweils eine lineare oder verzweigte Alkylgruppe mit 1 bis 8 Kohlenstoffatomen darstellen, sowie deren Additionssalze.

5. Als Medikamente die Verbindungen der allgemeinen Formel (I) gemäß Anspruch 4, in der $R_a$ und $R_b$ jeweils eine Isopropylgruppe darstellen.

6. Als Medikamente die Verbindungen der allgemeinen Formel (I) wie in einem der Ansprüche 1 bis 3 definiert, in der $R_a$ ein Wasserstoffatom ist und $R_b$ eine Tritylgruppe ist, sowie deren Additionssalze.

7. Als Medikamente die Verbindungen der allgemeinen Formel (I) wie in einem der Ansprüche 1 bis 6 definiert, in der $R_1$, $R_2$, $R_3$ und $R_6$, die gleich oder verschieden sind, ein Wasserstoff-, Chlor-, Iod-, Brom-, Fluoratom, einen Methyl-, Ethyl-, Propyl-, Isopropyl-, Cyano-, Nitro-, Amino-, Dimethylamino-, Methoxy-, Ethoxy-, Methylthio-, Ethylthio-, Trifluormethylrest darstellen und $R_4$ und $R_5$, die gleich oder verschieden sind, dieselben Werte wie $R_1$, $R_2$, $R_3$ oder $R_6$ darstellen oder zusammen eine -CH=CH-CH=CH-Gruppe bilden, sowie deren Additionssalze.

8. Als Medikamente die Verbindungen der allgemeinen Formel (I) wie in einem der Ansprüche 1, 2 und 4 bis 7 definiert,

in der A eine Carboxylgruppe darstellt, $B_a$ eine Einfachbindung, eine $-CH_2-$ oder $-O-CH_2$-Gruppe darstellt,

entweder $R_a$ und $R_b$ jeweils eine lineare oder verzweigte Alkylgruppe mit 1 bis 8 Kohlenstoffatomen darstellen, oder $R_a$ ein Wasserstoffatom darstellt und $R_b$ eine Tritylgruppe darstellt, $R_1$, $R_2$, $R_3$, $R_4$, $R_5$ und $R_6$, die gleich oder verschieden sind, unabhängig voneinander ein Wasserstoff-, Chlor-, Iod-, Brom-, Fluoratom, einen Methyl-, Ethyl-, Propyl-, Isopropyl-, Cyano-, Nitro-, Amino-, Dimethylamino-, Methoxy-, Ethoxy-, Methylthio-, Ethylthio-, Trifluormethylrest darstellen, sowie die Additionssalze mit diesen.

**9.** Als Medikamente die Verbindungen der allgemeinen Formel (I) wie in einem der Ansprüche 1, 2 und 4 bis 8 definiert, in der A eine Carboxylgruppe darstellt und $B_a$ eine $-O-CH_2$-Gruppe darstellt.

**10.** Als Medikamente die Verbindungen der allgemeinen Formel (I) wie in Anspruch 1 definiert, deren Namen folgen:

- 4'-[2-[Bis(1-methylethyl)amino]-2-oxoethoxy]-(1,1'-biphenyl)-4-carbonsäure,
- 4'-[2-[Bis(1-methylethyl)amino]-2-oxoethoxy]-3',5'-bis(1-methylethyl)-(1,1'-biphenyl)-4-carbonsäure,
- 4'-[2-[Bis(1-methylethyl)amino]-2-oxoethoxy]-3'-fluor-5'-nitro-(1,1'-biphenyl)-4-carbonsäure,
- 4'-[2-Oxo-2-([triphenylmethyl)amino]ethoxy]-(1,1'-biphenyl)-4-carbonsäure,
- 3'-Fluor-5'-nitro-4'-[2-oxo-2-[(triphenylmethyl)amino]ethoxy]-(1,1'-biphenyl)-4-carbonsäure,
- 4'-[2-[Bis(1-methylethyl)amino]-2-oxoethoxy]-2'-ethyl-(1,1'-biphenyl)-4-carbonsäure,
- 4'-[2-[Bis(1-methylethyl)amino]-2-oxoethoxy]-3'-ethyl-(1,1'-biphenyl)-4-carbonsäure,
- 4'-[2-[Bis(1-methylethyl)amino]-2-oxoethoxy]-2-chloro-(1,1'-biphenyl)-4-carbonsäure,
- 4'-[2-[Bis(1-methylethyl)amino]-2-oxoethoxy]-2-(trifluormethyl)-(1,1'-biphenyl)-4-carbonsäure,

sowie die Additionssalze mit diesen.

**11.** Die pharmazeutischen Zusammensetzungen, die als Wirkstoff wenigstens eines der Medikamente wie in den Ansprüchen 1 bis 10 definiert enthalten.

**12.** Die Verbindungen der Formel (I) wie in den Ansprüchen 1 bis 10 definiert, wobei es sich versteht, dass die Produkte der Formel (I), in der $B_a$ eine Einfachbindung ist, ausgeschlossen sind.

**13.** Verfahren zur Herstellung der Verbindungen der allgemeinen Formel (I) wie in Anspruch 1 definiert, dadurch gekennzeichnet, dass man eine Verbindung der Formel (II):

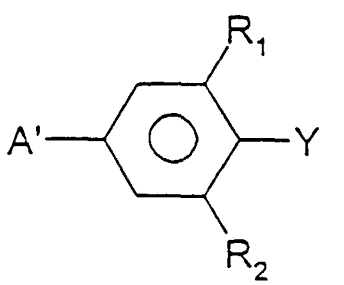

(II)

in Gegenwart eines Katalysators der Wirkung einer Verbindung der allgemeinen Formel (III):

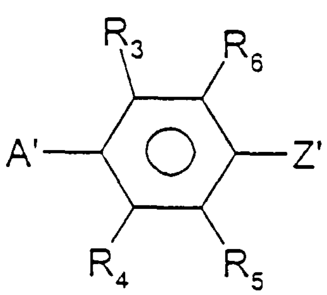

(III)

unterwirft, in denen $R_1$, $R_2$, $R_3$, $R_4$, $R_5$ oder $R_6$ wie in Anspruch 1 definiert sind, A' die gegebenenfalls geschützte Formylgruppe, gegebenenfalls veresterte Carboxygruppe, Cyano-, Stickstoff-Heterocyclus-, Nitro-, Methyl- oder gegebenenfalls geschützte $CH_2OH$-Gruppe darstellt, Z' eine $B_a$-CO-$NR_aR_b$-Gruppe oder eine gegebenenfalls geschützte OH-Gruppe darstellt und in denen:

(a) entweder Y eine $B(OH)_2$- oder $SnBu_3$-Gruppe darstellt und X eine $OSO_2CF_3$-Gruppe, ein Bromatom oder ein Iodatom darstellt,
(b) oder Y eine $OSO_2CF_3$-Gruppe, ein Bromatom oder ein Iodatom darstellt und X eine $B(OH)_2$- oder $SnBu_3$-Gruppe darstellt,
(c) oder Y ein Iodatom darstellt und X ein Chloratom darstellt,
(d) oder Y ein Chloratom darstellt und X ein Iodatom darstellt,
(e) oder Y ein Bromatom darstellt und X ein Wasserstoffatom darstellt,
(f) oder Y ein Wasserstoffatom darstellt und X ein Bromatom darstellt, um ein Produkt der Formel (I'):

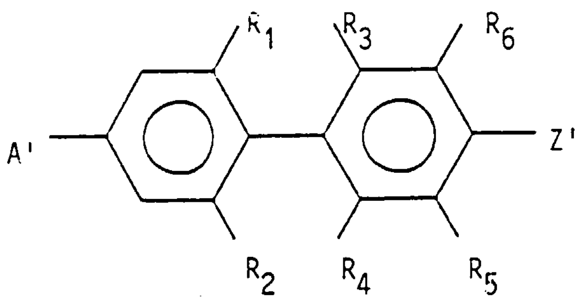

(I')

in der $R_1$, $R_2$, $R_3$, $R_4$, $R_5$, $R_6$, A' und Z' wie zuvor definiert sind, zu erhalten, das gegebenenfalls bestimmten Produkten der Formel (I) entsprechen kann, ein Produkt der Formel (I'), das man, wenn gewünscht oder wenn notwendig, in einer geeigneten Reihenfolge einer oder gegebenenfalls mehreren der folgenden Reaktionen unterwirft, um das Produkt der Formel (I) zu erhalten:

- Oxidation der Formylgruppe, die A' darstellen kann,
- Oxidation der Methylgruppe, die A' darstellen kann,
- Oxidation der $CH_2OH$-Gruppe, die A' darstellen kann,
- Verseifung des Esters, den A' darstellen kann,
- Einwirkung von Azidotertiärbutylzinn ($Bu_3SnN_3$), dann von Chlorwasserstoffsäure, wenn A' eine Cyanogruppe darstellt, um das Produkt der Formel (I) zu erhalten, in dem A ein Tetrazolrest ist,
- Einwirkung von Benzylhydroxylaminchlorhydrat, wenn A' $CO_2H$ darstellt, um die Produkte der Formel (I) zu erhalten, in denen A eine CONHOH-Gruppe ist,
- Schutz der $CH_2OH$-, CHO-, $CO_2H$-Gruppen, die A' darstellen kann,
- Schutz der OH-Gruppe, die Z' darstellen kann,
- Entfernen der Schutzgruppen von den geschützten $CH_2OH$-, CHO-, $CO_2H$-Gruppen, die A' darstellen kann,
- Entfernen der Schutzgruppe von der geschützten OH-Gruppe, die Z' darstellen kann,
- Einwirkung einer Hal-$(CH_2)_n$-CO-$NR_aR_b$-Gruppe, wenn Z' einen Hydroxyrest darstellt,
- Einwirkung von $Alk_1$-X in Gegenwart einer starken Base, wenn Z oder Z' eine $(CH_2)_n$-CO-$NR_aR_b$-Gruppe darstellen, um die Produkte der Formel (I) zu erhalten, in denen A eine $(CH_2)_{n-1}$-CH($Alk_1$)-CO-$NR_aR_b$-Gruppe ist,
- vollständige oder partielle Reduktion der $NO_2$-Gruppen, die $R_1$, $R_2$, $R_3$, $R_4$, $R_5$ oder $R_6$ darstellen können,
- Substitution des $NH_2$-Rests, den $R_1$, $R_2$, $R_3$, $R_4$, $R_5$, $R_6$ darstellen können, durch ein Brom- oder Iodatom,
- und Salzbildung durch eine Säure oder eine Base.

**14.** Verfahren gemäß Anspruch 13 zur Herstellung der Produkte der Formel (I) wie in Anspruch 1 definiert, dadurch gekennzeichnet, dass man eine Verbindung der Formel ($II_1$):

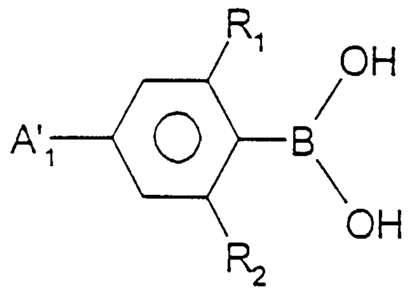

$(II_1)$

in Gegenwart eines Katalysators, der unter den Pd-Derivaten ausgewählt ist, der Wirkung einer Verbindung der allgemeinen Formel ($III_1$):

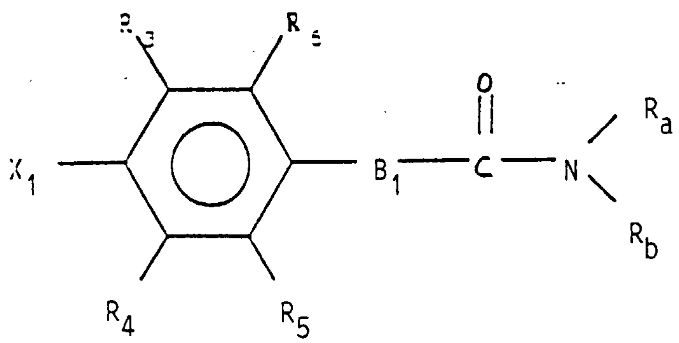

$(III_1)$

unterwirft, in denen $R_a$, $R_b$, $R_1$, $R_2$, $R_3$, $R_4$, $R_5$ oder $R_6$ wie in Anspruch 1 definiert sind, $A'_1$ die Formylgruppe, gegebenenfalls veresterte Carboxygruppe darstellt, $X_1$ eine $O\text{-}SO_2CF_3$-Gruppe, ein Bromatom oder ein Iodatom darstellt und $B_1$ eine Einfachbindung, eine $CH_2$-Gruppe oder eine $OCH_2$-Gruppe darstellt, um ein Produkt der Formel ($I'_1$):

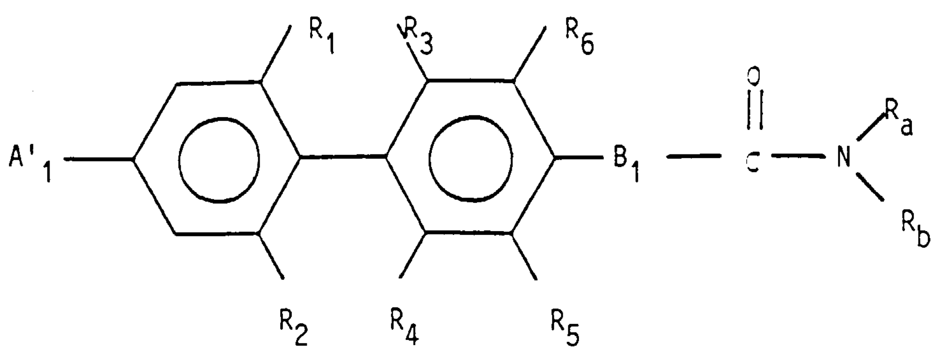

$(I'_1)$

in der $R_a$, $R_b$, $R_1$, $R_2$, $R_3$, $R_4$, $R_5$, $R_6$, $B_1$ und $A'_1$ wie zuvor definiert sind, zu erhalten, das gegebenenfalls bestimmten Produkten der Formel (I) entsprechen kann, ein Produkt der Formel ($I'_1$), das man, wenn gewünscht oder wenn notwendig, in einer geeigneten Reihenfolge einer oder gegebenenfalls mehreren der folgenden Reaktionen unterwirft, um das Produkt der Formel (I) zu erhalten:

- Oxidation der Formylgruppe, die $A'_1$ darstellen kann,
- Verseifung des Esters, den $A'_1$ darstellen kann,
- vollständige oder partielle Reduktion der $NO_2$-Gruppen, die $R_1$, $R_2$, $R_3$, $R_4$, $R_5$ oder $R_6$ darstellen können,
- Substitution des $NH_2$-Rests, den $R_1$, $R_2$, $R_3$, $R_4$, $R_5$, $R_6$ darstellen können, durch ein Brom- oder Iodatom,
- und Salzbildung durch eine Säure oder einer Base.

**15.** Als industrielle Produkte die Verbindungen der Formel (III) wie in Anspruch 13 definiert, in der Z' eine $B_a$-CO-$NR_aR_b$-Gruppe ist und X eine $OSO_2CF_3$-, $B(OH)_2$- oder $SnBu_3$-Gruppe ist, wobei es sich versteht, dass die Verbindungen der Formel (III), in der $B_a$ eine Einfachbindung oder eine -$CH_2$-Gruppe ist, ausgeschlossen sind.

**16.** Als industrielle Produkte die Verbindungen der Formel (I') wie in Anspruch 13 definiert, in der Z' eine $B_a$-CO-$NR_aR_b$-Gruppe ist, wobei es sich versteht, dass die Verbindungen der Formel (IV), in der $B_a$ eine Einfachbindung ist, ausgeschlossen sind.

## Claims

**1.** As medicaments the compounds of formula (I):

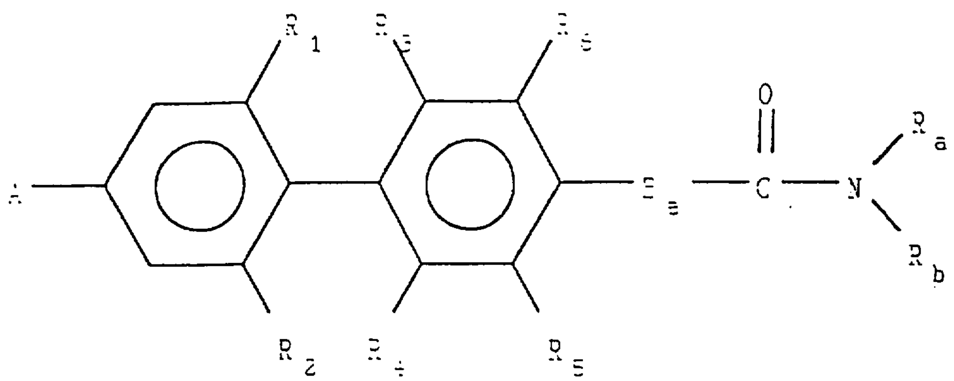

(I)

in which A represents a carboxy, cyano, nitro, HONHCO-radical or a nitrogenous heterocycle, $B_a$ represents a single bond, a $(CH_2)_n$ group, n being an integer which can vary from 1 to 6, $(CH_2)_{n-1}$-CH($Alk_1$), $Alk_1$ being a linear or branched alkyl radical containing 1 to 8 carbon atoms, or O-$(CH_2)_{n'}$, n' being an integer which can vary from 1 to 6, $R_a$ and $R_b$, identical or different, represent a hydrogen atom, a linear or branched alkyl radical containing from 1 to 8 carbon atoms, an acyl radical containing from 1 to 12 carbon atoms, a phenyl radical, a benzyl radical, a diphenylmethyl radical, a trityl radical or together with the nitrogen atom to which they are linked form a saturated or unsaturated nitrogenous heterocycle with 5 or 6 members which can contain a second heteroatom chosen from nitrogen, oxygen and sulphur atoms, $R_1$, $R_2$, $R_3$ and $R_6$ identical or different, independently of one another represent a hydrogen atom, a halogen atom, an optionally substituted, linear or branched alkyl radical containing from 1 to 8 carbon atoms, an alkoxy or alkylthio radical containing from 1 to 8 carbon atoms, a nitro, cyano, -$CF_3$, amino, alkylamino or dialkylamino radical in which each alkyl radical contains from 1 to 8 carbon atoms, an -$NR'_aR'b$ radical in which $R'_a$ and $R'_b$ together with the nitrogen atom to which they are linked form a saturated or unsaturated nitrogen heterocycle with 5 or 6 members which can contain a second heteroatom chosen from nitrogen, oxygen and sulphur atoms and optionally substituted by an alkyl radical containing from 1 to 4 carbon atoms, $R_4$ and $R_5$, identical or different, represent the same values as $R_1$, $R_2$, $R_3$ or $R_6$ or together form a -CH=CH-CH=CH- group as well as their addition salts.

**2.** As medicaments, the compounds of general formula (I) as defined in claim 1 in which A represents a carboxyl group and -$B_a$- represents a single bond, a -$CH_2$-, -CH(Me)- or -O-$CH_2$- group as well as their addition salts with acids and bases.

**3.** As medicaments, the compounds of general formula (I) as defined in claim 1 in which A represents a tetrazole group and -$B_a$- represents a single bond, a -$CH_2$-, -CH(Me)- or -O- $CH_2$- group as well as their addition salts.

**4.** As medicaments, the compounds of general formula (I) as defined in one of claims 1 to 3 in which $R_a$ and $R_b$ each represent a linear or branched alkyl group containing from 1 8 carbon atoms as well as their addition salts.

**5.** As medicaments, the compounds of general formula (I) according to claim 4 in which $R_a$ and $R_b$ each represent an isopropyl group.

**6.** As medicaments, the compounds of general formula (I) as defined in one of claims 1 to 3 in which $R_a$ is a hydrogen atom and $R_b$ is a trityl group as well as their addition salts.

**7.** As medicaments, the compounds of general formula (I) as defined in one of claims 1 to 6 in which $R_1$, $R_2$, $R_3$ and $R_6$ identical or different, represent a hydrogen, chlorine, iodine, bromine, or fluorine atom, a methyl, ethyl, propyl, isopropyl, cyano, nitro, amino, dimethylamino, methoxy, ethoxy, methylthio, ethylthio, trifluoromethyl radical, and $R_4$ and $R_5$, identical or different, represent the same values as $R_1$, $R_2$, $R_3$ or $R_6$ or form together a -CH=CH-CH=CH- group as well as their addition salts.

**8.** As medicaments, the compounds of general formula (I) as defined in one of claims 1, 2 and 4 to 7 in which A represents a carboxyl group, $B_a$ represents a single bond, a $-CH_2-$ or $-O-CH_2-$ group,

either $R_a$ and $R_b$ each represent a linear or branched alkyl group containing from 1 to 8 carbon atoms, or $R_a$ represents a hydrogen atom and $R_b$ represents a trityl group, $R_1$, $R_2$, $R_3$, $R_4$, $R_5$ and $R_6$ identical or different, independently of one another represent a hydrogen, chlorine, iodine, bromine, fluorine atom, a methyl, ethyl, propyl, isopropyl, cyano, nitro, amino, dimethylamino, methoxy, ethoxy, methylthio, ethylthio, trifluoromethyl radical as well as their addition salts.

**9.** As medicaments, the compounds of general formula (I) as defined in one of claims 1, 2 and 4 to 8 in which A represents a carboxyl group and $B_a$ represents an $-O-CH_2$ group.

**10.** As medicaments, the compounds of general formula (I) as defined in claim 1 the names of which follow:

- 4'-[2-[bis(1-methylethyl)amino]-2-oxoethoxy)-(1,1'-biphenyl)-4-carboxylic acid,
- 4'-[2-[bis(1-methylethyl)amino]-2-oxoethoxy]-3',5'-bis(1-methylethyl)-(1,1'-biphenyl)-4-carboxylic acid,
- 4'-[2-[bis(1-methylethyl)amino]-2-oxoethoxy]-3'-fluoro-5'-nitro-(1,1'-biphenyl)-4-carboxylic acid,
- 4'-[2-oxo-2-([triphenylmethyl)amino] ethoxy]-(1,1'-biphenyl)-4-carboxylic acid,
- 3'-fluoro-5'-nitro- 4'-[2-oxo-2-[(triphenylmethyl) amino]ethoxy]-(1,1'-biphenyl)-4-carboxylic acid,
- 4'-[2-[bis(1-methylethyl)amino)-2-oxoethoxy]-2'-ethyl-(1,1'-biphenyl)-4-carboxylic acid,
- 4'-[2-[bis(1-methylethyl)amino)-2-oxoethoxy]-3'-ethyl-(1,1'-biphenyl)-4-carboxylic acid,
- 4'-[2-[bis(1-methylethyl)amino]-2-oxoethoxy]-2-chloro (1,1'-biphenyl)-4-carboxylic acid,
- 4'-[2-[bis(1-methylethyl)amino]-2-oxoethoxy]-2-(trifluoromethyl)-(1,1'-biphenyl)-4-carboxylic acid as well as their addition salts.

**11.** The pharmaceutical compositions containing at least one of the medicaments as defined in claims 1 to 10 as active ingredient.

**12.** The compounds of formula (I) as defined in claims 1 to 10, it being understood that they exclude the products of formula (I) in which Ba is a single bond.

**13.** Preparation process for the compounds of general formula (I) as defined in claim 1, characterized in that a compound of formula (II):

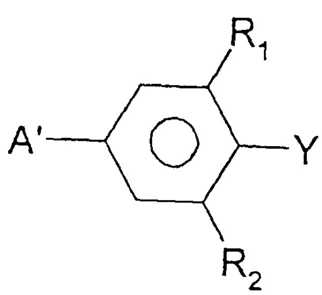

(II)

is subjected, in the presence of a catalyst, to the action of a compound of general formula (III):

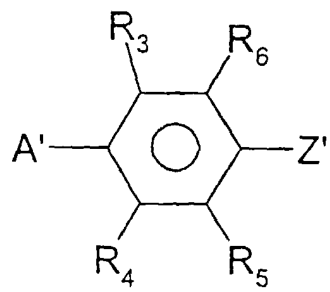

(III)

in which, $R_1$, $R_2$, $R_3$, $R_4$, $R_5$ or $R_6$ are as defined in claim 1, A' represents the following groups: optionally protected formyl, optionally esterified carboxy, cyano, nitrogenous heterocycle, nitro, methyl or optionally protected $CH_2OH$, Z' represents a $B_a$-CO-$NR_aR_b$ group or an optionally protected OH group and in which:

(a) either Y represents a $B(OH)_2$ or $SnBu_3$ group and X represents an $OSO_2CF_3$ group, a bromine or iodine atom,
(b) or Y represents an $OSO_2CF_3$ group, a bromine or iodine atom and X represents a $B(OH)_2$ or $SnBu_3$ group,
(c) or Y represents an iodine atom and X represents a chlorine atom,
(d) or Y represents a chlorite atom and X represents an iodine atom,
(e) or Y represents a bromine atom and X represents a hydrogen atom,
(f) or Y represents a hydrogen atom and X represents a bromine atom,

in order to obtain a product of formula (I'):

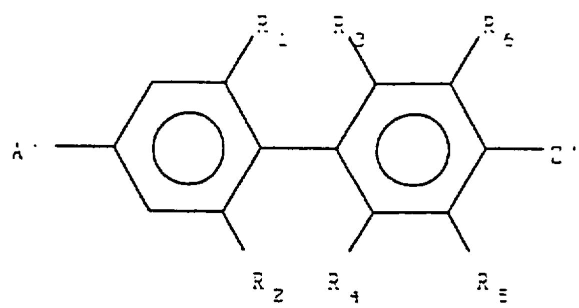

(I')

in which $R_1$, $R_2$, $R_3$, $R_4$, $R_5$, $R_6$, A' and Z' are as defined previously, which can, if appropriate, correspond to certain products of formula (I), which product of formula (I') if desired or if necessary, is subjected, in an appropriate order to one or, if appropriate, more of the following reactions in order to obtain the product of formula (I):

- oxidation of the formyl group which can be represented by A',
- oxidation of the methyl group which can be represented by A',
- oxidation of the $CH_2OH$ group which can be represented by A'
- saponification of the ester which can be represented by A',
- the action of azido terbutyl tin ($Bu_3SnN_3$) then hydrochloric acid when A' represents a cyano group in order to obtain the product of formula (I) in which A is a tetrazole radical,
- the action of benzyl hydroxylamine hydrochloride when A' represents $CO_2H$ in order to obtain the products of formula (I) in which A is a CONHOH group,
- protection of the $CH_2OH$, CHO, $CO_2H$ groups which can be represented by A',
- protection of the OH group which can be represented by Z',
- deprotection of the protected $CH_2OH$, CHO, $CO_2H$ groups which can be represented by A',
- deprotection of the protected OH group which can be represented by Z',
- the action of a Hal-$(CH2)_n$-CO-$NR_aR_b$ group when Z' represents a hydroxyl,
- the action of $Alk_1$-X in the presence of a strong base when Z or Z' represents a $(CH2)_n$-CO-$NR_aR_b$ group in order to obtain the products of formula (I) in which A is a $(CH2)_{n-1}$-CH($Alk_1$)-CO-$NR_aR_b$ group,
- total or partial reduction of the $NO_2$ groups which can be represented by $R_1$, $R_2$, $R_3$, $R_4$, $R_5$ or $R_6$,
- substitution of the $NH_2$ radical which can be represented by $R_1$, $R_2$, $R_3$, $R_4$, $R_5$, $R_6$ by a bromine or iodine atom,

- and salification by an acid or a base.

**14.** Preparation process according to claim 13 for the products of formula (I) as defined in claim 1, characterized in that a compound of formula ($II_1$):

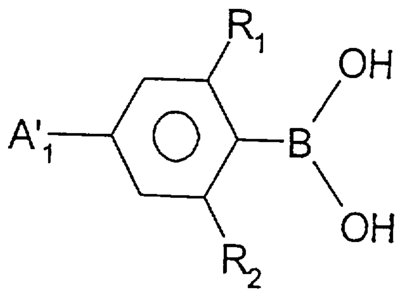

($II_1$)

is subjected, in the presence of a catalyst chosen from the derivatives of Pd, to the action of a compound of general formula ($III_1$):

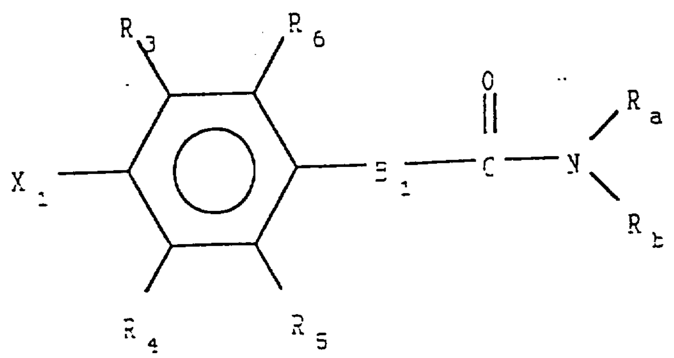

($III_1$)

in which $R_a$, $R_b$, $R_1$, $R_2$, $R_3$, $R_4$, $R_5$ or $R_6$ are as defined in claim 1, $A'_1$ represents the formyl, optionally esterified carboxy groups, $X_1$ represents an $O\text{-}SO_2CF_3$ group, a bromine or iodine atom and $B_1$ represents a single bond, a $CH_2$ group or an $OCH_2$ group, in order to obtain a product of formula ($I'_1$):

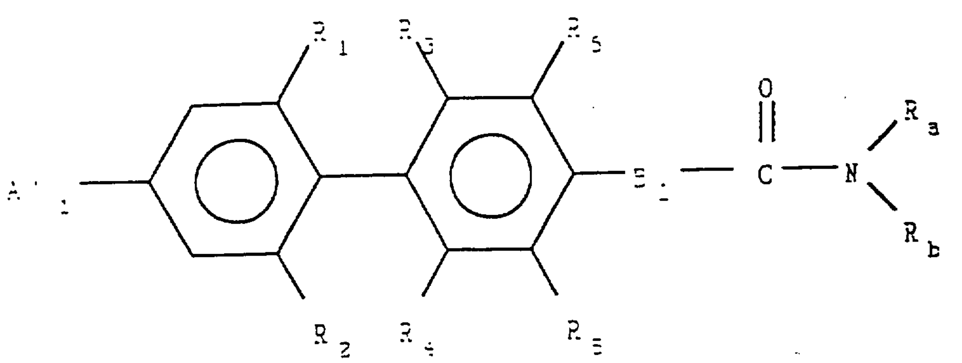

($I'_1$)

in which $R_a$, $R_b$, $R_1$, $R_2$, $R_3$, $R_4$, $R_5$, $R_6$, $B_1$ and $A'_1$ are as defined previously, which can, if appropriate, correspond to certain products of formula (I), which product of formula (I'i) if desired or if necessary is subjected in an appropriate order to one or, if appropriate, more of the following reactions in order to obtain the product of formula (I):

- oxidation of the formyl group which can be represented by $A'_1$,
- saponification of the ester which can be represented by $A'_1$,
- total or partial reduction of the $NO_2$ groups which can be represented by $R_1$, $R_2$, $R_3$, $R_4$, $R_5$ or $R_6$,
- substitution of the $NH_2$ radical which can be represented by $R_1$, $R_2$, $R_3$, $R_4$, $R_5$, $R_6$ by a bromine or iodine atom,

- and salification by an acid or a base.

**15.** As industrial products, the compounds of formula (III) as defined in claim 13, in which Z' is a $B_a$-CO-$NR_aR_b$ group and X is an $OSO_2CF_3$, $B(OH)_2$ or $SnBu_3$ group it being understood that the compounds of formula (III) in which Ba is a single bond or a -$CH_2$- group are excluded.

**16.** As industrial products, the compounds of formula (I') as defined in claim 13, in which Z' is a $B_a$-CO-$NR_aR_b$ group it being understood that the compounds of formula (IV) in which Ba is a single bond are excluded.